# EUROPEAN PATENT SPECIFICATION

(11) **EP 3 142 664 B1**
(45) Date of publication and mention of the grant of the patent: **04.12.2019**
(21) Application number: 15792756.7
(22) Date of filing: 15.05.2015
(51) Int. Cl.: A61K 31/416, A61K 49/00, A61P 27/02, C12Q 1/00, A61P 27/12

(54) **COMPOSITIONS AND METHODS FOR TREATING AND DIAGNOSING OCULAR DISORDERS**
ZUSAMMENSETZUNGEN UND VERFAHREN ZUR BEHANDLUNG UND DIAGNOSE VON AUGENERKRANKUNGEN
COMPOSITIONS ET MÉTHODES POUR LE TRAITEMENT ET LE DIAGNOSTIC D'AFFECTIONS OCULAIRES

(30) Priority: 15.05.2014 US 201461993915 P; 15.10.2014 US 201462064344 P; 06.11.2014 US 201462076162 P; 06.05.2015 US 201562157773 P
(43) Date of publication of application: 22.03.2017
(73) Proprietor: Translatum Medicus Inc., Toronto, Ontario M5A 2M5 (CA)
(72) Inventor: BOYD, Shelley Romayne, Toronto, Ontario M5A 2M5 (CA)
(74) Representative: Grund, Martin
(86) International application number: PCT/IB2015/053609
(87) International publication number: WO 2015/173786

(56) References cited:
- WO-A1-2013/163758
- WO-A1-2013/163758
- WO-A2-2008/061671
- WO-A2-2009/109613

## Description

### FIELD OF THE INVENTION

This invention relates to, in part, compounds for use and compositions that are useful for the diagnosis, treatment, or prevention of an ocular disorder, including the discovery of agents, including immunomodulatory agents, that are efficacious against these disorders.

### BACKGROUND

Ocular disorders, which may reduce or eliminate ones sight, among other effects, are a major medical concern.

For instance, age-related macular degeneration (AMD) is a leading cause of ocular dysfunction, including irreversible blindness, especially in patients over 50 years old. All patients start with early so-called "dry" AMD. This is characterized by deposits that can be seen clinically in the posterior pole of the eye known as the macula. Advanced AMD can take two forms - late "wet" or late "dry". The primary pharmaceutical option in advanced "wet" AMD is regular intra-ocular injections of antiangiogenic drugs. These injections are given after pathological new blood vessels grow beneath or into the central retina, where they leak, bleed or cause tissue damage and scarring. By contrast, there are no treatments for late or early dry AMD. Late dry AMD is characterized by the death or atrophy of the photoreceptors and their nurse cells, the retinal pigment epithelium (RPE). Patches of tissue loss are known as "geographic atrophy" GA.

WO 2013/163758 A1 discloses the use of bindarit for the treatment of age-related macular degeneration. WO 2008/061671 A2 discloses the use of bindarit in the reduction of blood triglyceride, cholesterol and glucose levels. WO 2009/109613 A2 discloses the use of bindarit in the treatment of a variety of diseases.

Accordingly, there is presently a paucity of satisfactory agents for effective treatment of ocular disorders. Therefore, there remains a need for therapies that are useful for treating these disorders. Further, there is a need for effective diagnosis of these disorders and identification of patient populations that will respond to treatments of these disorders.

### SUMMARY OF THE INVENTION

The present invention relates to a composition comprising a compound of Formula I for use in treating a diabetic eye disease (for instance, diabetic retinopathy and DME), including, without limitation, a compound of Formula I such as bindarit. In some embodiments, there is provided a compound of Formula I such as bindarit for use in treating diabetic retinopathy in a patient in need thereof.

The details of the invention are set forth in the accompanying description below. Although methods and materials similar or equivalent to those described herein can be used in the practice or testing of the present invention, illustrative methods and materials are now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs. The scope of the invention is defined by the claims. Any references in the description to methods of treatment refer to the compounds, pharmaceutical compositions and medicaments of the present invention for use in a method for treatment of the human (or animal) body by therapy (or for diagnosis).

### BRIEF DESCRIPTION OF THE FIGURES

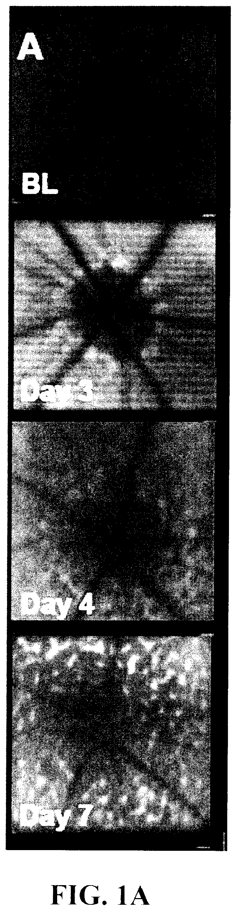

**FIGs. 1A-D** show that *in vivo* FAF imaging following NaIO₃ injection identifies geographic areas of damage that develop a reticular pattern. ICG angiography identifies transient chorioretinal permeability.
**FIG. 1A** shows a time course of early FAF change around the optic nerve head. A hyperfluorescent line or border formed 2-3 days after NaIO₃ injection. The border may or may not completely surround the optic nerve head, and is not contiguous with it. A lacy or reticular pattern of fluorescence appeared between days 3 and 7, and lay peripheral to this line. By day 7 this pattern was distinct.

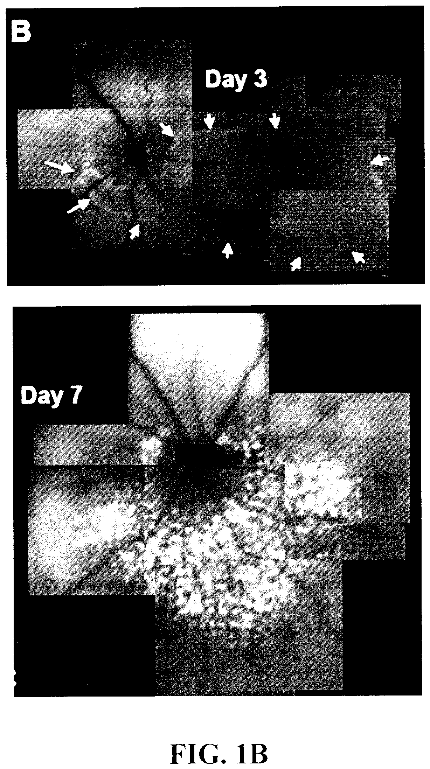

**FIG. 1B** shows composite images that include the optic nerve head and mid-peripheral retina and illustrate that the hyperfluorescent borders defined areas of future lacy or reticular hyperfluorescence. In this example, the borders seen at Day 3 post-NaIO₃ defined an island in the inferior retina. By day 7 the border was no longer visible, and instead, profound reticular patterns emerged. Note that the image showing two distinct islands at Days 3 and 7 are from different animals and demonstrate how consistently the patterns is observed. Small islands such as these were seen in about 7% of eyes.

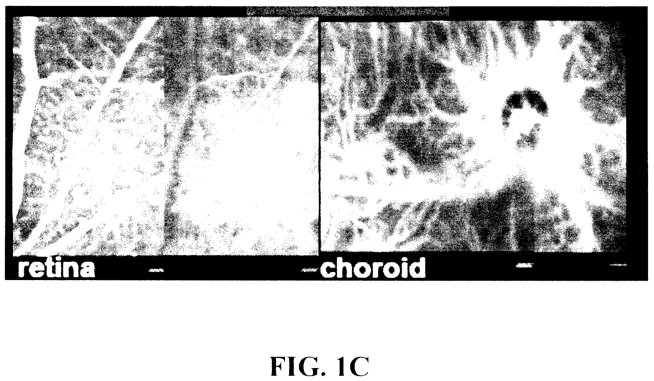

**FIG. 1C** shows that the pattern of FAF is distinctly different from the retinal and choroidal vasculature patterns seen on fluorescein (left and middle image of the inner and mid-retina, respectively) or ICG angiography (right image) of the choroid

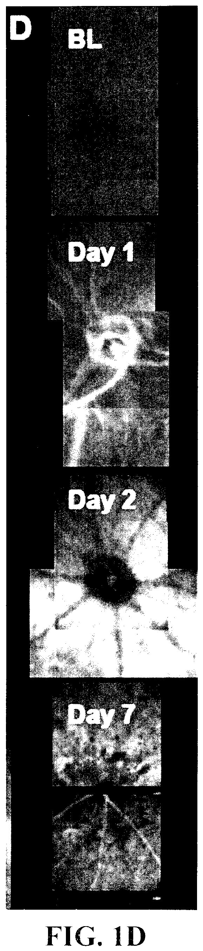

**FIG. 1D** shows a transient increase in trans-epithelial permeability that occurred around Day 2 before pattern progression and is evident by a significant flush during ICG angiography (790 nm, approximately 6 minutes after dye injection). This ICG flush was not detected on Day 1 following toxin injection, appeared briefly, and is rarely seen by the next time point routinely tested, at Day 3 or 4.
**FIGs. 2A-D** show maturation of FAF change after NaIO₃ injection in large 360° rings.
**FIG. 2A** shows large composite FAF images encompassing much of the posterior pole and identified a 360° ring of damage defined by both a peri-papillary and peripheral retinal border. FAF images taken 7 days after NaIO₃ identified multiple discrete and contiguous areas of curvilinear hyperfluorescence that form oval, round, scalloped, rosette, or paw-print patterns. These typically emerged between days 7 and 14. Detail is provided in the upper enlargement. These complex FAF patterns arise within the earlier hyperfluorescent borders, and coalesce over time.
**FIG. 2B** shows that by day 14 most animals show a near contiguous pattern of reticular hyperfluorescence throughout the entire 360° ring. Detail is provided in the middle box.
**FIG. 2C** shows that by 28 days after NaIO₃ exposure, the pattern took on a more granular appearance. Small areas where the pattern is less apparent are seen as darker, grey areas.
**FIG. 2D** shows a clinical image of the "diffuse trickling" pattern of FAF for comparison. The present invention, *inter alia,* recapitulates many of the features including reticular, lobular or scalloped ovals of grey hypofluorescence associated with peripheral hyperfluorescence.
**FIGs. 3A-C** show that a curvilinear, reticular pattern of outer retinal deformation develops in the weeks after NaIO₃ injection.

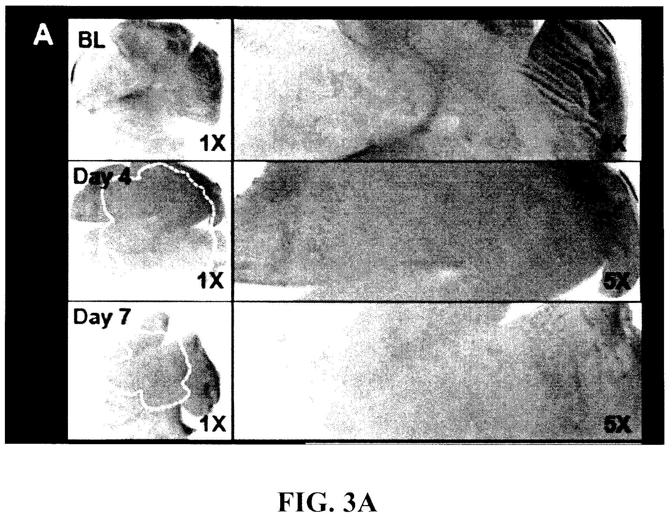

**FIG. 3A** shows that this pattern corresponds with the distribution of autofluorescent cells. Low power (1x, left column; 5x, right column) white light images of excised retinal whole mounts taken prior to NaIO₃ injection (baseline), and at days 4 and 7 post-injection are shown. A subtle curvilinear pattern is noted 3-4 days after compared to baseline, and is apparent at day 7.

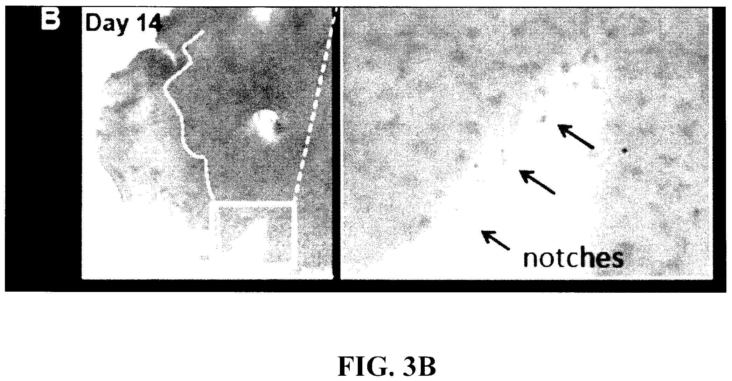

**FIG. 3B** shows that by day 14 post-NaIO₃, this reticular pattern is pronounced and corresponds with gross deformation of the outer retina. This appeared as notches, or folds, in cross-sections seen at the site of relaxing incisions (enlarged in box).

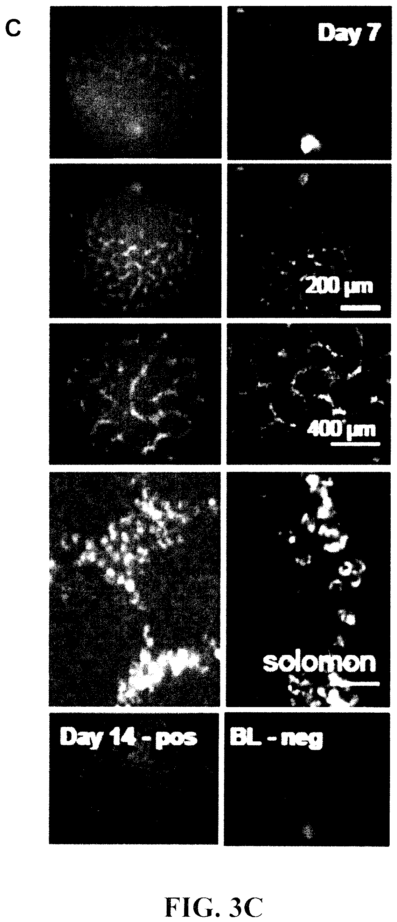

**FIG. 3C** shows that epifluorescent microscopy in the 488 nm channel, with no cellular staining, showed the presence of autofluorescent cells that lie within the reticular grooves. This was evident both using broad light emission (left column) as well as narrow excitation and emission (right column) and corresponds to the wavelength used during FAF imaging. Folds in the retina were associated with the presence of small fluorescent dots by Day 7. Further enlargement clearly showed the distribution of small fluorescent cells, with voids for their nuclei. Positive control tissue following systemic fluorescein-dextran injection (as for angiography) confirmed the ability to detect 488 nm fluorescence in excised retina or eyecup (similar in wavelength to that used for FAF in HRAII imaging) without tissue processing or staining. Negative control at baseline shows absence of autofluorescent cells.

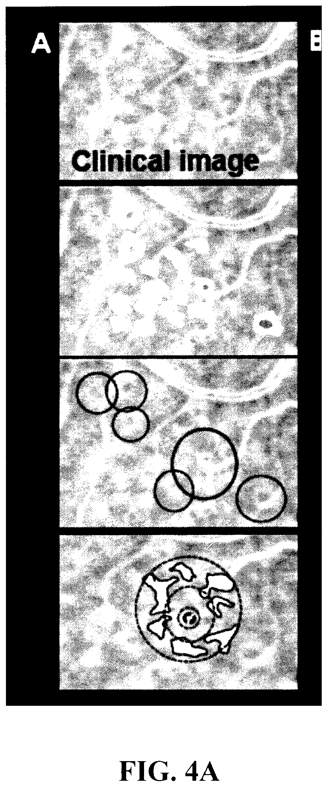

**FIGs. 4A-C** show interpretative illustrations which show how histologically-determined tissue findings can account for *in vivo* imaging following NaIO₃-induced RPE loss.
**FIG. 4A** shows clinical images from a patient with early AMD and shows reticular pseudodrusen and so-called "target" lesions in the NIR channel (top). Immediately below are two schematic overlays that emphasize the hypofluorescent spots (those typically defined as drusenoid deposits) first in black for emphasis, and then with a bright background to emphasis the intervening bright signal. The bottom two images highlight multiple small target lesions of RPD (within the circles), while the bottom-most schematic, illustrates the presence of a potential halo and target lesion formed by concentric folds of tissue.

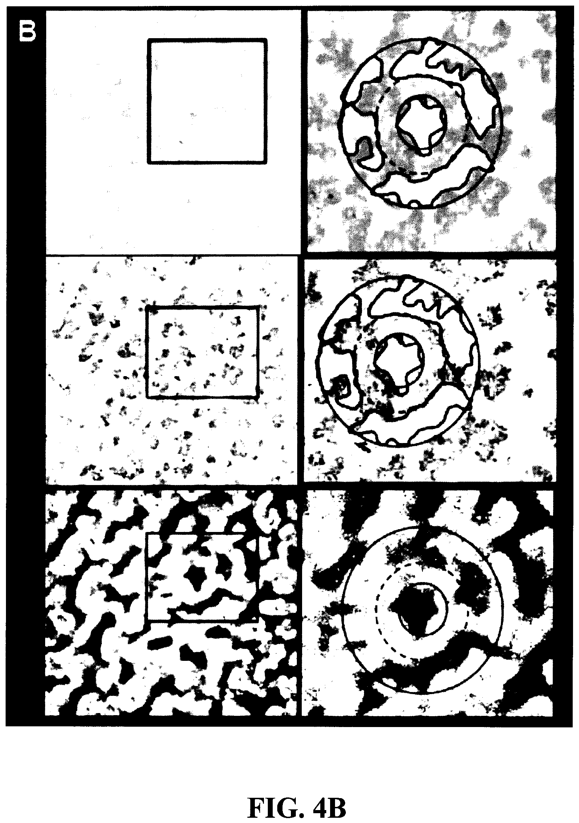

**FIG. 4B** shows schematic renderings which indicate a direct relationship between three-dimensional whole mount ONL histology and two-dimensional *en face* clinical FAF images. By day 14 after NaIO₃ exposure, the outer retina shows complex folds of the mid-ONL (shown at relative low magnification [original 40x] and digitally enlarged). Digital magnification shows concentric rings of tissue folds. This same area illustrated in black and white shows how this might appear on FAF to be a circular or oval region with a central void. Reversed black and white rendering (a "negative" image), shows that this arrangement of ONL distortion would appear to have a central bright target.

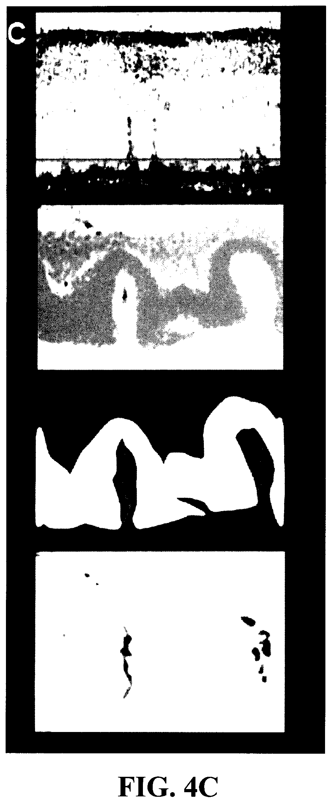

**FIG. 4C** shows OCT imaging of the rat retina 14 days after NaIO₃ injection clearly shows bright "spikes" through the outer retina. These are described clinically. Similar histological sections through the outer retina demonstrate ONL folds with underlying inflammatory cells in the enlarged subretinal space that form narrow columns of cells. For comparison with *in vivo* images, these are rendered in black and white. In reversed black and white (a pseudo-negative image to match OCT images), it is seen that the ONL disappears while the subretinal densities appear bright.

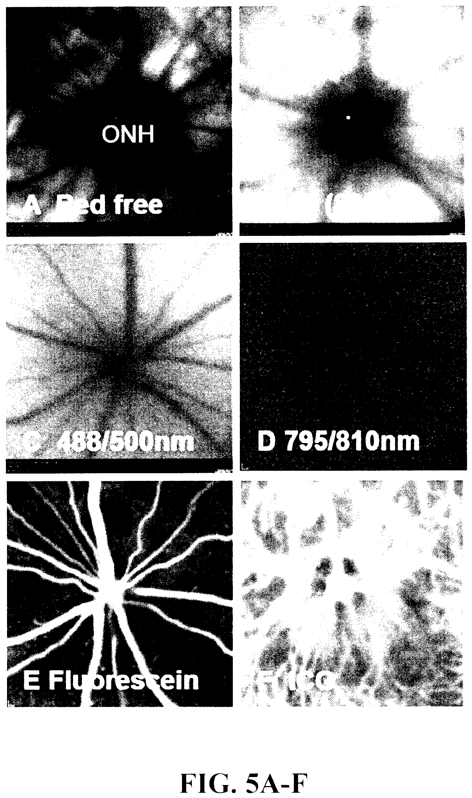

**FIGs. 5A-H** show that speckled NIR fluorescence is detected by 795/810 nm cSLO imaging, but cannot be detected without previous ICG injection, *i.e*., this fluorescence can only be detected with the excitation/emission filters in place, but is not coincident with or in the transit period following dye injection (that is, using the DNIRA method) . **FIG. 5A-F** show representative normal confocal scanning laser ophthalmoscopy (cSLO) and angiographic images of wild-type SD rats obtained using:
**FIG. 5A****:** red free.
**FIG. 5B****:** infrared reflectance (830 nm).
**FIG. 5C****:** 488/500 nm autofluorescence (FAF).
**FIG. 5D****:** 795/810 nm NIR fluorescence.
**FIGs. 5E-F** illustrate normal retinal and choroidal vasculature using fluorescein dextran and ICG dye, respectively. These and similar images served as normal control for all experiments. Such imaging is used to properly align the depth of focus within and between the retina and RPE layers.

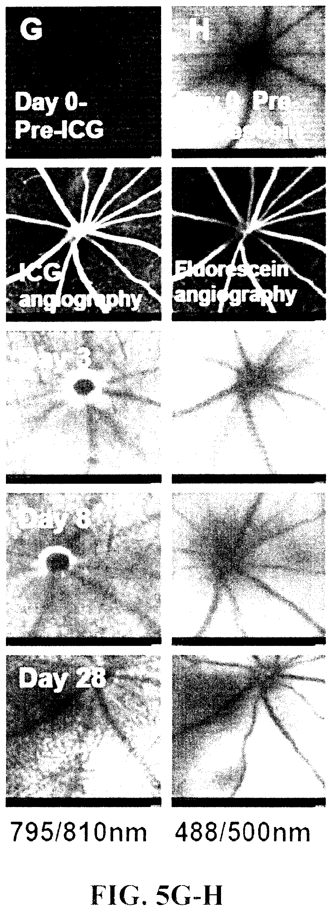

**FIG. 5G** shows a time course of DNIRA in a representative animal that received a single injection of 5 mg/kg ICG dye at t=0. No detectable signal is seen prior to dye injection (note homogenously dark image, upper row). During angiography (second row) the retinal blood vessels were visualized, and background was not detectable noting that the gain (sensitivity) of the cSLO is reduced to prevent saturation during the transit phase. In the days after angiography, (rows 3-5) there is an increased speckled or punctate background fluorescence compared to baseline. The delayed NIR fluorescence is blocked by overlying blood vessels and is absent at the optic nerve head, but unlike FAF (**FIG. 5H**) forms a bright arc or ring around the nerve head. The signal remains strong and distributed throughout the fundus out to 28 days after injection at the concentration used (5 mg/kg). The slightly darker segment of the bottom row likely reflects the presence of a choroidal vortex vein.
**FIG. 5H** shows, in contrast to the DNIRA time course, the 488/500 nm autofluorescence signal reverts to pre-injection intensity (top row) when viewed at 3, 8 and 28 days after fluorescein angiography (lower rows). The slightly darker segment of the bottom row likely reflects the presence of a choroidal vortex vein.

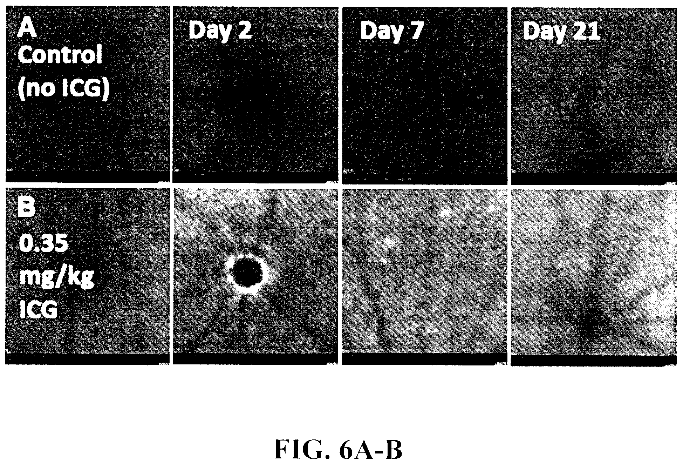

**FIGs. 6A-D** show that the intensity of NIR fluorescent signal correlates with ICG dosage using DNIRA.
**FIG. 6A** shows control cSLO images illustrate an absence of 795/810 nm fluorescent signal when no ICG dye injection has been given. Control animals that did not receive ICG maintained the same level of background fluorescence with negligible signal over the 21 day period evaluated.
**FIG. 6B** show that using the same gain, delayed NIR fluorescence was readily detectable 48 hours after ICG injection when given at the low dose of 0.35 mg/kg. However, with time, DNIRA shows gradual fading from 48 hours to 21 days post-injection.

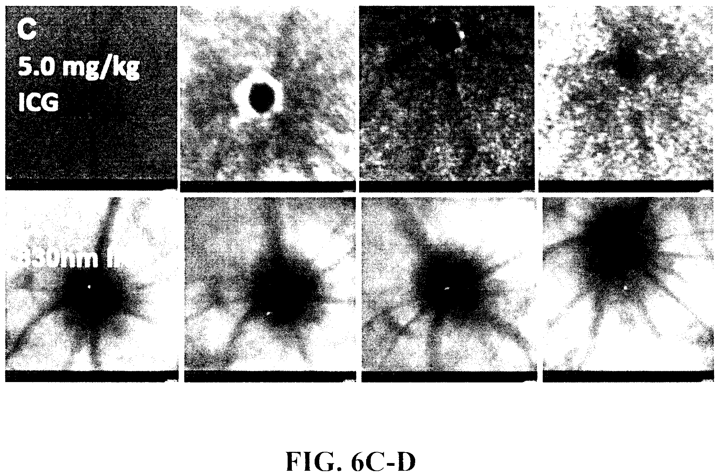

**FIG. 6C** show that using the same gain, 5 mg/kg ICG angiography resulted in brighter (higher intensity) delayed NIR fluorescence at 48 hours than does the lower dose (**FIG. 6B**). This fluorescence persisted largely unchanged out to 21 days.
**FIG. 6D** shows that by contrast, the 830 nm NIR reflectance channel does not show similar change.

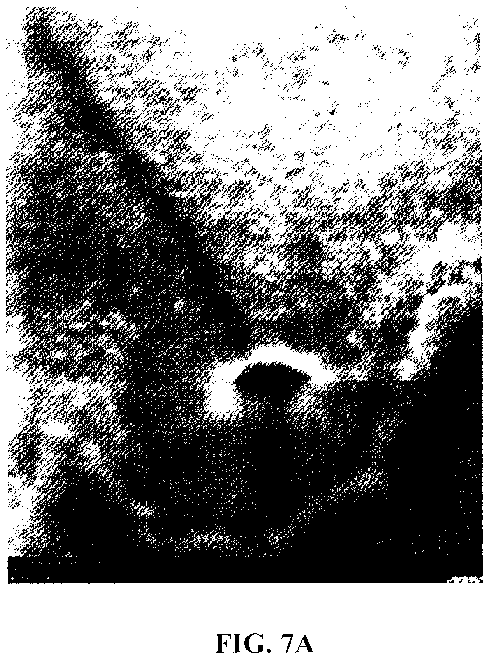

**FIGs. 7A-B** show that sodium iodate causes patchy loss of delayed NIR fluorescence through which develops a clear view to the choroidal vasculature
**FIG. 7A** shows DNIRA performed 3 days after ICG and NaIO₃ injection. Loss of speckled fluorescence in clearly-defined patches or areas of the posterior pole was seen. These areas appeared dark, or hypofluorescent, with clearly defined borders.

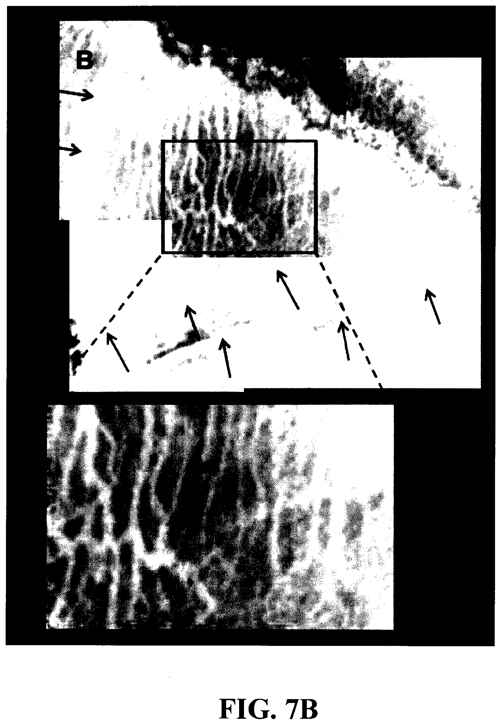

**FIG. 7B** shows that within these areas, if the gain (sensitivity) of the cSLO is increased, the choroidal blood vessels were readily evident. The box (enlarged) identifies some of the prominent choroidal vessels that travel in directions that are distinct from the retinal vasculature that run radially from the optic nerve (arrows). The speckled DNIRA fluorescence obscures the view to the choroid in the upper right of the image.

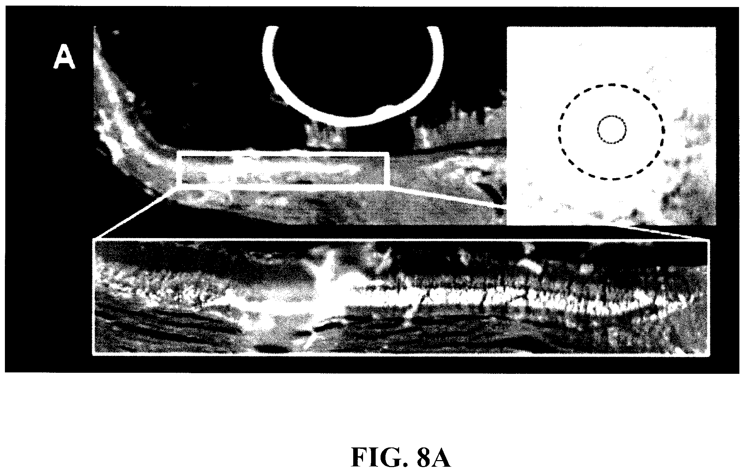

**FIGs. 8A-C** show that the reticular FAF pattern matures *in vivo* and in excised retina, and spares the area adjacent to the optic nerve.
**FIG. 8A** shows that despite significant outer retinal loss by month 3 after NaIO₃ administration, the ONL was preserved in the area immediately adjacent to the optic nerve head (ONH). Higher magnification (bottom), confirmed that the photoreceptor nuclei are preserved, and that the outer retina is not thrown into folds. FAF imaging of a representative eye from a Day 7 post-NaIO3 injection animal, confirmed that the reticular pattern is not seen. Fine dotted line (inner circle) represents the location of the ONH, while the coarse dotted line (outer circle) delineates approximate extent of normal, non-reticular FAF.

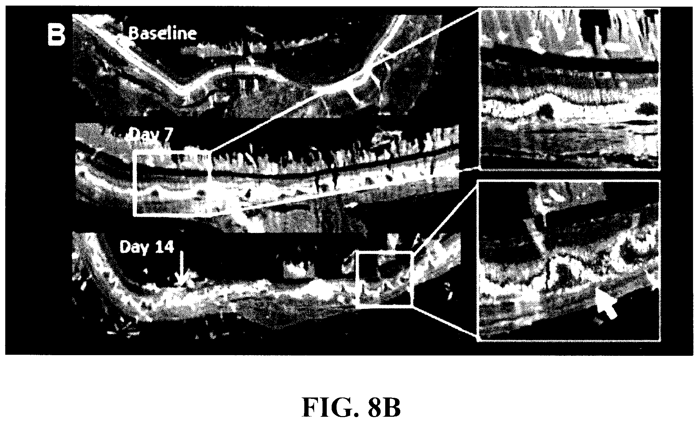

**FIG. 8B** shows H&E staining which demonstrated clear deformation of the outer retina following NaIO₃ administration. Prior to NaIO₃ administration (BL), the ONL appeared as a dark linear band that extended from the ONH to the retinal periphery. By 7 days, the outer retina was thrown into folds but the ONL remained grossly intact. This is seen in the enlarged figure in the right column. However, by day 14 post-NaIO3, the ONL showed areas of frank loss, with the inner retina lying directly against Bruch's Membrane (BM), the specialized basement membrane of the RPE. This is seen in the enlarged figure (black arrow).

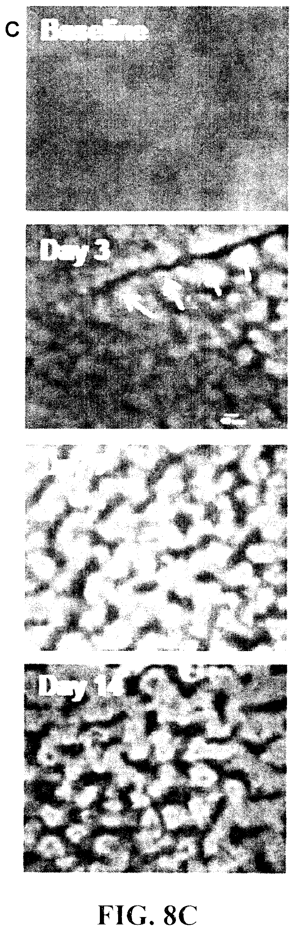

**FIG. 8C** shows confocal microscopy through the ONL imaged with the nuclear stain TO-PRO-3 (a carbocyanine monomer nucleic acid stain with far-red fluorescence only, Invitrogen). At baseline, this layer was flat, with no distinguishing features. With increasing time after NaIO₃ administration, the ONL became progressively more deranged. Linear grooves, curvilinear shapes, concentric rings, and isolated small ovals or circles were identified at days 3, 6 and 14.

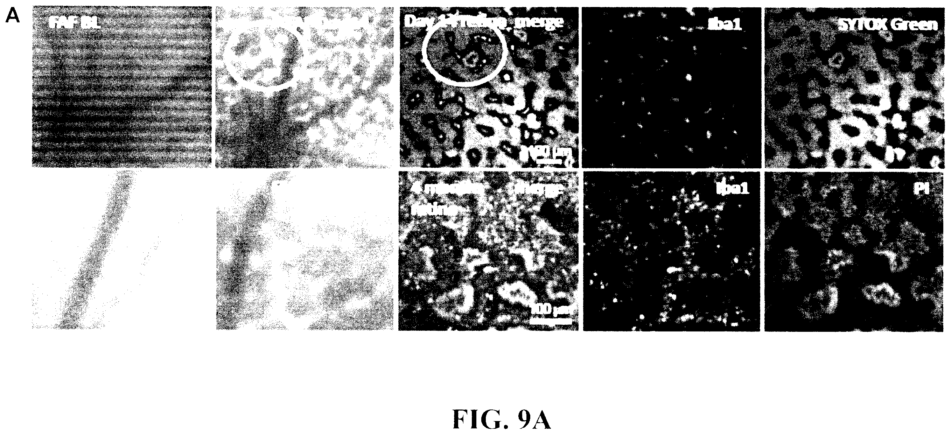

**FIGs. 9A-C** show that Iba1⁺ cells contribute to the reticular FAF pattern in the absence of RPE early and late in disease.
**FIG. 9A** shows lower (top row) and higher (bottom row) magnification of both *in vivo* and *ex vivo* imaging which displays the comparison between the reticular pattern seen on FAF and IHC early and late in disease progression. The pattern of FAF identified *in vivo* is recapitulated by fluorescent immunohistochemistry using anti-Iba antibodies. Compared against whole mount retina, excised immediately after HRAII imaging on Day 7, the same reticular pattern was seen using antibodies against Iba-1, a marker of microglia and macrophages (top). Nuclear staining using TO-PRO-3 showed densely packed cells of the outer nuclear layer (the photoreceptor layer) that appear, in two dimensions, to form curvilinear, oval or lobular patterns. Merging of the two confirmed that the Iba-1⁺ staining is found interlaced between the nuclear staining. Enlargement of late stage reticular pattern confirmed that Iba-1⁺ cells remained in the grooves between the folded photoreceptor nuclei (bottom).

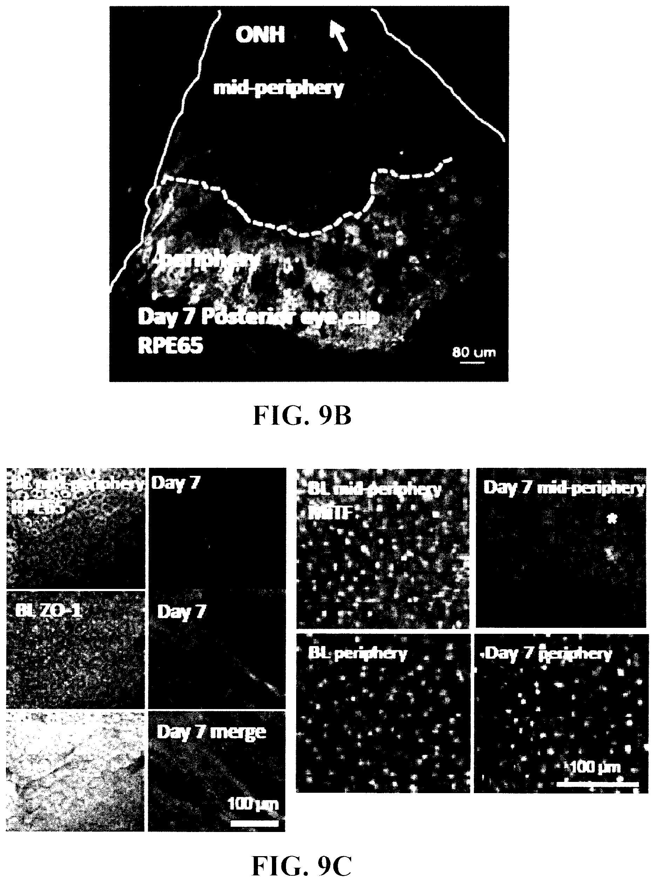

**FIG. 9B** shows that FAF signal can be detected even in the absence of RPE. Left: posterior eye cup, stained with RPE65 (green) shows loss of this monolayer in the central and mid retina (a ring of peripheral RPE remains. The remaining images utilized two different RPE labels (MiTF; microphthalmic transcription factor) and the characteristic double nuclear stain, and confirms loss of the RPE layer in the mid and central eyecup, in areas seen to have a complex pattern of fundus autofluorescence. By contrast, normal or near-normal RPE remains in the eyecup periphery.
**FIG. 9C** shows nuclear staining indicating the disappearance of RPE cells in the central part of a whole mount retina as evident by the absence of double nuclei which denote RPE cells. RPE was still visible in peripheral retinal tissue. This is in contrast to baseline control, where RPE cells were present in both central and peripheral retinal tissue.

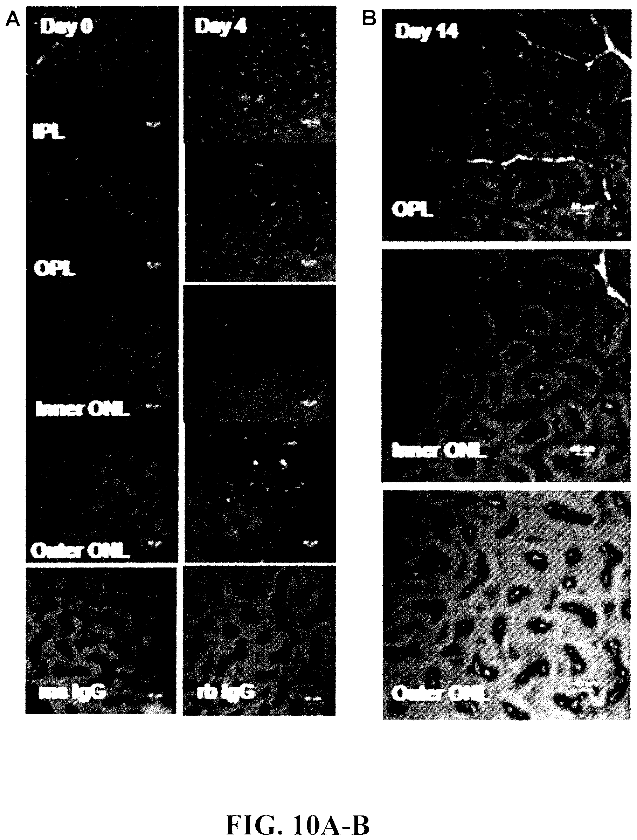

**FIGs. 10A-C** show serial confocal microscopy through the outer retina, which displays a complex 3-dimensional deformation with corresponding interlacing distribution of Iba-1⁺ and CD68⁺ phagocytic cells.
**FIG. 10A** shows morphological changes of the outer retina optically sectioned into four layers extending from the inner plexiform layer (IPL) to the subretinal space. The ONL is divided into inner and outer layers. Red = Iba-1, Green = CD68, Blue = nuclei. An increase in the number of phagocytic cells was seen in the retinal layers at Day 4 after NaIO₃ administration compared to baseline, at which time Iba-1 cells are found primarily in the inner retina. This was absent in antibody negative controls for both cell markers.
**FIG. 10B** shows that by day 14 after NaIO₃ administration, circular and oval patterns were evident in the ONL and as far internally as the OPL (identified by the presence of blood vessels). The pattern was curvilinear in the inner ONL, and more so in the outer ONL where the tightly folded layer of nuclei are much more dense, with smaller areas of curvilinear voids. In the outer ONL, there was significant bridging between folds. These nuclear voids contained Iba-1⁺ and CD68⁺ cells.

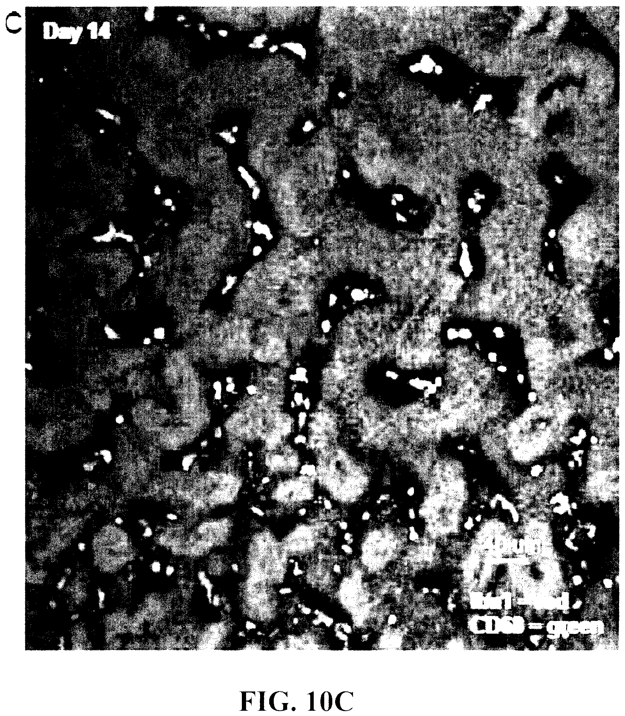

**FIG. 10C** shows an enlarged image in the outermost ONL or expanded subretinal space (which is normally a hypothetical space) shows a reticular pattern of Iba-1⁺ and CD68⁺ cells interlaced between photoreceptor nuclei.

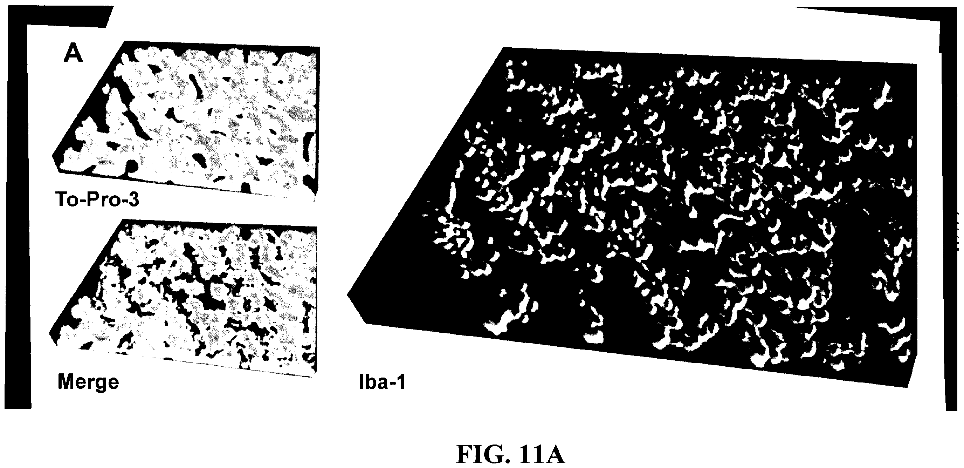

**FIGs. 11A-B** show three dimensional reconstruction of excised retinal whole mount tissue which confirmed that phagocytic cells lie within the deformed photoreceptor layer forming a reticular pattern
**FIG. 11A** shows confocal Z-series through the outer retina were used to generate a three dimensional reconstruction of the ONL and Iba1⁺ cells. Nuclear staining (red) of the photoreceptor nuclei layer showed that it is thrown into folds. Merged with Iba-1⁺ staining (green), it was evident that inflammatory cells lie between or within folds defined by retina deformation. Reconstruction of Iba-1 positive cells alone demonstrated the reticular pattern.

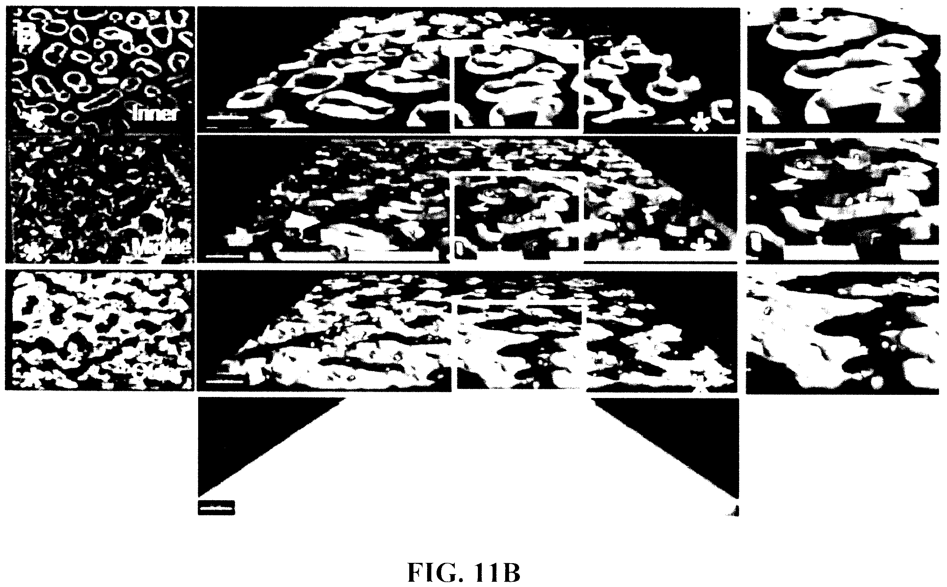

**FIG. 11B** shows Z-series reconstruction of segments of the total image stack of the outer retina 28 days after NaIO₃ administration illustrates the complexity of the folds, that are more narrow at their peaks (inner), and broader and more interlacing at their bases (middle, outer). For comparison with cross-sectional imaging, and to better represent the 3D nature of these findings, an oblique perspective is also shown (middle panels). The position of the asterisk on the *en face* images (left) corresponded with the location of the asterisk in the oblique images. Digital enlargements (right) showed a groove between the photoreceptor layer (red) that is curvilinear and contiguous in the outer retina, but broken into at least three smaller ovals in the inner-ONL. Iba1⁺ cells (green) were largely located within, *i.e.* external to, the photoreceptor layer. Control reconstruction of the outer retina showed neither Iba-1 positive cells nor deformation of the ONL (bottom row). Bitplane software (Imaris) was used to generate these images.

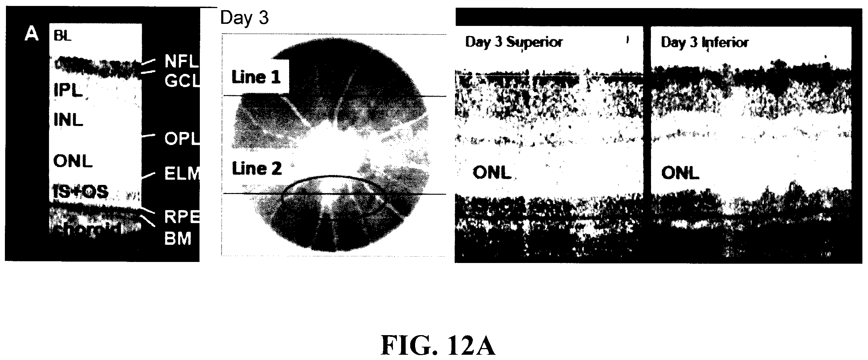

**FIGs. 12A-D** shows that progressive NaIO₃-induced outer retinal deformation was identified by OCT imaging *in vivo* and compared to clinical findings (abbreviations: NFL = nerve fiber layer; GCL = ganglion cell layer; IPL = inner plexiform layer; INL = inner nuclear layer; OPL = outer plexiform layer; ONL = outer nuclear layer; IS+OS inner segment & outer segment layer; RPE = retinal pigment epithelium; BM = Bruch's membrane).
**FIG. 12A** shows *in vivo* OCT and illustrates early changes that occurred in the inferior retina, but not the superior retina, were observed in the first three days following NaIO₃ administration. Baseline image indicated the retinal layers visible on OCT. *En face* image of the retina at day 3 illustrated the position of the two optical sections, with superior and inferior portions shown. The superior portion was unchanged compared to baseline. By contrast the inferior showed small undulations of the photoreceptor inner and outer segments, along with loss of the thin RPE layer.

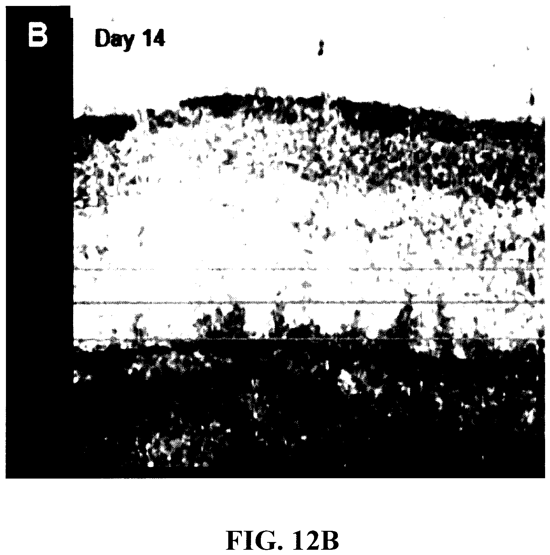

**FIG. 12B** shows that by Day 14 after NaIO₃ administration, the outer retina showed marked changes including the formation of multiple curved folds, base-down triangles and occasional spikes of bright signal that lie external (inferior) to the dark outer nuclear layer.

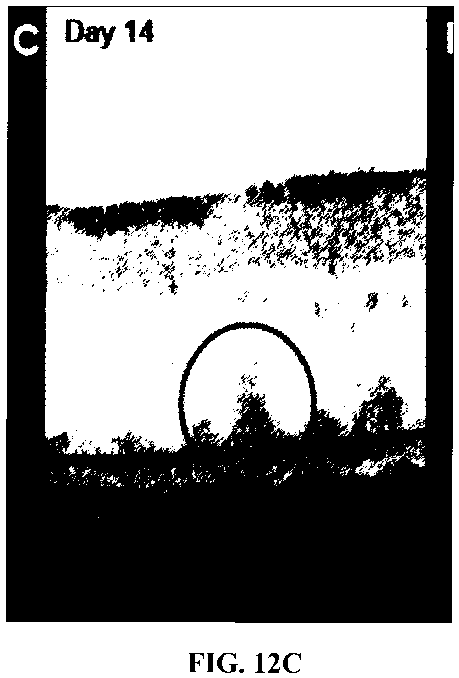

**FIG. 12C** shows a well-formed base-down triangle is indicated in a day 14 image of the rodent retina.

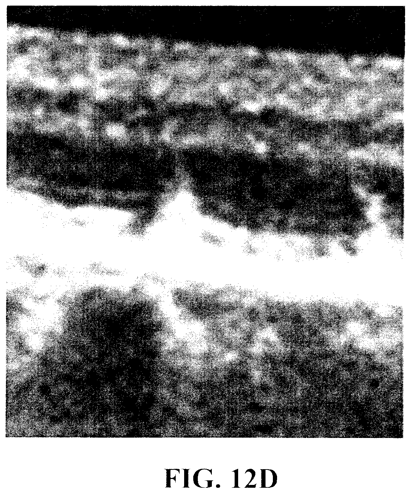

**FIG. 12D** shows OCT image of a patient with reticular pseudodrusen showing a similar base-down triangle as that seen in the rodent model.

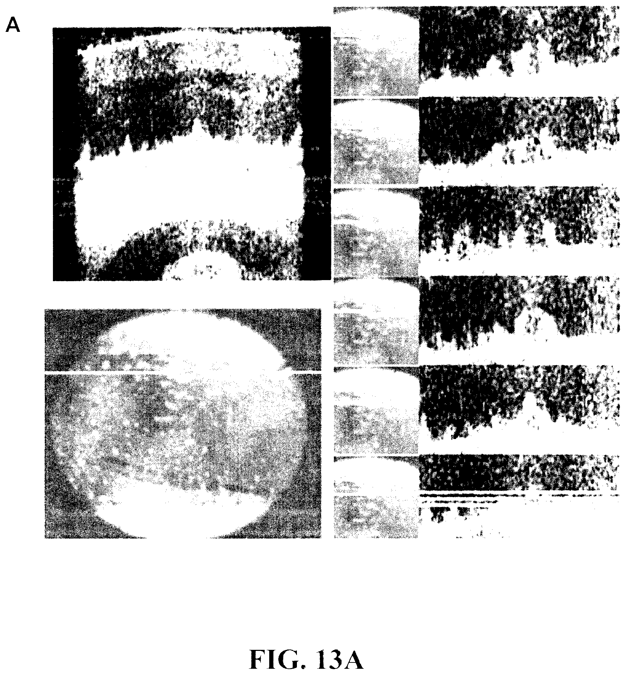

**FIGs. 13A-B** shows that subretinal changes contributed to complex 3D structures in the Y-axis and Z-axis *in vivo.*
**FIG. 13A** shows adjacent, consecutive OCT images which confirm that subretinal changes contribute to complex 3D structures in the Y-axis. Small subretinal domes or pyramidal shapes are plentiful, and as shown centrally, also formed a shape similar to an upside down "Y" or "wishbone". Compared against the volumetric reconstruction of serial images this image coincides with optical sections through a structure that appears circular when viewed *en face.* Images showing the position of serial OCT sections through the circular structure correspond to those in the adjacent optical cross-sections. The two major subretinal signals of these cross-sections (arrows) are seen to be close together at the upper and lower extremities of the circle, and further apart in the center. The inter-spike distances are provided. Evaluation of the structures immediately left of the circle show a similar contribution of subretinal structures to smaller loop-like structures.

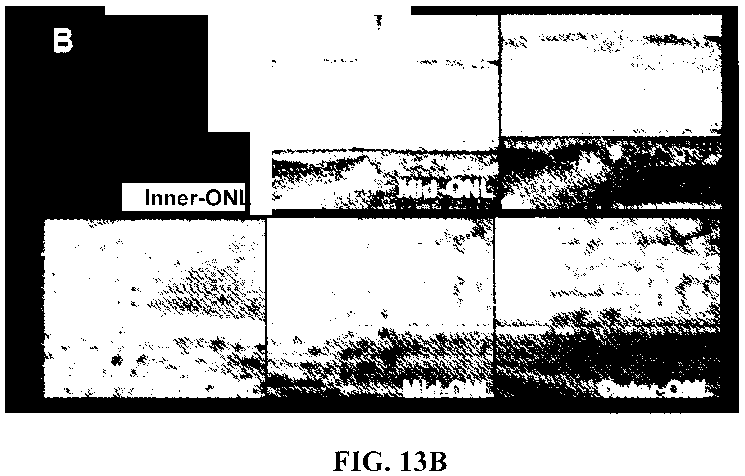

**FIG. 13B** shows volumetric reconstructions of segmental stacks of serial Z-sections through the inner, mid, and outer ONL confirm the different *en face* appearance of signal under the deformed outer retina. OCT sections through the same tissue plane. (Upper Row) Volumetrically reconstructed images formed within the three planes bounded by the parallel green lines shown in upper row. Such segmental reconstruction through the inner, mid and outer-ONL demonstrates three distinct patterns. (C. lower row) Regular array of punctate spots in the inner ONL. (C, lower left) More curvilinear pattern in the mid-ONL. (C, middle) Outer-most ONL, largely within the expanded subretinal space, shows multiple complex curvilinear, looping, complex structures with significant bridging between them. These images correlate to the 3D reconstructed confocal microscopy findings.(C, lower right)

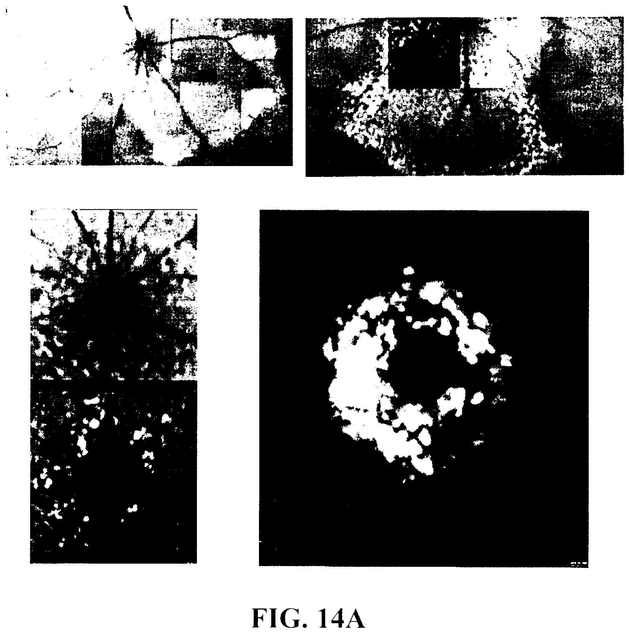

**FIGs. 14A-B** show that gadolinium, which depletes circulating monocytes and resident macrophages alters the FAF pattern of NaIO₃-induced outer retinal damage.
**FIG. 14A** shows how, compared to untreated (bottom panel), GAD treated lesion have less well-demarcated borders at Day 3 (top, left) and far less well demarcated patterns of FAF within the representative island of damage at Day (top, right).

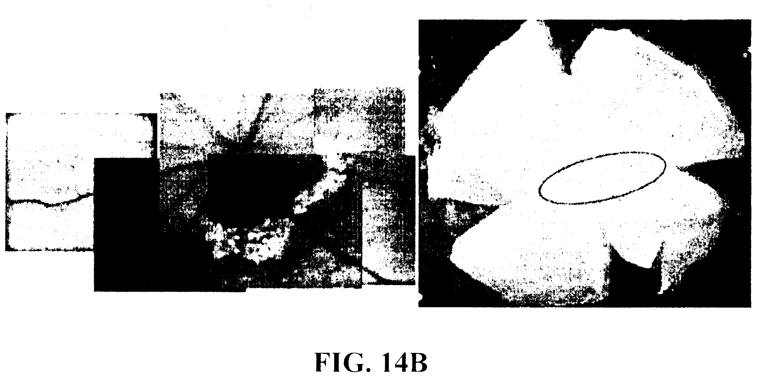

**FIG. 14B** shows how macrophage depletion leads to smaller areas of damage. Left: F AF image shows small crescent of reticular pattern 7 days after NaIO₃ and simultaneous GAD depletion of the macrophages. Right: Relative to the whole excised retina, the area of damage is clearly smaller that typically seen.

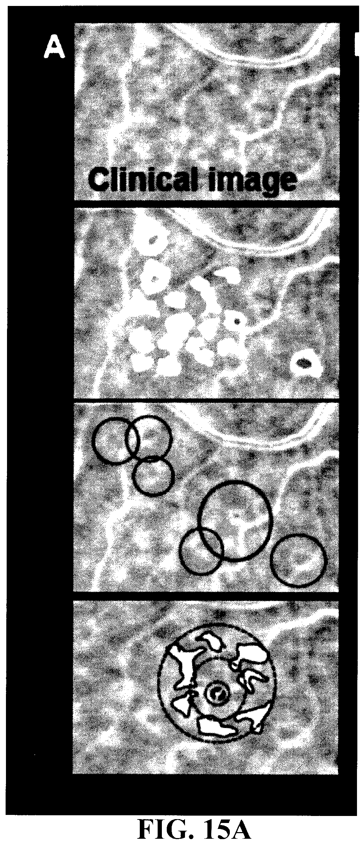

**FIGs. 15A-C** show interpretative illustrations of how, without wishing to be bound by theory, histologically-determined tissue findings can account for *in vivo* imaging following NaIO₃-induced RPE loss.
**FIG. 15A** shows clinical images from a patient with early AMD showing reticular pseudodrusen and so-called "target" lesions in the NIR channel (top). Immediately below are two schematic overlays that show the hypofluorescent spots (those typically defined as drusenoid deposits) first in black for emphasis, and then with a bright background to emphasis the intervening bright signal. The bottom two images highlight multiple small target lesions of RPD (within the circles), while the bottom-most schematic, illustrates the presence of a potential halo and target lesion formed by concentric folds of tissue.

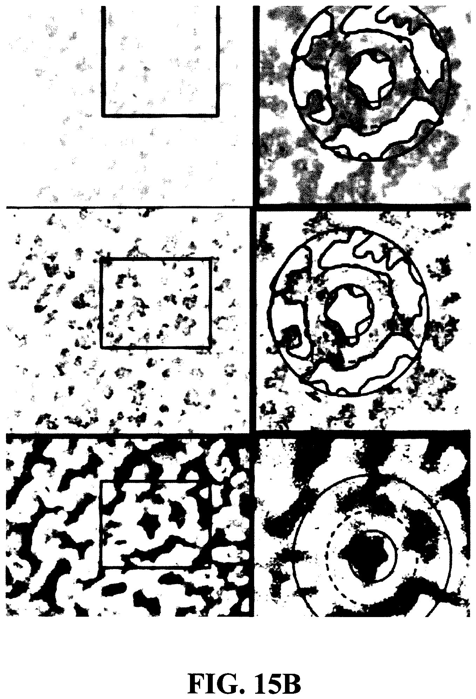

**FIG. 15B** shows schematic renderings suggest a direct relationship between three-dimensional whole mount ONL histology and two-dimensional *en face* clinical FAF images. By day 14 after NaIO₃ administration, the outer retina shows complex folds of the mid-ONL (shown at relative low magnification [original 40x] and digitally enlarged). Digital magnification shows concentric rings of tissue folds. This same area illustrated in black and white showed how this might appear on FAF to be a circular or oval region with a central void. Reversed white & black rendering (*i.e*., a "negative" image), showed that this arrangement of ONL distortion would appear to have a central bright target.

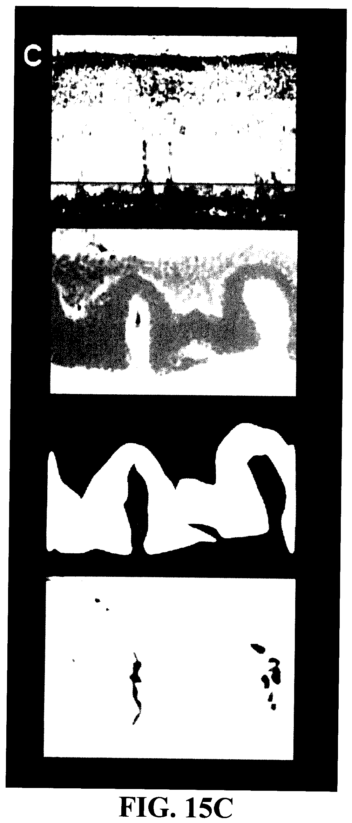

**FIG. 15C** shows OCT imaging of the rat retina 14 days after NaIO₃ administration clearly shows bright "spikes" through the outer retina. These are described clinically. Similar histological sections through the outer retina demonstrate ONL folds with underlying inflammatory cells in the enlarged subretinal space that form narrow columns of cells. For comparison with *in vivo* images, these are rendered in black and white. In reversed black and white (*i.e*., a pseudo-negative image to match OCT images), it is seen that the ONL disappears while the subretinal densities appear bright.

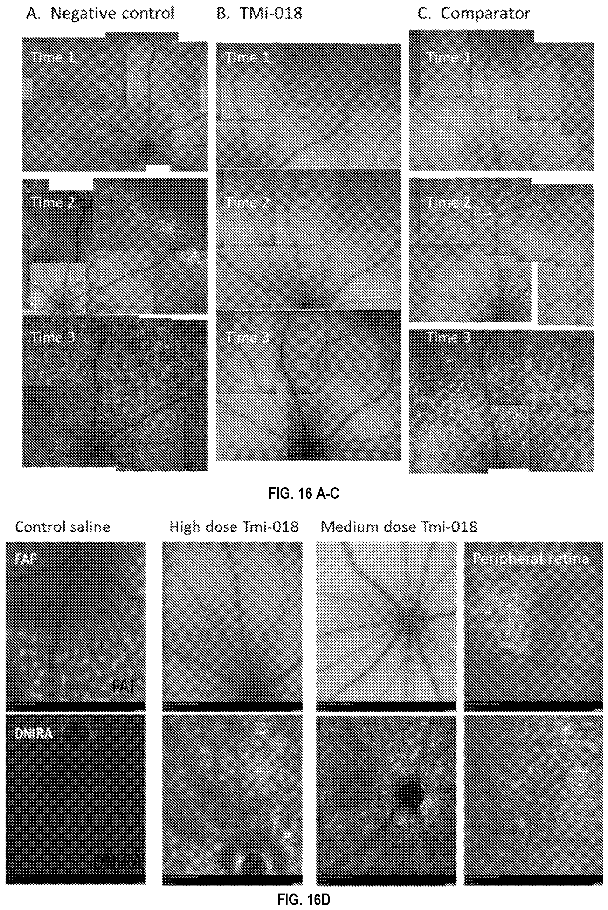

**FIGs. 16A-F** show that, when evaluated over time using Fundus Autofluorescence (FAF), intra-vitreal bindarit reduces the development of complex patterns of hyperfluorescent FAF.
**FIG. 16A** is a negative control samples which received saline and developed progressive change evaluated using FAF.
**FIG. 16B** shows that at high dose (20 µg per eye) bindarit, the FAF remains normal.
**FIG.16C** shows that abnormal FAF develops in the presence of a comparator small molecule.
**FIG. 16D** shows that FAF (upper row), combined with DNIRA imaging (lower row), confirms preservation of the RPE layer. Control animals (left) develop complex FAF patterns and dark DNIRA signal (absent RPE). At high dose TMi-018 (20 ug per eye), the FAF is normal and DNIRA signal preserved. At medium dose (10 ug per eye), there are small patches of abnormal FAF and RPE loss.

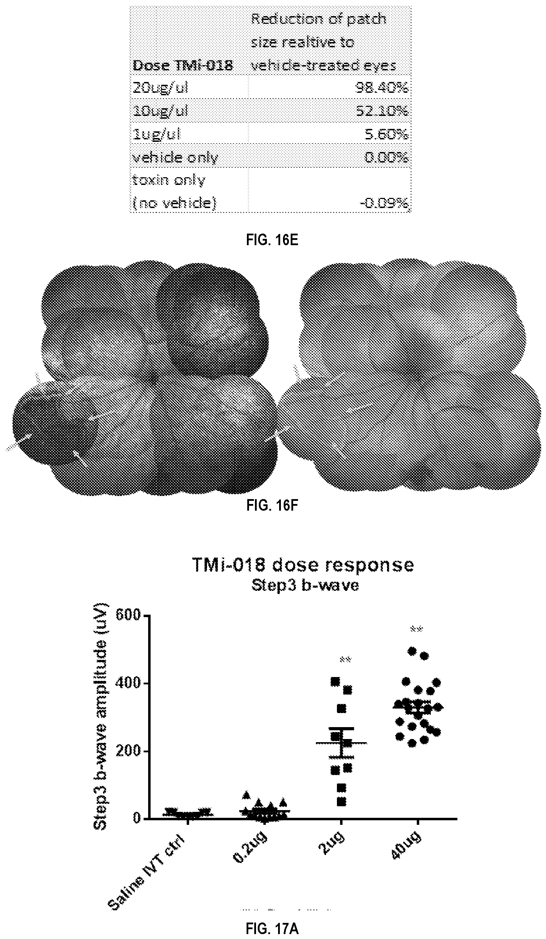

**FIG. 16E** shows that intra-vitreal bindarit dose-dependently reduces patch size. Various doses are used: 20 ug/ml, 10 ug/ml, 1 ug/ml, and controls (vehicle only and toxin only).
**FIG. 16F** shows DNIRA (left) and FAF (right) representative images of a small patch of damage that occurs with high-dose NaIO₃ toxin in the presence of 1 mg/ml (2 ug) of bindarit, given intra-vitreally.
**FIGs. 17A-C** shows that bindarit dose-dependently protects retinal function (electrophysiology), and demonstrates a minimally-effective dose.
**FIG. 17A** shows electroretinogram analysis demonstrates dose-dependent protection of retinal function. Does are amounts of bindarit: 40 ug, 2 ug, and 0.2 ug. The minimally effective dose (MED), below which bindarit's effect is lost was confirmed to be 0.1 mg/ml, or 200ng per eye.

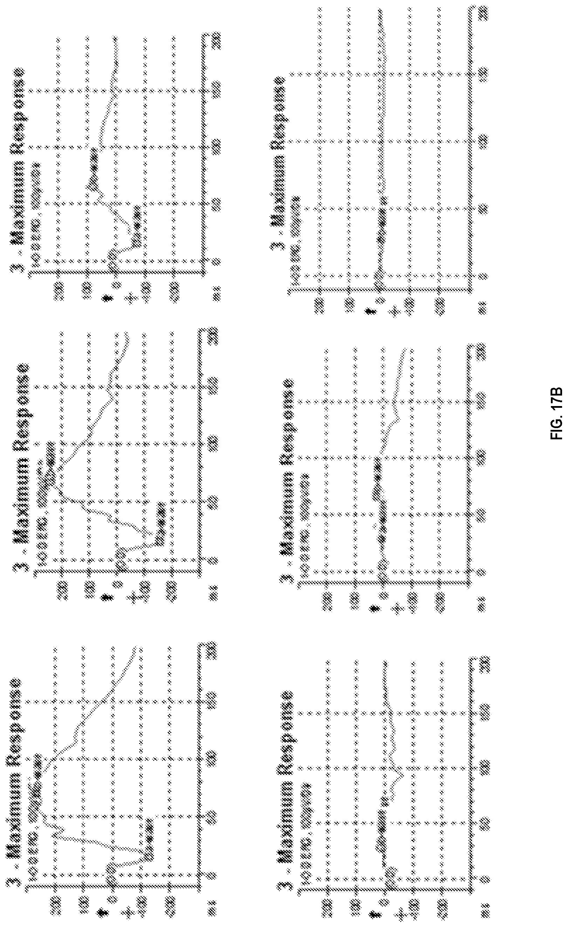

**FIG. 17B** shows electroretinogram traces. A bright-flash b-wave amplitude is preserved at high dose, and extinguished at low dose and in the presence of saline control.

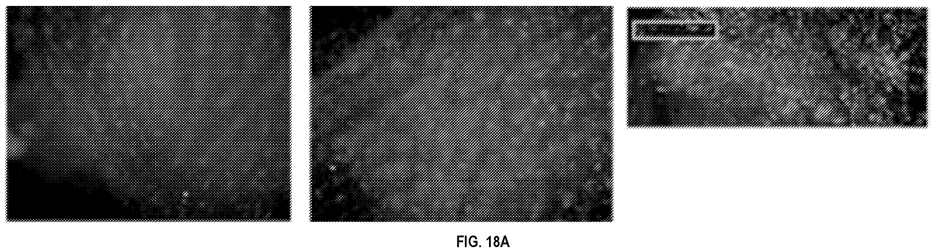

**FIG. 18A-C** shows that bindarit protects against tissue damage, RPE loss and disruption of the outer retina.
**FIG. 18A** shows that bindarit dose-dependently protects against tissue damage, evaluated using autofluorescent epifluorescent microscopy at 488/512 nm. Small cuboidal RPE cells are seen in both the very high (10 mg/kg) and high doses (50 mg/kg) (left and middle) bindarit arms. Cellular debris and enlarged hyperfluorescent RPE cells are seen in the saline control (right).

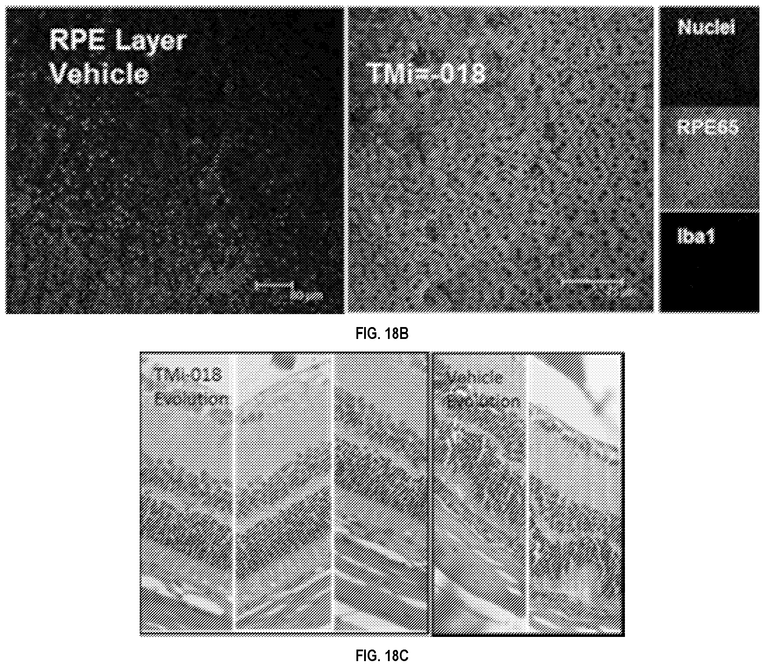

**FIG. 18B** shows intra-vitreal bindarit protects against RPE cell loss. In the presence of intra-vitreal saline (vehicle), RPE cells are lost and confirmed by fluorescent immunohistochemistry (left). In the presence of intra-vitreal bindarit, RPE cells are preserved. Green = RPE65; red = Iba1; blue = nuclei
**FIG. 18C** shows H&E histology confirms preservation of the RPE layer and outer retina in the presence of bindarit (left two images). These layers are damaged or lost in the presence of saline (vehicle).

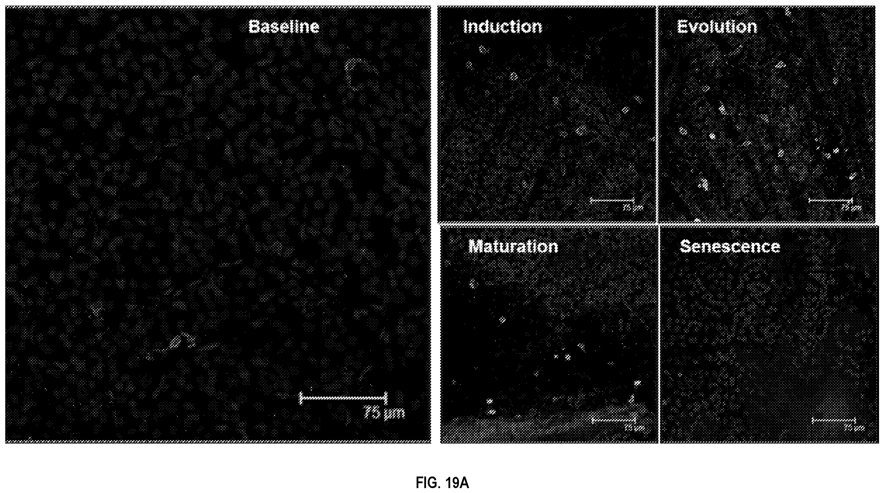

**FIGs. 19A-H** shows that bindarit reduces Monocyte Chemoattractant protein (MCP)-1 expression and reduces the M1 macrophage response, tipping the balance in favor of M2.
**FIG. 19A** shows fluorescent immunohistochemistry. In the model of AMD/RPD described herein, toxin-induced damage to the RPE leads to a sequence of change in polarization of the innate immune system. Immunohistochemistry confirms that at baseline, markers of the M2 response (*e.g*. CD163 and others) are predominant. After induction of damage, there is a large increase in M1 gene expression that returns to baseline after evolution and maturation give way to disease senescence. Red = iNOS (M1), Green = CD163 (M2), Blue = nuclei, Original magnification: 100x.

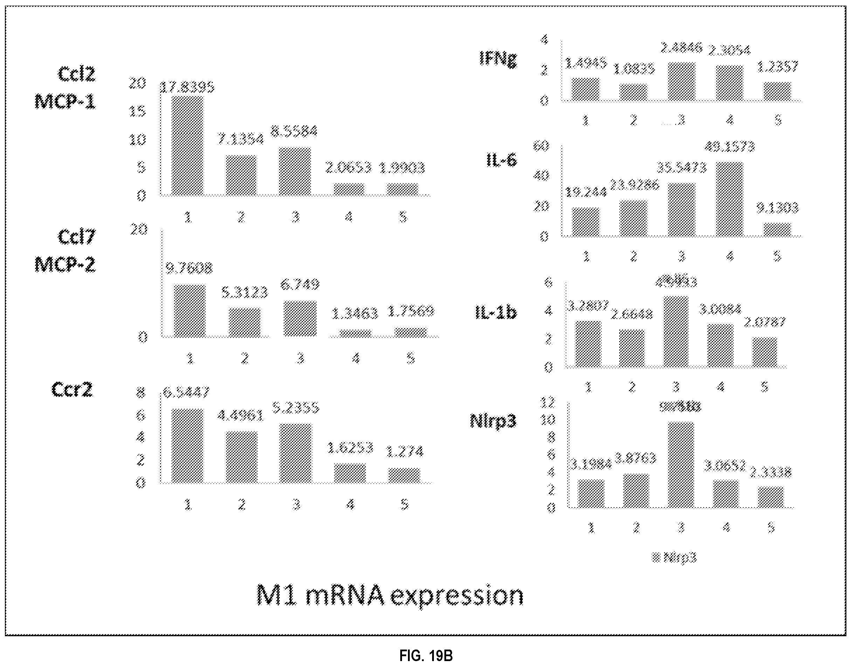

**FIG. 19B** shows quantitative PCR confirms decrease in M1 gene expression (mRNA) with little change in M2 expression. Fold-expression of mRNA transcripts are calculated relative to baseline (pre-damage) levels. Numbers 1-5 on x-axis represent time course of change as follows: 1 = induction, 2 = early evolution, 3 = late evolution, 4 = maturation, and 5 = senescence. M1-specific markers are evaluated in this figure.

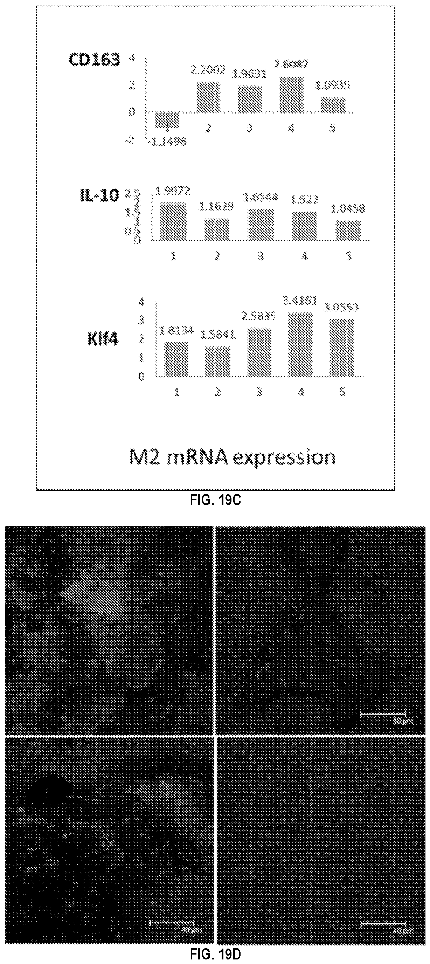

**FIG. 19C** shows quantitative PCR confirms decrease in M1 gene expression (mRNA) with little change in M2 expression. Fold-expression of mRNA transcripts are calculated relative to baseline (pre-damage) levels. Numbers 1-5 on x-axis represent time course of change as follows: 1 = induction, 2 = early evolution, 3 = late evolution, 4 = maturation, and 5 = senescence. M2-specific markers are evaluated in this figure.
**FIG. 19D** shows epifluorescent IHC which qualitatively demonstrates a dose-dependent reduction in MCP-1 protein expression in the subretinal space following intra-vitreal bindarit administration. MCP-1 (red) & CD163 (green); nuclei blue. Original magnification, 10x. Upper left panel is saline (negative control); upper right panel is low dose bindarit (1 mg/kg); lower left panel is high dose bindarit (10 mg/kg); and Lower right panel is normal (no toxin).

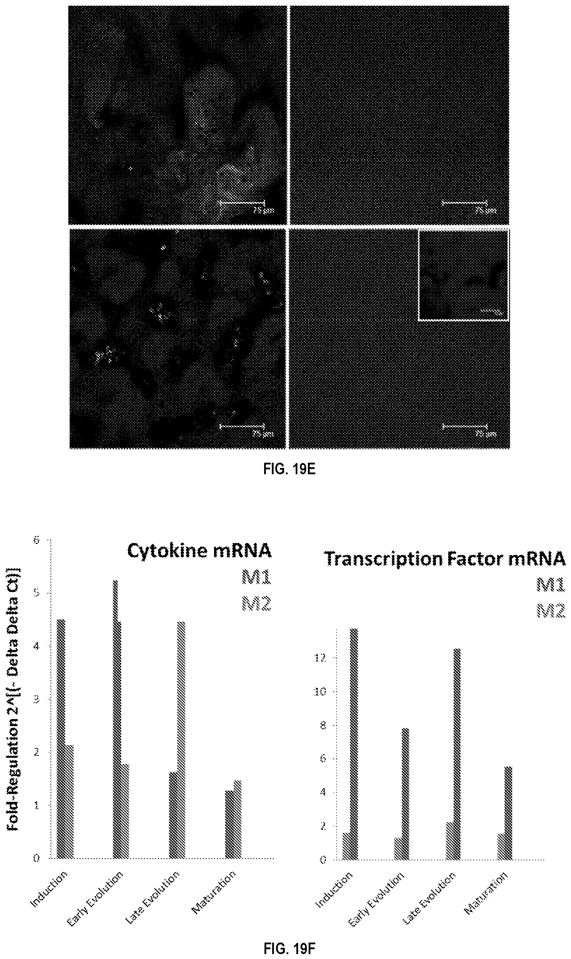

**FIG. 19E** shows that epifluorescent IHC qualitatively demonstrates a dose-dependent reduction in TNF-α and CD68 protein expression in the subretinal space following intra-vitreal bindarit. TNF α (red) & CD68 (green); nuclei blue. Original magnification, 10x. Upper left panel is saline (negative control); upper right panel is low dose bindarit (1 mg/kg); lower left panel is high dose bindarit (10 mg/kg); and Lower right panel is normal (no toxin).
**FIG. 19F** shows that intra-vitreal bindarit reduces M1-related mRNA transcription. Compared against baseline, induction and early evolution of RPE damage and highly associated with M1 cytokine expression; this tapers off during late evolution and maturation. By contrast, M2 mRNA rises later in disease as M1 levels are reduced. A change of "1" represents no change from baseline, biologically significant changes may, in some cases, be said to exist when the fold-change exceeds 2 or 3. Similar observations are made regarding M1 and M2 related transcription factors. M1 transcripts predominate during disease, with little change in M2. In the left panel (cytokine mRNA), for each time point the M1 samples are the left bar and the M2 samples are the right bar. In the right panel (transcription factor mRNA), for each time point the M1 samples are the right bar and the M2 samples are the left bar.

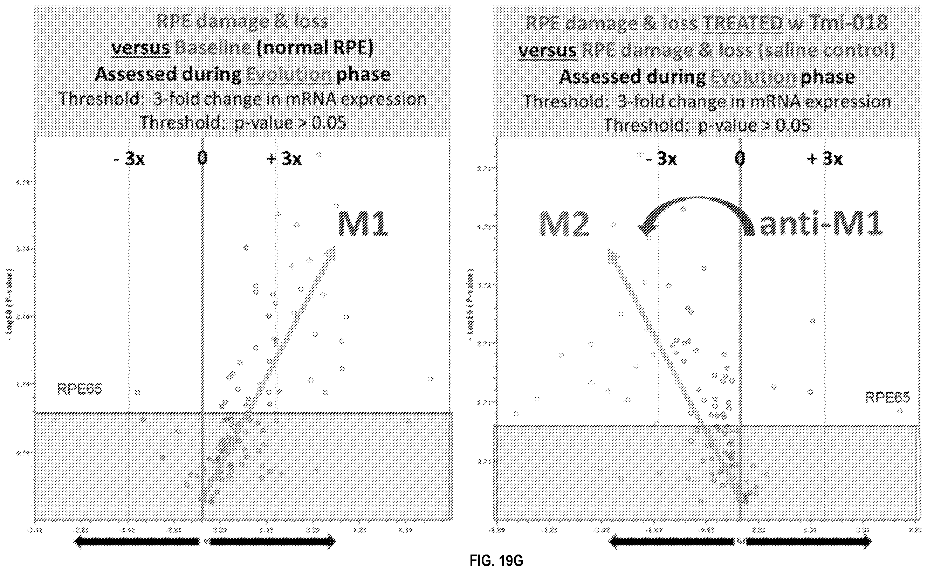

**FIG. 19G** a schematic of the shift in M1 vs M2 gene expression. Following toxin-induced damage, gene expression shifts towards M1; in the presence of bindarit (TMi-018), gene expression shits back to neutral (*i.e*. towards M2). RPE65 gene transcripts are significantly decreased during disease consistent with observed cell loss. By comparison, bindarit reduces this loss.

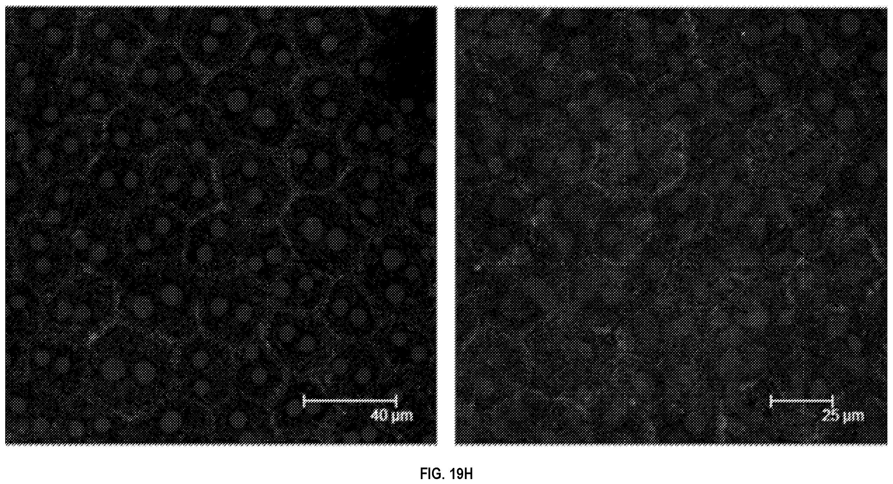

**FIG. 19H** shows bindarit (TMi-018) does not significantly reduce the M2 response in the absence of toxin-induced RPE damage, at the protein level. Fluorescent immunohistochemistry shows that despite high-dose intra-vitreal bindarit, the protein levels of MCP-1, a key target, there is little change between saline and drug-treated eyes. These data show that that bindarit differentially regulates baseline or "normal" levels of MCP-1 expression, and may act to differentially reduce "inflammation-associated" transcription. Such differential activity is consistent with bindarit's effect to differentially use the 5' upstream promoter and enhancer activity in the MCP gene complex. MCP-1 = red, Nuclei = blue.

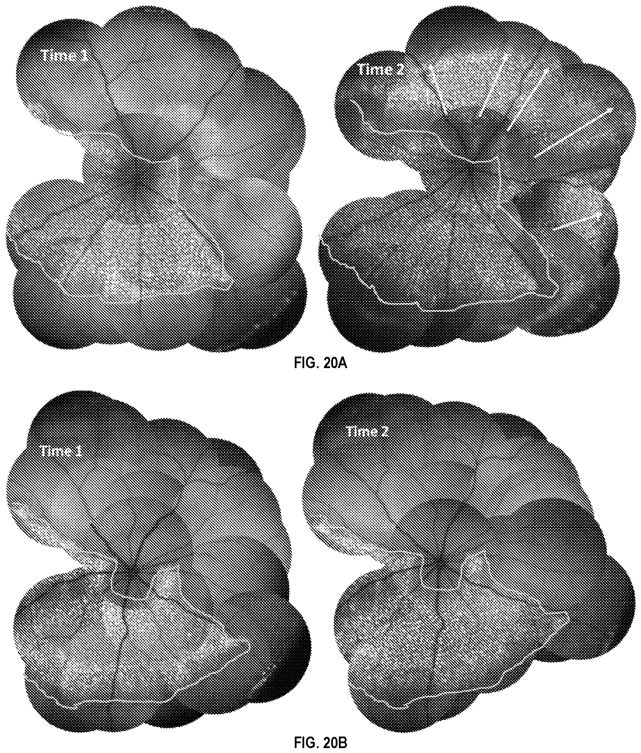

**FIGs. 20A-C** show that bindarit reduces patch expansion (as opposed to patch prevention). In addition to preventing GA formation, bindarit treats pre-existent GA, by reducing the growth of these patches of RPE and outer retinal loss. The goal of such treatment is to reduce the size of the "blind spot" that the patient observes in their central field of vision.
**FIG. 20A** shows a negative control (saline) treatment of an existing patch, which expands.
**FIG. 20B** shows bindarit treatment of an existing patch, which does not expand.

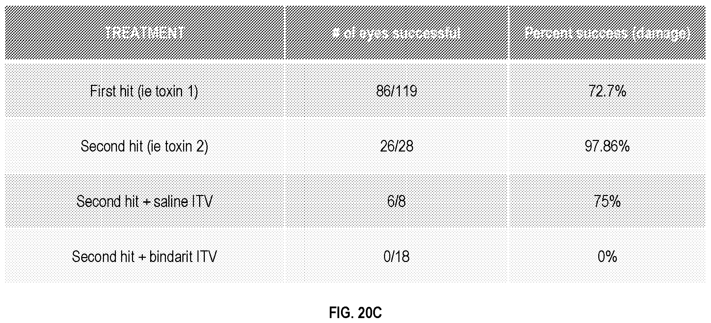

**FIG. 20C** summarizes data for patch expansion ("success" in this context is patch expansion). A hit is a challenge with a toxin. The data show that the experimental model can cause patch expansion with toxins and with saline, but such expansion is prevented with bindarit ("TMi"). "IVT" is intra-vitreal.

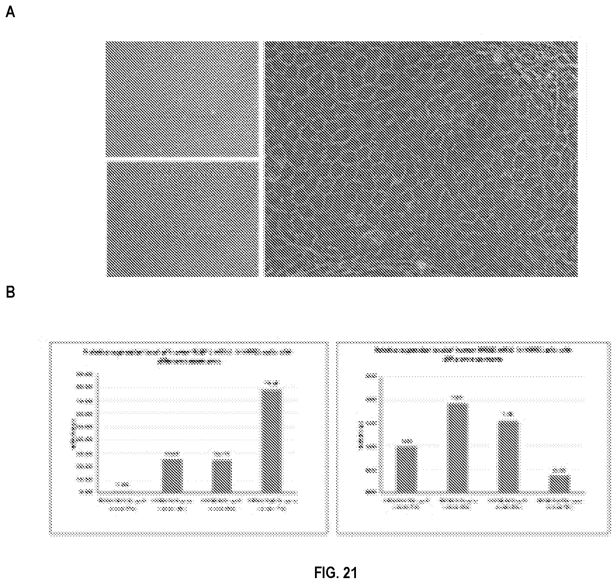

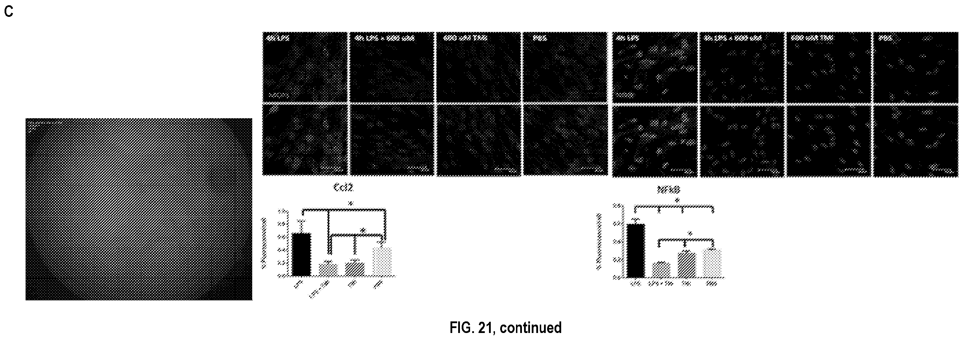

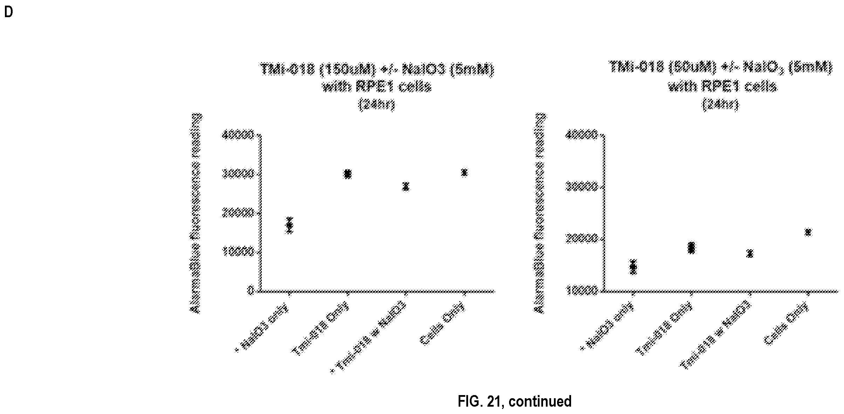

**FIG. 21** shows, in panels A and B, that hTERT, an immortalized (telomerase deficient) human RPE cell line, are confirmed to express markers of RPE differentiation. In panel C, it is shown that TMi-018 reduces NFkb expression, NFkb translocation to the nucleus, and reduces MCP-1 in hTERT. Panel D shows that TMi-018 directly increases RPE survival against toxin challenge *in vitro.*

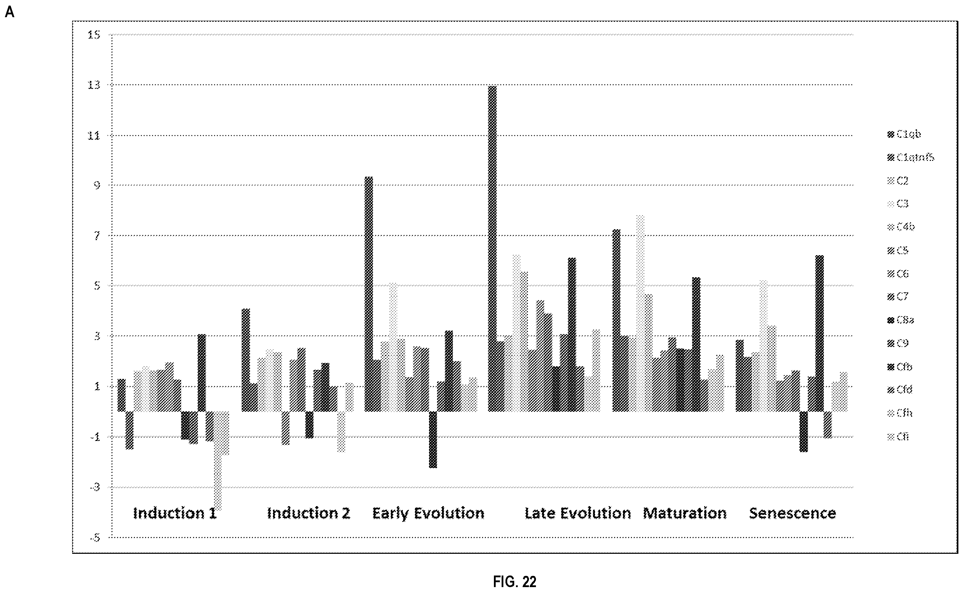

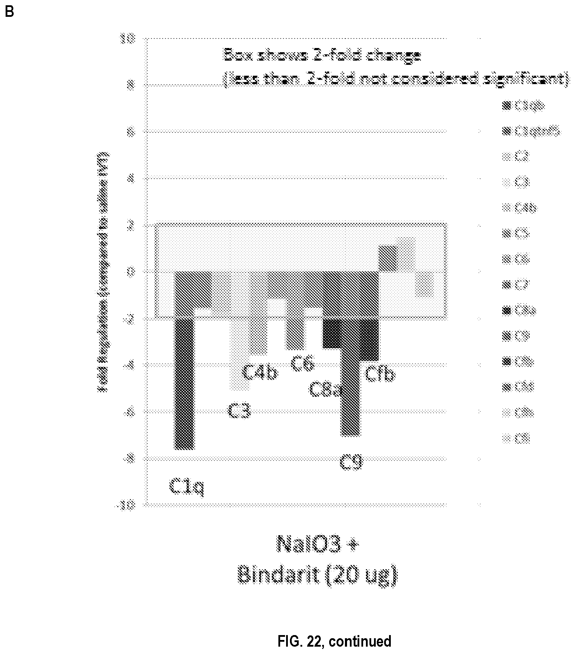

**FIG. 22** shows, in panel A, that mRNA species of the complement cascade components change following toxin-induced RPE damage and, in panel B, bindarit alters complement mRNA expression. For each set of histograms, the following genes are assessed (from left to right): C1qb, C1qtnf5, C2, C3, C4b, C5, C6, C7, C8a, C9, Cfb, Cfd, Cfh, and Cfi. Fold expression less than 2 is considered non-significant. Protein levels were not analysed.

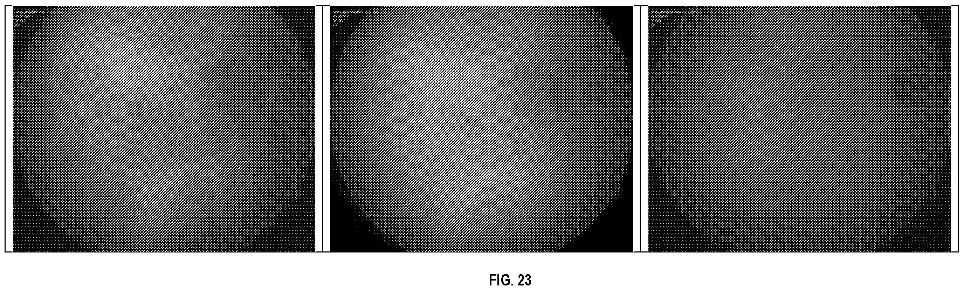

**FIG. 23** shows ICG angiography of a patient with "dry" AMD. The left panel shows early stage (transit phase) ICG angiography of a patient with dry AMD and identifies normal large blood vessels of the choroid (the richly vascular bed external [deep] to the retina). The center panel shows mid-stage ICG angiography of the same patient and identifies small central areas that subtley block the underlying fluorescence. These correspond to regions of abnormal pigmentation seen on color photography (data not shown). The right panel shows late-stage angiography and demonstrates a generalized dim flush of the choroidal tissue as ICG leaks from the blood vessels into the tissue. Other than overlying blockage of the central signal (barely detectable) little abnormality is observed.

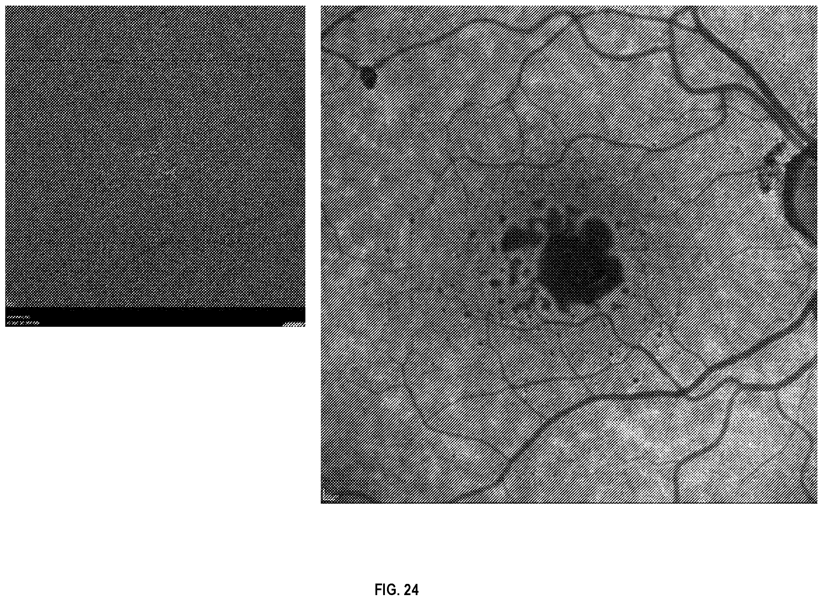

**FIG. 24****,** by contrast to **FIG. 23****,** shows DNIRA imaging of the same patient with dry AMD taken two days after systemic injection of ICG dye, with no further injection In the left panel, baseline DNIRA image of the patient in **FIG. 23** and shows neglible signal with the ICG excitation/emission filters in place, but prior to injection of ICG. The right panel shows a day 2 DNIRA image from the same patient and identifies areas of marked hypofluorescence in the central macula. Images were obtained with the ex/em filters in place but no further injection of dye (since baseline several days earlier). In addition, a leopard-like or lacy pattern is observed in the peripheral macula that has not been identified by any imaging technique to date.

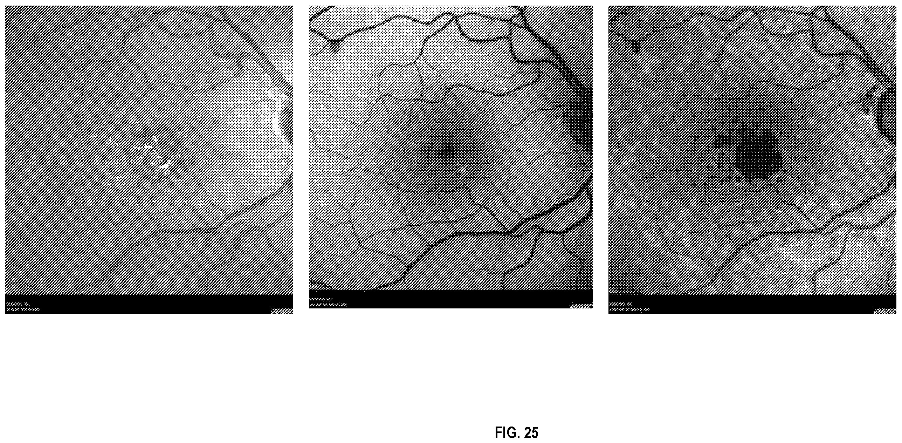

**FIG. 25** shows DNIRA images as compared against FAF and infra-red (IR). In the left panel InfraRed imaging of the fundus of a patient with dry AMD and shows areas of slightly hyper- and hypo-fluorescence. Bright areas reflect the presence of preumptive crystalline deposits (observed by clinical examination). The center panel shows Fundus Autofluorescence imaging of the same patient and shows a normal ground-glass appearance throughout the macula that is blocked by overlying blood vessels, is absent at the optic nerve (far right of image) and shows a gradual reduction toward the center. The righ panel shows DNIRA images of the same patient and contrasts markedly with FAF and IR image. Of note, a region of profound hypofluorescence (dark) is noted centrally. Smaller patches are noted at the margin of central lesion and in proximity to the optic nerve head.

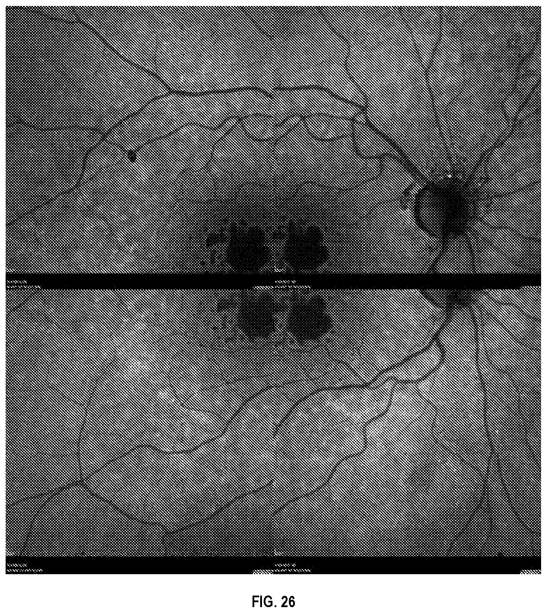

**FIG. 26** shows that composite DNIRA images identify novel changes in the mid- and peripheral macula. Composite images of the same patient shows a lacy, reticular or leopard-like pattern of alternating hyper- and hypo-fluorescent DNIRA that is not seen in other imaging modalities, and not described previously (top panel).

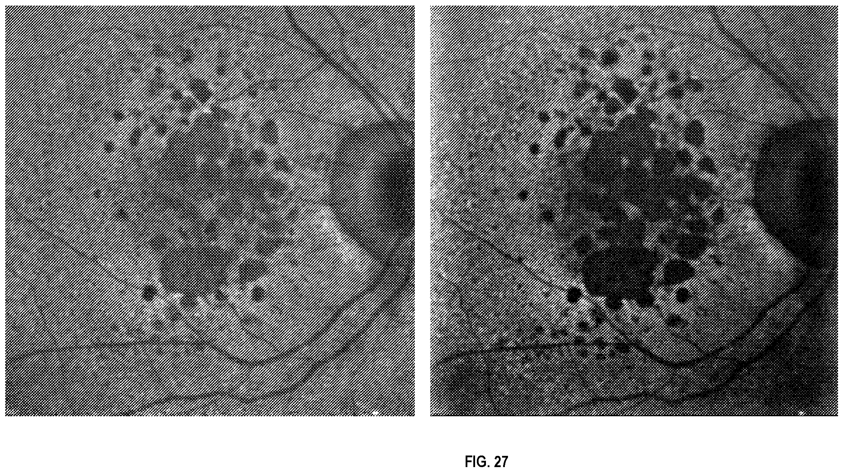

**FIG. 27** shows repeated imaging of the same patient over time and indicates that the DNIRA pattern also changes, indicating that DNIRA is a novel marker of disease progression. The left panel shows DNIRA images of this patient with late dry AMD at time 0 and show regions of hypofluorescence (and hyperfluorescence). The right panel shows repeated DNIRA imaging of the same patient, six months later, and shows that regions of hypofluroescent DNIRA signal have expanded.

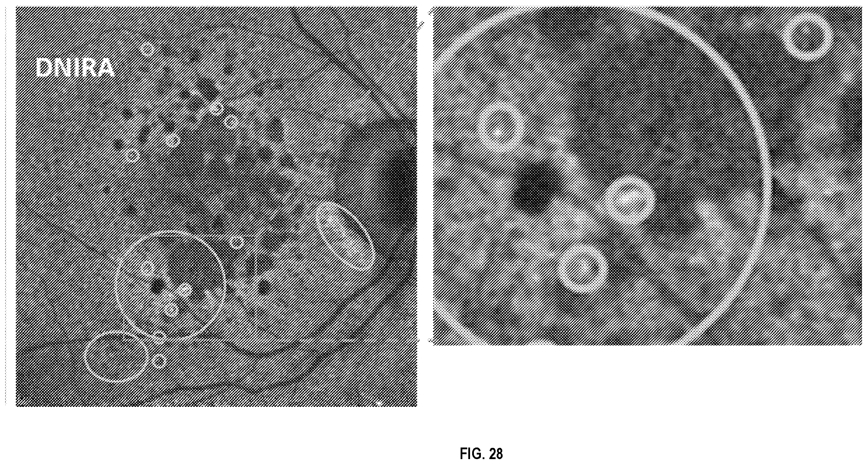

**FIG. 28** shows DNIRA image of a patient with dry AMD identifies discrete bright punctate spots of hyperfluorescence. Discrete dots of hyperfluorescent DNIRA are identified scattered throughout the macula of patients with AMD. Some are in proximity to the optic nerve head, and other immediately adjacent to regions of geographic atrophy, both at the border and in the so-called junctional zone. The right panel is a magnified view of part of the left panel.

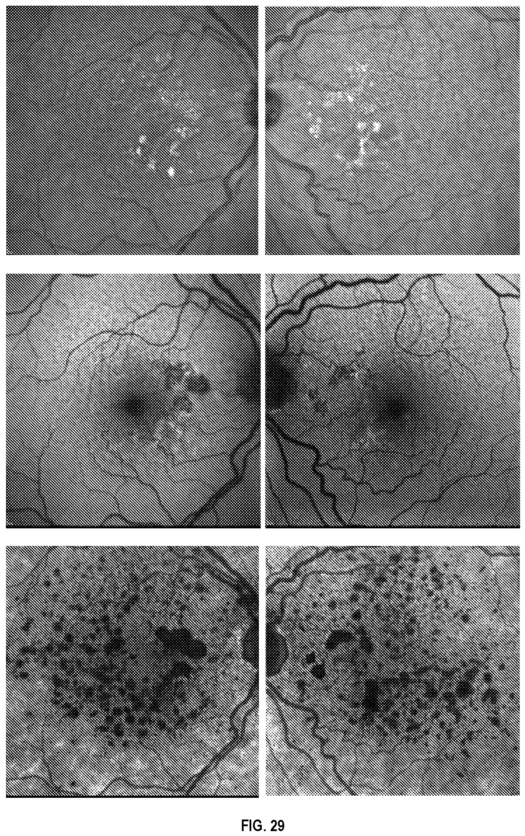

**FIG. 29** shows DNIRA imagining of a human patient with RPD. Upper row of images shows infra-red imaging and shows hyper-reflective figures centrally, with superior "ribbons & dots" that characterizes RPD. Middle row of images shows FAF and demonstrates normal foveal hypofluorescence, with regions of hypoFAF nasally, more so in the right eye (left image, middle row); superior RPD are noted as alternating hyper/hypoFAF with a ribbons and dots configuration. Lower row of images shows DNIRA imaging and demonstrates multiple regions of hypoFAF not seen in any other modality (including other modalities not shown here). These regions are distributed throughout the macula. The leopard-like or lacy patterns are again seen in the macular periphery.

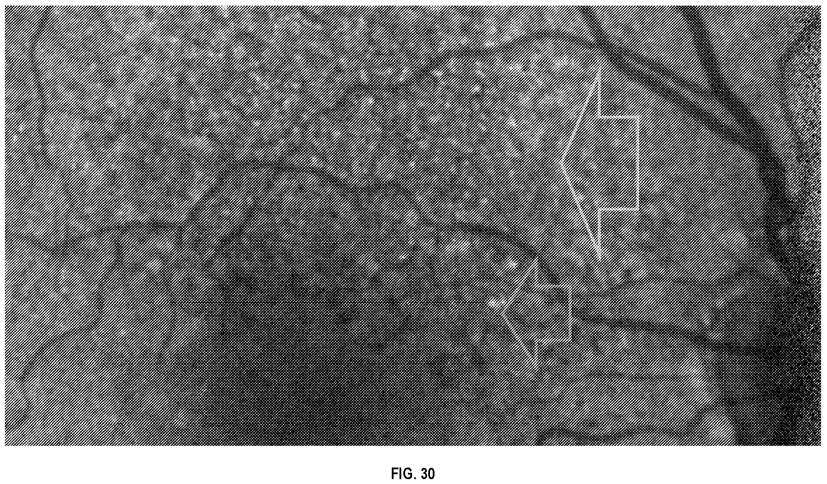

**FIG. 30** shows a gradient of hypofluorescent and hyperfluorescent signal in a human patient with RPD. DNIRA imaging of another patient with RPD is shown. The lower arrow shows region of discrete hypofluorescent (black) DNIRA signal immediately superior to the central macula. Superior to this, there is an increasing accumulation of hyperfluorescent dots seen using DNIRA.

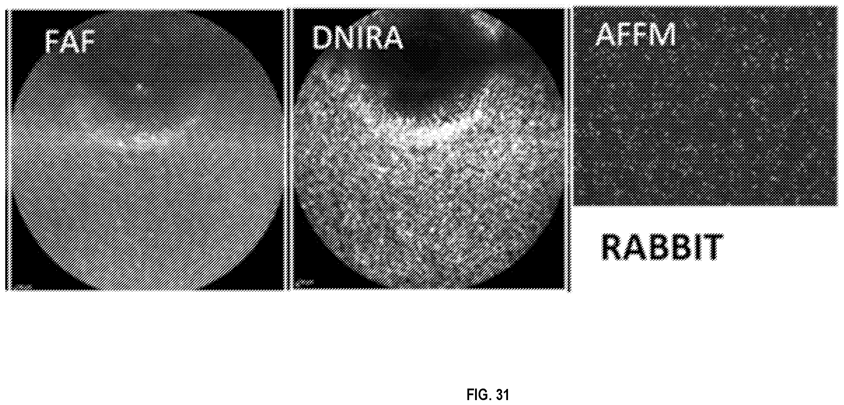

**FIG. 31** shows a correspondence between hyperfluorescent FAF signal and hyperfluorescent DNIRA signal in a rabbit eye. The left image is a FAF of a rabbit fundus after toxin injection. Small hyperfluorescent dots are observed in blue FAF. By using DNIRA, these, and many more bright dots appear (center image). Ex vivo autofluorescent epirfluorescent microscopy in the blue channel identifies nucleate cells in the subretinal space that correspond to the hyperfluorescent dots seen in FAF. NIR microscopy is not available. Immunohistochemistry confirms the identity of these cells as macrophages (noted previously in the rat, right panel).

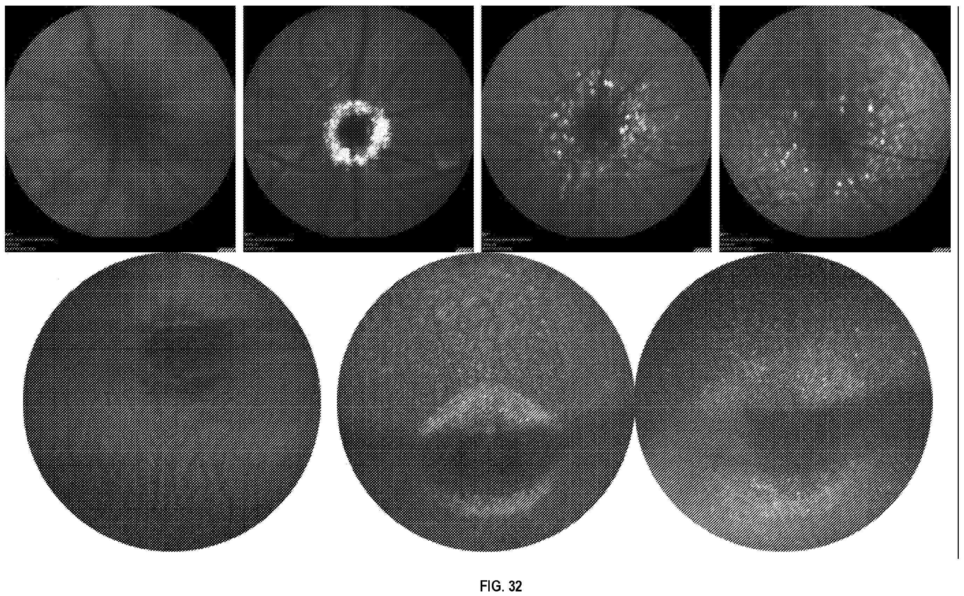

**FIG. 32** shows hyperfluorescent DNIRA dots, i.e. preseumptive macrophages, that change position over time. The upper row of images shows DNIRA imaging after systemic toxin injection in a rat and shows punctate hyperfluorescent dots encircling the optic nerve head. Over time, these dots appear to migrate centrifugally. The lower row shows DNIRA imaging ina rabbit eye with a similar apparent migration of individually labeled dots that by immunohistochemistry stain positively for macrophage markers. In both rows, left-most image is baseline DNIRA (*i.e*., pre-ICG injection with ICG filters in place).

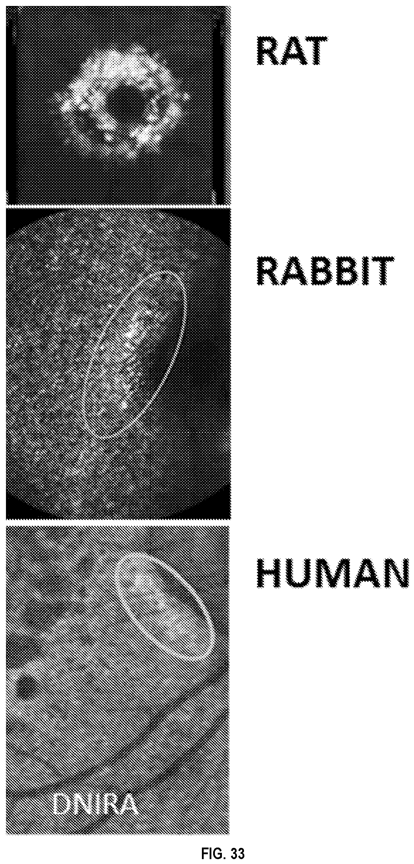

**FIG. 33** shows brightly labeled small DNIRA-positive dots are observed in proximity to the optic nerve head in rat (upper), rabbit (middle) and human (bottom) images. Comparison of the optic nerve head of rat, rabbit and human demonstrates the accumulation of punctate hyperfluorescent dots. Differences in magnification reflect the differences in the size of the eyes, imaged using the same clinical cSLO system. These data suggest for the first time, without wishing to be bound by theory, that macrophages can be evaluated in the living eye across multiple species, including human. They further suggest, without wishing to be bound by theory, that patients with hyperfluroescent dots seen by DNIRA may benefit from treatments directed against unwanted macrophage activity.

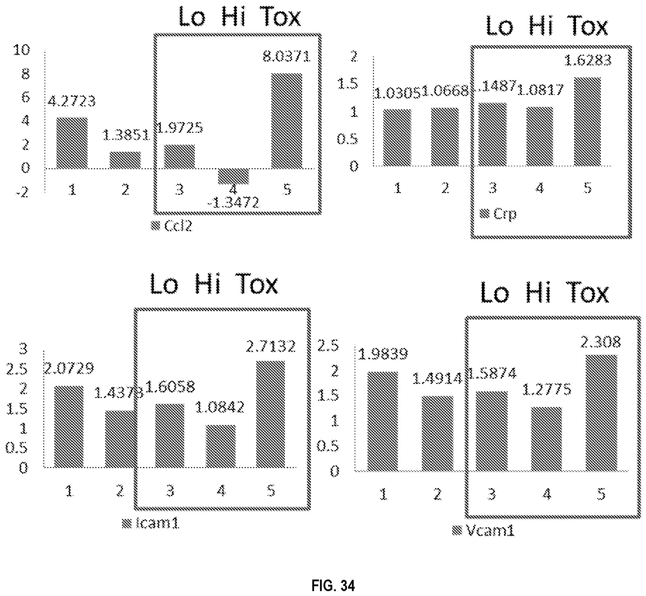

**FIG. 34** shows that bindarit reduces inflammatory mediators known to be involved in diabetic retinopathy. Fold mRNA changes were compared against baseline (normal) conditions, under the following conditions: 1. Intra-vitreal saline (vehicle), 2. High-dose bindarit, 2ug/ul, (no toxin), 3. Toxin + low dose 2ug/ul bindarit, 4. Toxin + high dose 20ug/ul bindarit + NaIO₃, and 5. Toxin alone.

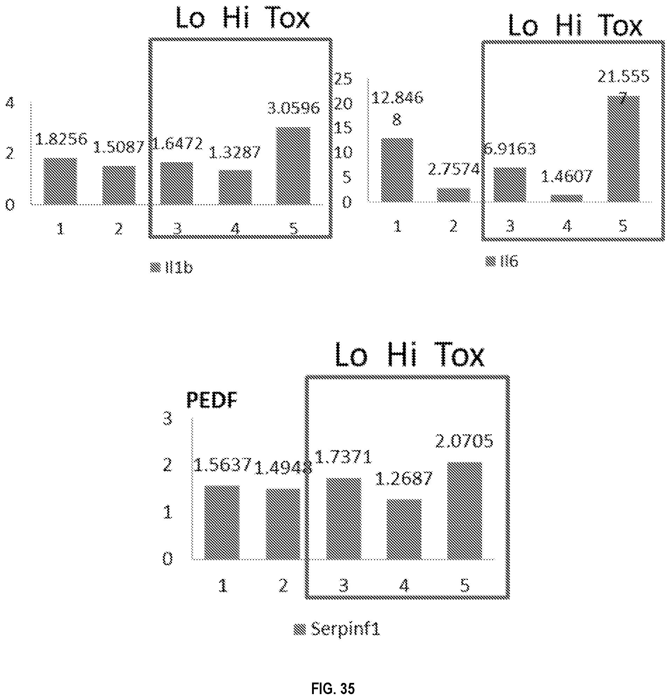

**FIG. 35** shows that bindarit significantly down-regulates inflammatory interleukins IL-1b and IL-6 and has a small negative regulatory effect on Pigment Epithelial Derived Factor (PEDF). Fold mRNA changes were compared against baseline (normal) conditions, under the following conditions: 1. Intra-vitreal saline (vehicle), 2. High-dose bindarit, 2ug/ul, (no toxin), 3. Toxin + low dose 2ug/ul bindarit, 4. Toxin + high dose 20ug/ul bindarit + NaIO₃, and 5. Toxin alone.

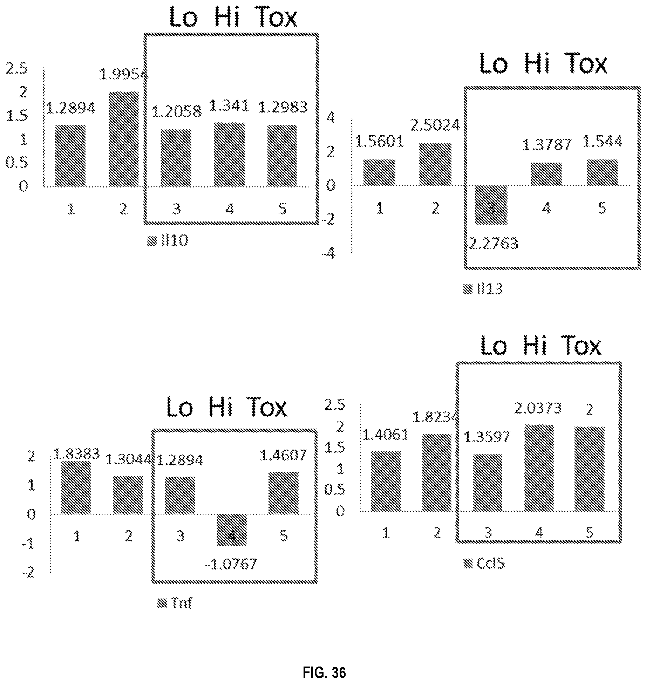

**FIG. 36** shows that bindarit, in this context, had no significant effect on other interleukins IL-10 and IL-3 or tumor necrosis factor alpha (TNFa) and ccl5 (MCP-3). Fold mRNA changes were compared against baseline (normal) conditions, under the following conditions: 1. Intra-vitreal saline (vehicle), 2. High-dose bindarit, 2ug/ul, (no toxin), 3. Toxin + low dose 2ug/ul bindarit, 4. Toxin + high dose 20ug/ul bindarit + NaIO₃, and 5. Toxin alone.

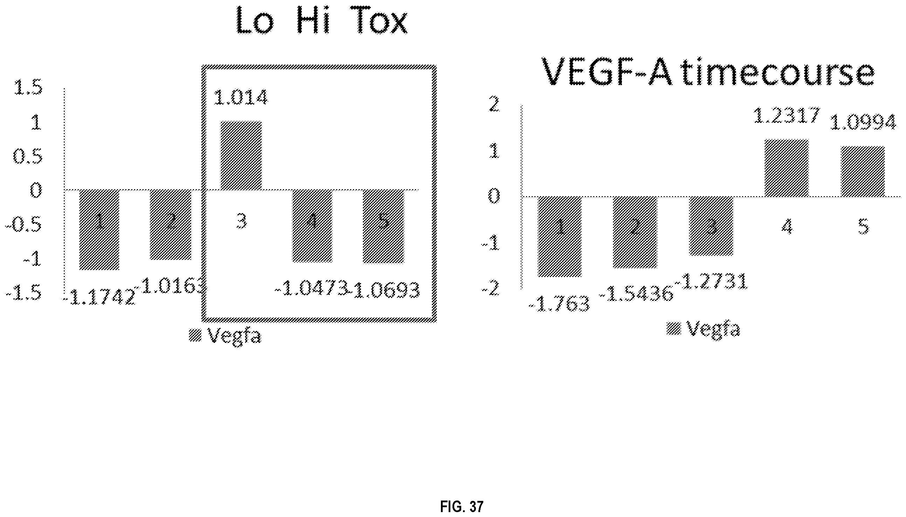

**FIG. 37** shows that bindarit had no significant effect on vascular endotheiial growth factor A (VEGF-A) mRNA. Fold mRNA changes were compared against baseline (normal) conditions, under the following conditions: 1. Intra-vitreal saline (vehicle), 2. High-dose bindarit, 2ug/ul, (no toxin), 3. Toxin + low dose 2ug/ul bindarit, 4. Toxin + high dose 20ug/ul bindarit + NaIO₃, and 5. Toxin alone. However, in this paradigm, VEGF-A was not elevaed (data not shown).

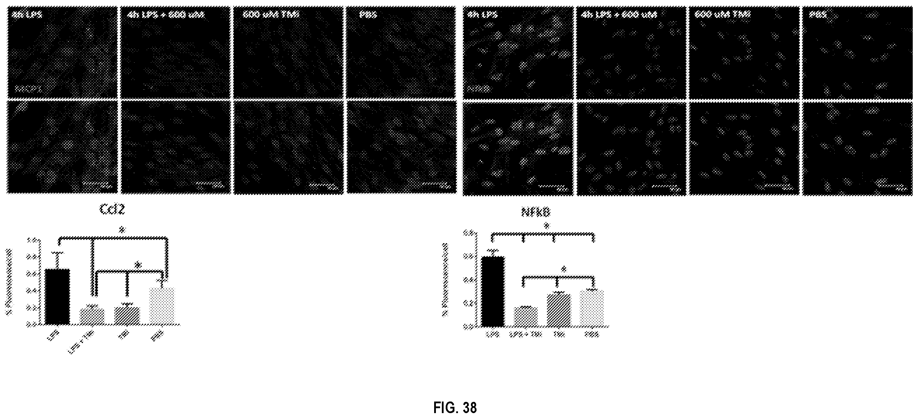

**FIG. 38** shows that, in an immortalized human RPE cell line, bindarit reduces NFkb translocation to the nucleus.

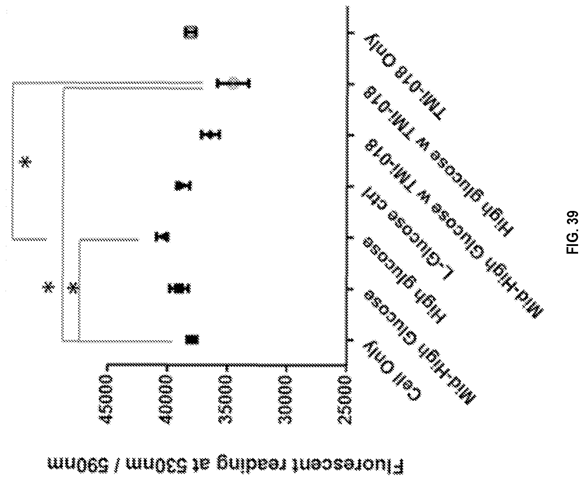

**FIG. 39** shows that bindarit normalizes cellular stress in hTERT cells incubated with high glucose. Cell Only: Medium only (contains 17.5mM D-Glucose); Mid-High D-Glucose: 30mM D-Glucose (17.5mM D-Glucose in medium + 12.5mM D-Glucose supplement); High D-glucose: 50mM D-Glucose (17.5mM D-Glucose in medium + 32.5mM D-Glucose supplement); L-Glucose ctrl: 50mM Glucose (D+L) (17.5mM D-Glucose in medium + 32.5mM L-Glucose supplement); Mid-High D-Glucose w TMi-018: 30mM D-Glucose with 150mM TMi-018; High D-glucose w TMi-018: 50mM D-Glucose with 150mM TMi-018; and TMi-018 Only: 150mM TMi-018.

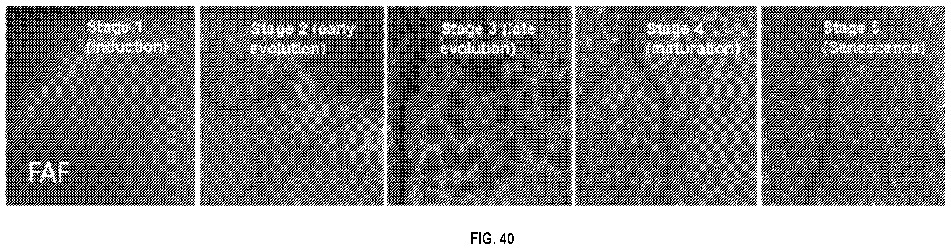

**FIG. 40** shows stages of change in RPE toxicity model that are referenced herein, as assessed with FAF. Specifically, representative images of the timecourse and stages of FAF images of rat eyes following NaIO₃ injection are shown.

### DETAILED DESCRIPTION OF THE INVENTION

The present invention relates to, in part, the surprising finding that bindarit has immunomodulatory effects, specifically on M1/M2 macrophage polarization, that makes it an effective agent for ocular disorders.

Accordingly, in one aspect, the invention provides an agent for use in a method of treating an ocular disorder. The agent is a compound of **Formula I** as shown herein, by way of limiting example, the compound bindarit.

The ocular disorder is a diabetic eye disease (for instance, diabetic retinopathy and DME)

In some embodiments, the subject is not undergoing treatment with and/or would benefit from therapy with and/or is unresponsive to one or more of an anti-factor D antibody (*e.g*. lampalizumab (Genentech)), an anti-β-amyloid (anti-Aβ) antibody (*e.g*. GSK933776 (GSK)), a corticosteroid (*e.g*. fluocinolone acetonide), MC-1101 (MacuCLEAR), a CD34+ stem cell therapy, an anti-VEGF antibody (*e.g*. Ranibizumab), brimonidine (Alphagan), an anti-C5 complement antibody (*e.g*. LFG316 (Novartis), doxycycline (ORACEA), emixustat hydrochloride, sirolimus (RAPAMUNE), and glatiramer acetate (COPAXONE). In some embodiments, the subject has evidence of AMD as confirmed by the presence of at least 1 druse greater than about 125 µm in diameter. In some embodiments, the subject may or may not have evidence of prior or concurrent active choroidal neovascularization (CNV). In some embodiments, the subject has one or more well-demarcated GA lesions of a total area of about 2 to about 20 mm² in one or more eye and/or 0.5 to 10 disc areas (*e.g*. 0.5 to 7, or about 0.5, or about 1, or about 2, or about 3, or about 4, or about 5, or about 6, or about 7, or about 8, or about 9, or about 10 disc areas). In some embodiments, the subject has a best-corrected visual acuity score of greater than about 35 letters or a Snellen VA equivalent of about 20/200 or better.

In some embodiments, the method further comprises administering an additional therapeutic agent. The additional therapeutic agent may be, in various embodiments, an anti-factor D antibody (*e.g*. lampalizumab (Genentech)), an anti-β-amyloid (anti-Aβ) antibody (*e.g*. GSK933776 (GSK)), a corticosteroid (*e.g*. fluocinolone acetonide), MC-1101 (MacuCLEAR), a CD34+ stem cell therapy, an anti-VEGF antibody (*e.g*. Ranibizumab), brimonidine (Alphagan), an anti-C5 complement antibody (*e.g*. LFG316 (Novartis), doxycycline (ORACEA), emixustat hydrochloride, sirolimus (RAPAMUNE), and glatiramer acetate (COPAXONE). The additional therapeutic agent may also be, in various embodiments, an anti-vascular endothelial growth factor (VEGF) agent, an angiotensin-converting enzyme (ACE) inhibitor, a peroxisome proliferator-activated receptor (PPAR)-gamma agonist, a renin inhibitor, a steroid, and an agent that modulates autophagy. In some embodiments, the additional therapeutic agent is a modulator of the complement cascade (*e.g*. a modulator of C3, C5, complement factor D, or complement factor B).

In some embodiments, the additional therapeutic agent is a nucleoside reverse transcriptase inhibitor (NRTIs), by way of non-limiting example zidovudine, didanosine, zalcitabine, stavudine, lamivudine, abacavir, emtricitabine, and entecavir. In some embodiments, the additional therapeutic agent is acyclovir.

The agent is a compound of **Formula I**: 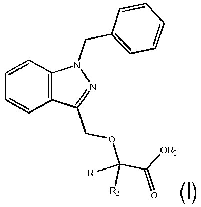 or a pharmaceutically acceptable salt thereof, wherein each of R₁ and R₂ is independently H or a C₁-C₆ alkyl and R₃ is H or a C₁-C₆ alkyl. In some embodiments, the agent is a compound of the structure: 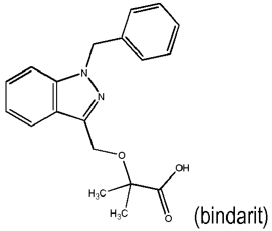 or a pharmaceutically acceptable salt thereof.

In some embodiments, the patient has type 1 or type 2 diabetes. In various embodiments, the patient has a blood glucose level that exceeds normal (about 70-130 milligrams per deciliter or mg/dL before meals, and less than 180 mg/dL one to two hours after a meal).

In other embodiments, the present invention pertains to a compound of **Formula I,** or a pharmaceutically acceptable salt thereof for use in treatment of a cataract or glaucoma, comprising administering to a patient in need thereof, where R₁, R₂ and R₃ are defined above. In one embodiment, the compound of **Formula I** is bindarit.

In some embodiments, the methods further comprise administering an additional therapeutic agent. In various embodiments, the additional therapeutic agent is one or more of an anti-vascular endothelial growth factor (VEGF).

In other embodiments, the subject is a human. In still other embodiments, the administering is effected orally or intra-vascularly, or intraocularly, or periocularly, or to the ocular surface.

### Definitions

The following definitions are used in connection with the invention disclosed herein. Unless defined otherwise, all technical and scientific terms used herein have the same meaning as commonly understood to one of skill in the art to which this invention belongs.

An "effective amount," when used in connection with a test compound and/or candidate compound is an amount that is effective for providing a measurable treatment, prevention, or reduction in the rate of pathogenesis of an ocular disorder such as, for example, a diabetic eye disease (for instance, diabetic retinopathy and DME), Vogt-Kayanagi-Harada disease (VKH); Sarcoid uveitis; Ocular histoplasmosis and/or Presumed Ocular Histoplasmosis Syndrome; Autoimmune uveitis; Uveitis associated with systemic diseases, e.g. lupus, Crohn's disease, rheumatoid arthritis, and other diseases of known immune origin; Posterior uvieits (including that which may not yet be diagnosed); Anterior uveitis (*e.g*. iritis); Bechet's disease; Polyarteritis nodosa; and Wegener granulomatosis, AMD and/or RPD.

An "effective amount," when used in connection with a fluorescent compound is an amount that allows optical detection.

An "effective amount," when used in connection with an agent effective for the treatment of an ocular disorder, a compound of **Formula I**, methotrexate, or a pharmaceutically acceptable salt thereof, is an amount that is effective for treating or preventing an ocular disorder such as, for example, a diabetic eye disease (for instance, diabetic retinopathy and DME).

An "effective amount," when used in connection with another therapeutic agent is an amount that is effective for treating or preventing an ocular disorder such as, for example, a diabetic eye disease (for instance, diabetic retinopathy and DME) alone or in combination with a compound of **Formula I**, methotrexate, or a pharmaceutically acceptable salt thereof. "In combination with" includes administration within the same composition and via separate compositions; in the latter instance, the other therapeutic agent is effective for treating or preventing a condition during a time when the agent a compound of **Formula I**, methotrexate, or a pharmaceutically acceptable salt thereof, exerts its prophylactic or therapeutic effect, or vice versa.

An "effective amount," when used in connection with a toxin, for example, sodium iodate, is an amount that is effective for inducing measurable atrophy of ocular tissue as described herein.

An agent is "useful for the treatment of an ocular disorder" if the agent provides a measurable treatment, prevention, or reduction in the rate of pathogenesis of an ocular disorder.

The term "ocular disorder" refers to one of various ophthalmic diseases and includes diseases of the eye and the ocular adnexa. The term "ocular disorder" includes, for example, disorders described herein (for instance, by way of non-limiting example, an ocular disorder such as, for example, a diabetic eye disease (for instance, diabetic retinopathy and DME), Vogt-Kayanagi-Harada disease (VKH); Sarcoid uveitis; Ocular histoplasmosis and/or Presumed Ocular Histoplasmosis Syndrome; Autoimmune uveitis; Uveitis associated with systemic diseases, e.g. lupus, Crohn's disease, rheumatoid arthritis, and other diseases of known immune origin; Posterior uvieits (including that which may not yet be diagnosed); Anterior uveitis (*e.g*. iritis); Bechet's disease; Polyarteritis nodosa; and Wegener granulomatosis, wet AMD, dry AMD, RPD, white-dot syndromes (*e.g*. serpiginous chorioretinopathy, serpiginous retinopathy, acute posterior multifocal placoid pigment epitheliopathy (APMPPE), multiple evanescent white dot syndrome (MEWDS), acute zonal occult outer retinopathy (AZOOR), punctate inner choroidopathy (PIC), diffuse subretinal fibrosis (DSF)), LORDs, and central serous retinopathy (CSR).

The term "neovascularization" refers to new blood vessel formation in abnormal tissue or in abnormal positions.

The term "VEGF" refers to a vascular endothelial growth factor that induces angiogenesis or an angiogenic process, including, but not limited to, increased permeability. As used herein, the term "VEGF" includes the various subtypes of VEGF (also known as vascular permeability factor (VPF) and VEGF-A) that arise by, *e.g*., alternative splicing of the VEGF-A/VPF gene including VEGF₁₂₁, VEGF₁₆₅ and VEGF₁₈₉. Further, as used herein, the term "VEGF" includes VEGF-related angiogenic factors such as PIGF (placental growth factor), VEGF-B, VEGF-C, VEGF-D and VEGF-E, which act through a cognate VEFG receptor (*i.e*., VEGFR) to induce angiogenesis or an angiogenic process. The term "VEGF" includes any member of the class of growth factors that binds to a VEGF receptor such as VEGFR-1 (Flt-1), VEGFR-2 (KDR/Flk-1), or VEGFR-3 (FLT-4). The term "VEGF" can be used to refer to a "VEGF" polypeptide or a "VEGF" encoding gene or nucleic acid.

The term "anti-VEGF agent" refers to an agent that reduces, or inhibits, either partially or fully, the activity or production of a VEGF. An anti-VEGF agent can directly or indirectly reduce or inhibit the activity or production of a specific VEGF such as VEGF₁₆₅. Furthermore, "anti-VEGF agents" include agents that act on either a VEGF ligand or its cognate receptor so as to reduce or inhibit a VEGF-associated receptor signal. Non-limiting examples of "anti-VEGF agents" include antisense molecules, ribozymes or RNAi that target a VEGF nucleic acid; anti-VEGF aptamers, anti-VEGF antibodies to VEGF itself or its receptor, or soluble VEGF receptor decoys that prevent binding of a VEGF to its cognate receptor; antisense molecules, ribozymes, or RNAi that target a cognate VEGF receptor (VEGFR) nucleic acid; anti-VEGFR aptamers or anti-VEGFR antibodies that bind to a cognate VEGFR receptor; and VEGFR tyrosine kinase inhibitors.

The term "anti-RAS agent" or "anti-Renin Angiotensin System agent" refers to refers to an agent that reduces, or inhibits, either partially or fully, the activity or production of a molecule of the renin angiotensin system (RAS). Non-limiting examples of "anti-RAS" or "anti-Renin Angiotensin System" molecules are one or more of an angiotensin-converting enzyme (ACE) inhibitor, an angiotensin-receptor blocker, and a renin inhibitor.

The term "steroid" refers to compounds belonging to or related to the following illustrative families of compounds: corticosteroids, mineralicosteroids, and sex steroids (including, for example, potentially androgenic or estrogenic or anti-andogenic and anti-estrogenic molecules). Included among these are, for example, prednisone, prednisolone, methyl-prednisolone, triamcinolone, fluocinolone, aldosterone, spironolactone, danazol (otherwise known as OPTINA), and others.

The terms "peroxisome proliferator-activated receptor gamma agent," or "PPAR-γ agent," or "PPARG agent," or "PPAR-gamma agent" refers to agents which directly or indirectly act upon the peroxisome proliferator-activated receptor. This agent may also influence PPAR-alpha, "PPARA" activity.

The term "an agent that modulates autophagy" refers to a modulator of cell survival, cell death, survival, autophagy, proliferation, regeneration, and the like.

The term "monocyte chemotactic protein," or "MCP" refers to the family or a member of the small inducible gene (SIG) family that plays a role in the recruitment of monocytes to sites of injury and infection. These terms can also refer to C-C motif chemokine molecules, whose activity include, for example, macrophage recruitment, and/or proliferation and/or activation.

The term "alkyl," as used herein unless otherwise defined, refers to a straight or branched saturated group derived from the removal of a hydrogen atom from an alkane. Representative straight chain alkyl groups include, but are not limited to, -methyl, -ethyl, -n-propyl, -n-butyl, -n-pentyl, and n-hexyl. Representative branched alkyl groups include, but are not limited to, isopropyl, -sec-butyl, -isobutyl, -tert-butyl, -isopentyl, -neopentyl, 1-methylbutyl, 2-methylbutyl, 3-methylbutyl, 1,1-dimethylpropyl and 1,2-dimethylpropyl.

As used herein, "a," "an," or "the" can mean one or more than one. Further, the term "about" when used in connection with a referenced numeric indication means the referenced numeric indication plus or minus up to 10% of that referenced numeric indication. For example, the language "about 50" covers the range of 45 to 55.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of terms such as including, containing, or having, is used herein to describe and claim the invention, the present invention, or embodiments thereof, may alternatively be described using alternative terms such as "consisting of or "consisting essentially of."

### DNIRA

In various embodiments, the present invention involves optical imaging, using various techniques that are known in the art. For example, such techniques include, but are not limited to cSLO, FAF, angiography, OCT, including three dimensional reconstructions of such. In various embodiments of the invention, exposing an eye to light comprises performing cSLO, FAF, DNIRA, angiography or OCT. In various embodiments, the imaging is DNIRA. In various embodiments, combinations of any of the above techniques may be used.

The inventor previously demonstrated that following systemic delivery of sodium iodate, patches of hypofluorescent FAF are not observed *in vivo* in areas of RPE loss (in the non-clinical setting, *i.e*., non-human eye) as would be predicted from clinical investigation (data not shown). However, by pre-labeling the RPE with a fluorescent dye, such as the near infra-red (NIR) dye indocyanine green (ICG), a technique called Delayed Near InfraRed Analysis (DNIRA), the RPE is made visible using cSLO imaging. Once labeled, areas of RPE loss become apparent as quantifiable patches of hypofluorescence similar to those observed clinically. In various embodiments, NaIO₃, FAF and DNIRA may be used together to show, by way of example, the relationship between RPE loss, macrophages, macrophage polarization, and regulation of the M1 response.

For DNIRA, a compound suitable for fluorescence detection including a near-infrared (NIR) dye, such as, ICG when given at non-toxic doses, can label the RPE and therefore make it visible when viewed using the ICG excitation/emission filters in the days or weeks thereafter. Importantly, this visualization in the days and weeks thereafter may be without re- administration of dye. Accordingly, in some aspects, a central component of the DNIRA technique lies in its timing. This is distinct from the present usage of ICG or other angiographic dyes that are viewed immediately after injection, during the transit phase, or in the immediate minutes to hours following injection, to determine the intra-vascular localization of dye and its immediate extravasation.

In some embodiments, DNIRA is used in a laboratory animal. In one embodiment, DNIRA may involve administration of a compound suitable for fluorescence detection, by way of non-limiting example, ICG (and, optionally, angiography) at about one or more days prior to administration with a toxin or other agent that causes patchy geographic areas of RPE loss (*e.g*. NaIO₃) and optionally followed by, at about 1 or more days (or about one week, or about one month, or about three months), an additional amount of NaIO₃ or another agent that causes expansion of the areas of patchy RPE loss. For example, the other challenge that causes geographic atrophy expansion (*e.g*. as an initial, or second, or third, or fourth administration) may be a modulator of cell survival, cell death, survival, autophagy, proliferation, regeneration, and the like.

In various embodiments, the DNIRA technique is used in a human patient. For example, DNIRA in a human patient may not comprise the use of a toxin. DNIRA in a human patient may comprise the evaluation of normal or disease-associated changes in the eye, using a fluorescent dye, with the excitation/emission filters in place but no angiography.

Expansion of geographic atrophy is a U.S. Food and Drug Administration (FDA) approved primary outcome for clinical trial design, and, accordingly, this invention makes possible observation of geographic atrophy, in particularly the expansion of geographic atrophy, in an animal model, thus permitting correlation between pre-clinical disease models and clinical trial design. The inability to clearly identify the geographic atrophy, or expansion of geographic atrophy, in an eye of an animal prior to the present invention has precluded direct correlation between pre-clinical studies and clinical observation. Further, in some embodiments, the present invention allows for clinical evaluation of geographic atrophy, including the expansion of geographic atrophy, in a human patient.

In some embodiments, the compound suitable for fluorescence detection is suitable for imaging with various wavelengths of fluorescence. In some embodiments, these wavelengths range from visible light to infrared, e.g., 390 nm to 1 mm, including, for example, blue light, white light, and near-infrared. In some embodiments, the dye is a near-infrared dye. In some embodiments, the dye is ICG.

In some embodiments, DNIRA is performed (and/or delayed near infrared fluorescence (DNIRF) is observed) at about 1 day, or about 2 days, or about 3 days, or about 4 days, or about 5 days, or about 6 days, or about 7 days, or about 10 days, or about 14 days, or about 21 day after the administration. In some embodiments, the DNIRA is performed at least 1 day after the administration, or at least 2 days, or at least 3 days, or at least 4 days, or at least 5 days, or at least 6 days, or at least 7 days, or at least 10 days, or at least 14 days, or at least 21 days after the administration. In other embodiments, the DNIRA is performed at least about 24 hours, or at least about 7 days, or at least about 30 days, or at least 60 days, or at least 90 days after administering. In other embodiments, the DNIRA is performed at least about 2 months, or about 3 months, or about 4 months, or about 5 months, or at a maximum about 6 months after administering. In some embodiments, the DNIRA is not performed during the transit stage (*i.e*. the first passage of dye as it flows through the ocular blood vessels and into the ocular tissue) or minutes thereafter.

In some embodiments, the visualization is effected using a cSLO. In some embodiments, the visualization is effected using white light and appropriate filters. In some embodiments, the ICG excitation/emission filters are 795 nm (excitation)/810 nm (emission) filters.

The RPE is a critical epithelial monolayer that serves a "nurse-cell" function for an eye's specialized photoreceptors, the rods and cones. Blinding eye diseases, such as, for example, AMD and RPD, are, without wishing to be bound by theory, causally linked in part to abnormalities of the RPE.

DNIRA makes it possible to clearly identify the RPE layer *in vivo* in an eye of an animal. Further, the leading technique used to detect the RPE in the human eye, FAF, is ineffective or poorly effective in the rodent eye (by way of non-limiting example), possibly owing to a relative paucity of fluorophores such as lipofuscin. FAF imaging in the human eye is performed using the blue spectrum of non-coherent light in the presence of stimulation/emission filters, or coherent blue light, and can identify areas of absent RPE (*e.g*. hypo-fluorescent signal) or abnormal RPE (*e.g*. hyper-fluorescent signal). The inability to clearly identify the RPE in an eye of an animal, in the absence of DNIRA, has precluded direct correlation between pre-clinical studies and clinical observation.

Accordingly, in various aspects of the present invention, methods to make visible the RPE layer, such as, for example, DNIRA, in an eye of an animal for pre-clinical investigation of ocular disorders are provided.

Further, as described herein, DNIRA, or variations thereof, allow for visualization of fluorescent immune cells in the eyes of an animal. In some embodiments DNIRA may optionally not comprise toxin administration. In some embodiments, DNIRA is performed in a human patient and a toxin is not applied.

Further, as described herein, DNIRA, or variations thereof, allow for visualization of fluorescent immune cells in the eyes of human subject. In some embodiments with a human subject DNIRA may not comprise toxin administration.

In some embodiments, DNIRA is used in the identification of an agent that is effective for treating an ocular disorder.

In some embodiments, DNIRA is used as a method to evaluate a patient that has, or may have, an ocular disorder (including, without limitation AMD and RPD). In some embodiments, DNIRA is a surrogate biomarker for diagnosis and/or prognosis and/or progression of an ocular disorder (including, without limitation AMD and RPD). For example, DNIRA may be used to identify patterns, including lacy, reticular or leopard-like pattern of alternating hyper- and hypo-fluorescent DNIRA that is not seen in other imaging modalities, that are indicative of a ocular disease state (without limitation AMD and RPD). DNIRA may also be used to identify, and quantify, areas of hyper- and hypo-fluroescent DNIRA.

In various embodiments, DNIRA is used to identify hypofluorescent features of an eye. For instance, these areas appear black when imaged and therefore allow for easy quantitation (in contrast to ICG imaging, or in contrast to hyperfluroescent signal, which is grey-scale rather than black/white). Detection of hypofluroescent DNIRA, in some embodiments, is predictive of damaged or dead RPE. For example, hypofluroescent DNIRA may indicate one or more of an absence of RPE, abnormal/unhealthy RPE (which is unable to uptake ICG dye), RPE that does not lie in contact with Bruch's Membrane (and so are no longer in a position to take up ICG dye from the choroidal vasculature), and the presence of lipid that could be located either between the RPE and BM (thus blocking ICG uptake), or could be internal to the RPE (thus blocking the RPE signal).

In various embodiments, DNIRA is used to identify hyperfluorescent features of an eye. For instance, these areas appear light when imaged and therefore allow for easy quantitation. Detection of hyperfluroescent DNIRA, in some embodiments, is predictive of macrophages, including M1 and/or M2 macrophages

In various embodiments, DNIRA is used biomarker for diagnosis of an ocular disease state (without limitation AMD and RPD) and prompts further evaluation and/or treatment with one of more agents, including without limitation those described herein.

In various embodiments, DNIRA is used as a biomarker for prognosis of an ocular disease state (without limitation AMD and RPD) and prompts further evaluation and/or treatment with one of more agents, including without limitation those described herein.

In various embodiments, DNIRA is used to improve identification of suitable patients for study recruitment and to evaluate progression of disease. In various embodiments, DNIRA is used to monitor disease progression.

In various embodiments, DNIRA is used as a companion diagnostic to any of the agents described herein. In various embodiments, DNIRA is used to evaluate patient response to any of the agents described herein (including evaluating the effectiveness of any of the agents described herein and/or the likelihood of response to any of the agents described herein). In various aspects, the present invention further relates to the use of DNIRA as entrance inclusion or exclusion criteria, or endpoint analysis for clinical trial design.

### Ocular Disorders

In various embodiments, an ocular disorder is evaluated and/or identifiable using DNIRA or a technique known in the art. Illustrative techniques include: cSLO, FAF, OCT (including with cross-sectional, three-dimensional and *en face* viewing), SD-OCT (with cross-sectional, three-dimensional and *en face* viewing), angiography, or other imaging modalities including other wavelengths of fluorescence. In some embodiments, these wavelengths range from blue to infrared, *e.g*., 390 nm to 1 mm, including, for example, blue light, white light, red-free, near infra-red, infrared.

In various embodiments, an ocular disorder is evaluated and/or identifiable by patterns of FAF within patches of RPE damage or loss or outer retinal loss. In some embodiments, the patterns are one or more of curvilinear, ribbon-like, reticular, oval, circular, scalloped, halo, and target-like lesions.

In various embodiments, an ocular disorder evaluated and/or identifiable by patterns of FAF within a border of patches of RPE damage or loss or outer retinal loss. In some embodiments, the patterns are one or more of curvilinear, ribbon-like, reticular, oval, circular, scalloped, halo, and target-like lesions.

In various other embodiments, an ocular disorder is evaluated and/or identifiable by cross-sectional patterns or transverse patterns. In some embodiments, the patterns are observed with OCT. In some embodiments, the patterns are RPE and/or outer retinal loss or mounds, triangles, peaks or spikes found in the sub-retinal space.

In other embodiments, an ocular disorder is evaluated and/or identifiable by the presence of areas of hyper-fluorescent FAF in an eye of the subject and/or the presence of one or more areas of abnormally fluorescent FAF in an eye of the subject and/or by changes in one or more of blue spectrum fundus imaging, white-light fundus imaging, red-free fundus imaging, and OCT in an eye of the subject and/or by an increase (*e.g*. a transient increase) in permeability across the subject's epithelial barrier between a choroid and a retina relative to an undiseased state and/or by a deformation of the outer retina, and/or deformation of the mid-retinal vasculature across the subject's epithelial barrier between a choroid and a retina relative to an undiseased state and/or by the presence of phagocytic immune cells across the subject's RPE relative to an undiseased state. Illustrative changes include differences in an imaging pattern in the same of different subject and/or animal at two different time points and/or experimental or clinical conditions. For example, changes can encompass changes in imaging data between two evaluations of the same subject and/or animal and/or changes in imaging data between a first subject and/or animal and the imaging data of a second subject and/or animal.

In still other embodiments, an ocular disorder is evaluated and/or identifiable by the presence of one or more areas of distinct patterns of retinal imaging in the eye of a subject, wherein the retinal imaging is one or more of white light, red-free light, blue light, FAF, near infra-red (NIR), infra-red (IR), angiography, and DNIRA and/or the presence of one or more areas of abnormally-fluorescent FAF in the eye of a subject and/or an increase (e.g. a transient increase) in permeability across the subject's epithelial barrier between a choroid and a retina relative to an undiseased state and/or a presence of phagocytic immune cells across the subject's RPE relative to an undiseased state.

The ocular disorder is a diabetic eye disease, by way non-limiting example, diabetic retinopathy and DME. In some embodiments, the diabetic eye disease is found in a patient with type 1 or type 2 diabetes. In various embodiments, a diabetic eye disease patient to be treated may have a blood glucose level that exceeds normal (about 70-130 milligrams per deciliter or mg/dL before meals, and less than 180 mg/dL one to two hours after a meal).

In some embodiments, the diabetic eye disease is diabetic retinopathy in one or more of the following stages: Mild Nonproliferative Retinopathy (in which microaneurysms may occur); Moderate Nonproliferative Retinopathy (in which some blood vessels that nourish the retina may be blocked); and Severe Nonproliferative Retinopathy (in which more blood vessels may be blocked, depriving several areas of the retina with their blood supply); and Proliferative Retinopathy (in which signals sent by the retina for nourishment trigger the growth of new blood vessels.

In some embodiments, the present methods for treatment or prevention of a diabetic eye disease (for instance, diabetic retinopathy and DME) further comprise performing laser surgery (*e.g*. scatter laser treatment, focal laser treatment, photocoagulation) and/or vitrectomy (*e.g*. in which blood is removed from the center of a patient's eye) and/or administering a steroid and/or administering an anti-VEGF agent such as Ranibizumb (LUCENTIS), Bevizumab (AVASTIN) or Aflibercept (EYLEA).

In some embodiments, the diabetic eye disease is a cataract or glaucoma (*e.g*., neovascular glaucoma).

In some embodiments, disorders associate with imaging (FAF) patterns such as "diffuse trickling" may be evaluated, treated or prevented using methods disclosed herein. Diffuse trickling patterns are known in the art *(see, e.g.,* Schmitz-Valckenberg et al. Survey of Ophthalmology. 2009 Jan-Feb; 54(1):96-117; Bindewald et al. British Journal of Ophthalmology. 2005 Jul; 89(7):874-8; Holz et al. Am. J Ophthalmology. 2007 Mar; 143(3):463-72).

In some embodiments, DNIRA is used to identify and/or diagnoses any of the ocular disorder described herein. In some embodiments, RPE toxicity, RPE loss, and the dry AMD are identifiable using DNIRA, e.g. with sodium iodate (NaIO₃) or in the presence of disease. Areas analogous to geographic atrophy can be detected by, for example, tissue analysis (for example, by observing the loss of an RPE cell marker such as RPE65 and/or the loss of binucleate cell staining), and/or *in vivo,* using DNIRA, of NaIO₃.treated- eyes. In some embodiments, RPE toxicity, RPE loss and the dry AMD are identifiable using FAF which shows a hyperfluorescent FAF signal, which may be complex, within the area of imminent tissue loss and/or at its margins. This complex hyperfluorescent FAF signal develops, in the days or weeks after NaIO₃ treatment, into a pattern of FAF, which can be complex and can include, but is not limited to ribbon-like, curvilinear, pisciform, scalloped, interlacing, branching, or reticular hyperfluorescence. Such patterns mimic those found in clinical dry AMD. Such FAF may also be hypofluorescent.

Further, using angiography alone, or as part of DNIRA, angiography performed immediately prior to, or coincident with, the emergence of the areas of altered FAF may demonstrate an increase (including a transient increase) in permeability across the epithelial barrier between choroid and retina (such as, for example, observed by leakage of dyes that may be injected prior to imaging; such dyes include ICG). This barrier normally includes the inner choroid, Bruch's Membrane, the RPE cells, and, possibly, the configuration of the outer photoreceptors in conjunction with the RPE and the outer limiting membrane. Without wishing to be bound by theory, a transient breakdown of this specialized epithelial barrier may underlie a sequence of events thereafter including folding, undulation, or deformation of the outer retina, photoreceptor layer, and/or movement/migration of inflammatory cells from the choroid to the subretinal space. However, in some embodiments, this phase may be transient and resolved, or subclinical in its extent.

In some embodiments, one or more of the ocular disorder are identifiable by the presence of areas of hyper- and/or abnormal-fluorescent FAF, or other wavelengths of light from 350 nm to 1,000 nm. In some embodiments, one or more of the ocular disorders are identifiable using DNIRA.

In some embodiments, one or more of the ocular disorders are identifiable by, for example, cSLO, FAF, OCT (including with cross-sectional, three-dimensional and *en face* viewing), SD-OCT (with cross-sectional, three-dimensional and *en face* viewing), or other imaging modalities including other wavelengths of fluorescence (e.g. wavelengths ranging from blue to infrared, e.g., 390 nm to 1 mm, including, for example, blue light, white light, red-free, near infra-red, or infrared).

In some embodiments, the ocular disorder may be of a certain stage or progression. In some embodiments, the ocular disorder may be incipient, emerging, quiescent, advancing or active.

### Agents of the Invention

In some embodiments, a compound of **Formula I**, or a pharmaceutically acceptable salt thereof, is also administered with an additional therapeutic agent. In various embodiments, the additional therapeutic agent may be used in a combination therapy with the agents effective for treating an ocular disorder described herein.. In various embodiments, the agents effective for treating an ocular disorder described herein is administered to a patient undergoing treatment with one or more additional therapeutic agents. Additional therapeutic agents, include, for example, one or more of an anti-VEGF agent, an ACE inhibitor, a PPAR-gamma agonist or partial agonist, a renin inhibitor, a steroid, and an agent that modulates autophagy, as well as a semapimod, a MIF inhibitor, a CCR2 inhibitor, CKR-2B, a 2-thioimidazole, CAS 445479-97-0, CCX140, clodronate, a clodonate-liposome preparation or gadolinium chloride.

An agent of the invention is a compound of **Formula I**: 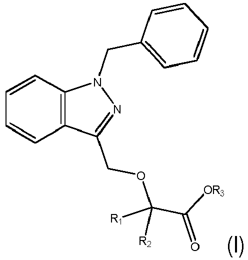 or a pharmaceutically acceptable salts thereof, wherein each of R₁ and R₂ is independently H or a C₁-C₆ alkyl and R₃ is H or a C₁-C₆ alkyl.

In various embodiments, R₁ and R₂ are independently hydrogen, methyl or ethyl.

In some embodiments R₁ and R₂ are independently methyl or ethyl.

In some embodiments R₁ and R₂ are methyl.

In some embodiments R₃ is hydrogen, methyl or ethyl.

In some embodiments R₃ is hydrogen.

In some embodiments, particularly where R₃ is hydrogen, the compounds of **Formula I** are in the form of a pharmaceutically acceptable salt.

In some embodiments R₁ and R₂ are methyl and R₃ is hydrogen. In such embodiments, the compound of **Formula I** is 2-((1-benzylindazol-3-yl) methoxy)-2-methyl propionic acid, also known as 2-methyl-2-[[1-(phenylmethyl)-1H-indazol-3yl]methoxy] propanoic acid. Such a compound is commonly known as bindarit and has the following structure:

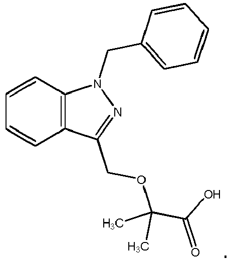

Synthesis of bindarit is described in detail in U.S. Patent No. 4,999,367.

Bindarit is an inhibitor of MCP-1 production *in vitro* and *in vivo* and, without wishing to be bound by theory, its beneficial effects in animal models of inflammation may be related to this anti-MCP-1 activity (see Mirolo, et al. Eur Cytokine Netw 2008;19:119-122). Bindarit has also been shown to selectively inhibit the production of MCP-2 and MCP-3 (see Mirolo, et al. Eur Cytokine Netw 2008;19:119-122).

### Fluorescent Compounds

In some embodiments, the fluorescent compound is suitable for imaging with various wavelengths of fluorescence. In some embodiments, these wavelengths range from visible light to infrared, *e.g*., 390 nm to 1 mm, including, for example, blue light, white light, and near-infrared. In some embodiments, the dye is a near-infrared dye. In some embodiments, the dye is ICG.

In some embodiments, the fluorescent compound is suitable for imaging with various wavelengths of fluorescence. In some embodiments, these wavelengths range from visible light to infrared, *e.g*., about 390 nm to about 1 mm, including, for example, blue light, white light, and near-infrared. In some embodiments, the absorption is from about 390 nm to about 1 mm. In some embodiments, the emission is from about 390 nm to about 1 mm.

In some embodiments, the fluorescent compound absorbs light at a wavelength of about 600 nm to about 900 nm and/or emits light at a wavelength of about 750 nm to about 950 nm.

In some embodiments, the dye is a near-infrared dye. In some embodiments, the dye is ICG. In some embodiments, the ICG excitation/emission filters are 795 nm (excitation)/810 nm (emission).

In some embodiments, the dose of the fluorescent compound, *e.g*. a dye (including ICG), is an effective amount of the fluorescent compound. In various embodiments, the dose of ICG is from about 0.1 to about 10 mg/kg of an animal. In some embodiments, the dose is about 0.1, or about 0.3, or about 0.5, or about 1.0, or about 2.0, or about 3.0, or about 4.0, or about 5.0, or about 6.0, or about 7.0, or about 8.0, or about 9.0, or about 10.0 mg/kg of an animal.

In various embodiments, the fluorescent compounds, or metabolites thereof, cause a fluorescence which occurs in RPE cells and/or immune cells.

In other embodiments, the methods described herein do not comprise administering (i) an additional amount of fluorescent compound to the animal or (ii) a second fluorescent compound to the animal.

### Toxins

In some embodiments, a toxin known to affect ocular tissue, including but not limited to, the RPE or retina, is provided.

In some embodiments, the toxin is one or more of aluminium, aminophenoxyalkanes (a non-limiting example includes, but is not limited to, 1,4,-bis(4-aminophenoxy)-2-phenylbenzene to rats), cationic amphophilic drugs /tricyclic antidepressants (non-limiting examples include but are not limited to amiodarone, chloroamitriptyline, chlorphentermine, clomipramine, imipramine, iprindole, various aminoglycosides, and other cationic amphophilic compounds), desferrioxamine, dl-(p-trifluoromethylphenyl) isopropylamine hydrochloride, fluoride (e.g. sodium fluoride), iodate (non-limiting examples include but are not limited to, sodium or potassium iodate), iodoacetate, lead, methanol and formic acid, 4,4'-Methylenedianiline, N-methyl-N-nitrosurea, naphthalene, napthol, nitroaniline (N-3-pyridylmethyl-N'-p-nitrophenylurea/nitroanilin/pyriminil), organophosphates (non-limiting examples include but are not limited to ethylthiometon, fenthion, and fenitrothion), oxalate (a non-limiting example includes but are not limited to dibutyl oxalate), phenothiazines (non-limiting examples include but are not limited to piperidylchlorophenothiazine, thioridazine, and chlorpromazine), quinolines (a non-limiting example includes, but is not limited to, chloroquine and hydroxychloroquine), streptozotocin, taurine deficiency, urethane, zinc deficiency caused by metal chelators, and derivatives and variants thereof.

In some embodiments, the toxin is an iodate. In some embodiments, the toxin is sodium or potassium iodate.

In some embodiments, the toxin induces atrophy of ocular tissue. In various embodiments, the atrophy comprises necrosis and/or apoptosis. In various embodiments, the atrophy comprising necrosis and/or apoptosis is of RPE cells. In some embodiments, the toxin reduces or modifies autophagy. In some embodiments, the toxin induces geographic atrophy and/or the expansion of geographic atrophy. In some embodiments, the toxin induces one or more of the above-mentioned effects.

In some embodiments, the toxin is administered one time, or two times, or three times. In some embodiments, the toxin is administered one time, or two times, or three times, or four times, or five times. In some embodiments, a second, or third, or fourth, or fifth administration is within about a day, or 1 week, or 1 month of the first.

In some embodiments, the toxin administered may be may be one or more of the agents described herein. In various embodiments, the second, or third, or fourth, or fifth pulse is a modulator of autophagy, cell survival, cell death, proliferation, regeneration, and the like, as described herein and as known in the art.

In another embodiment, the methods described herein comprise administering (i) an additional amount of a first toxin to the animal and/or (ii) a second toxin to the animal. In some embodiments, the methods described herein comprise further comprise observing a reduction in the rate of formation, growth or expansion of patches of ocular tissue atrophy or patches of tissue loss.

In various embodiments, doses of the toxins are known to those in the art. For example, a suitable dosage may be in a range of about 0.1 mg/kg to about 100 mg/kg of body weight of the subject, for example, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, or about 100 mg/kg body weight, inclusive of all values and ranges therebetween.

In one embodiment, the toxin is NaIO₃ and the dose is about 50 mg/kg of body weight. In one embodiment, the toxin is NaIO₃ and the dose is about 45 mg/kg of body weight. In one embodiment, the toxin is NaIO₃ and the dose is about 30 mg/kg of body weight.

### Pharmaceutically Acceptable Salts and Excipients

Any agent described herein can possess a sufficiently basic functional group, which can react with an inorganic or organic acid, or a carboxyl group, which can react with an inorganic or organic base, to form a pharmaceutically acceptable salt. A pharmaceutically acceptable acid addition salt is formed from a pharmaceutically acceptable acid, as is well known in the art. Such salts include the pharmaceutically acceptable salts listed in Journal of Pharmaceutical Science, 66, 2-19 (1977) and The Handbook of Pharmaceutical Salts; Properties, Selection, and Use. P. H. Stahl and C. G. Wermuth (eds.), Verlag, Zurich (Switzerland) 2002.

Pharmaceutically acceptable salts include, by way of non-limiting example, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, camphorsulfonate, pamoate, phenylacetate, trifluoroacetate, acrylate, chlorobenzoate, dinitrobenzoate, hydroxybenzoate, methoxybenzoate, methylbenzoate, o-acetoxybenzoate, naphthalene-2-benzoate, isobutyrate, phenylbutyrate, α-hydroxybutyrate, butyne-1,4-dicarboxylate, hexyne-1,4-dicarboxylate, caprate, caprylate, cinnamate, glycollate, heptanoate, hippurate, malate, hydroxymaleate, malonate, mandelate, mesylate, nicotinate, phthalate, teraphthalate, propiolate, propionate, phenylpropionate, sebacate, suberate, p-bromobenzenesulfonate, chlorobenzenesulfonate, ethylsulfonate, 2-hydroxyethylsulfonate, methylsulfonate, naphthalene-1-sulfonate, naphthalene-2-sulfonate, naphthalene-1,5-sulfonate, xylenesulfonate, and tartarate salts.

The term "pharmaceutically acceptable salt" also refers to a salt of the compounds of the present invention having an acidic functional group, such as a carboxylic acid functional group, and a base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or tri-alkylamines, dicyclohexylamine; tributyl amine; pyridine; N-methyl, N-ethylamine; diethylamine; triethylamine; mono-, bis-, or tris-(2-OH-lower alkylamines), such as mono-; bis-, or tris-(2-hydroxyethyl)amine, 2-hydroxy-tert-butylamine, or tris-(hydroxymethyl)methylamine, N,N-di-lower alkyl-N-(hydroxyl-lower alkyl)-amines, such as N,N-dimethyl-N-(2-hydroxyethyl)amine or tri-(2-hydroxyethyl)amine; N-methyl-D-glucamine; and amino acids such as arginine, lysine, and the like.

In some embodiments, an agent of the invention is in the form or a pharmaceutically acceptable salt. In some embodiments, the pharmaceutically acceptable salt is a sodium salt. In some embodiments, particularly where R₃ of the compounds of **Formula I** is hydrogen, the compounds of **Formula I** are in the form of a pharmaceutically acceptable salt. In some embodiments, the compound of **Formula I** is a pharmaceutically acceptable salt of bindarit. In some embodiments, methotrexate is in the form or a pharmaceutically acceptable salt. In some embodiments, the pharmaceutically acceptable salt of methotrexate is methotrexate sodium.

Further, any agent described herein can be administered to a subject as a component of a composition that comprises a pharmaceutically acceptable carrier or vehicle. Such compositions can optionally comprise a suitable amount of a pharmaceutically acceptable excipient so as to provide the form for proper administration.

Pharmaceutical excipients can be liquids, such as water and oils, including those of petroleum, animal, vegetable, or synthetic origin, such as peanut oil, soybean oil, mineral oil, sesame oil and the like. The pharmaceutical excipients can be saline, gum acacia, gelatin, starch paste, talc, keratin, colloidal silica, urea and the like. In addition, auxiliary, stabilizing, thickening, lubricating, and coloring agents can be used. In one embodiment, the pharmaceutically acceptable excipients are sterile when administered to a subject. Water is a useful excipient when any agent described herein is administered intravenously. Saline solutions and aqueous dextrose and glycerol solutions can also be employed as liquid excipients, specifically for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Any agent described herein, if desired, can also comprise minor amounts of wetting or emulsifying agents, or pH buffering agents.

### Formulations, Administration, and Dosing

Any agent described herein can take the form of solutions, suspensions, emulsion, intra-ocular injection, intra-vitreal injection, topical ophthalmic drops, sub-conjunctival injection, sub-Tenon's injection, trans-scleral formulations, tablets, pills, pellets, capsules, capsules containing liquids, powders, sustained-release formulations, suppositories, emulsions, aerosols, sprays, suspensions, or any other form suitable for use. In one embodiment, the composition is suitable for an intra-vitreal injection (*see, e.g.,* ILUVIEN or similar forms) or implantation (*see, e.g.,* RETISERT or similar forms). In one embodiment, the composition is in the form of a capsule (*see, e.g.,* U.S. Patent No. 5,698,155). Other examples of suitable pharmaceutical excipients are described in Remington's Pharmaceutical Sciences 1447-1676 (Alfonso R. Gennaro eds., 19th ed. 1995).

In one embodiment, any agent described herein is formulated for ophthalmic administration, including, for example, intravitreal or intraocular administration, topical, and/or intravenous administration. Typically, compositions for administration comprise sterile isotonic aqueous buffer. Where necessary, the compositions can also include a solubilizing agent. Also, the agents can be delivered with a suitable vehicle or delivery device as known in the art. Combination therapies outlined herein can be co-delivered in a single delivery vehicle or delivery device. Compositions for administration can optionally include a local anesthetic such as lignocaine to lessen pain at the site of the injection.

In one embodiment, any agent described herein is formulated in accordance with routine procedures as a composition adapted for intra-ocular administration.

In one embodiment, any agent described herein is formulated in accordance with routine procedures as a composition adapted for oral administration to human beings. Compositions for oral delivery can be in the form of tablets, lozenges, aqueous or oily suspensions, granules, powders, emulsions, capsules, syrups, or elixirs, for example. Orally administered compositions can comprise one or more agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving any agent described herein are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be useful. Oral compositions can include standard excipients such as mannitol, lactose, starch, magnesium stearate, sodium saccharin, cellulose, and magnesium carbonate. In one embodiment, the excipients are of pharmaceutical grade.

The ingredients may be supplied either separately or mixed together in unit dosage form, for example, as a pre-mixed solution, dry lyophilized-powder, or water-free concentrate in a hermetically sealed container such as an ampule, pre-filled syringe, or sachette indicating the quantity of active agent. Where any agent described herein is to be administered by intra-vitreal or intra-ocular delivery, it can be dispensed, for example, with a pre-filled syringe or injector, or in an ampule for withdrawal into a suitable syringe. Where any agent described herein is to be administered by infusion, it can be dispensed, for example, with an infusion bottle containing sterile pharmaceutical grade water or saline. Where any agent described herein is to be administered by injection, an ampule of sterile water for injection or saline can be provided so that the ingredients can be mixed prior to administration.

Any agent described herein can be administered by controlled-release or sustained-release means or by delivery devices that are well known to those of ordinary skill in the art. Examples include, but are not limited to, those described in U.S. Patent Nos. 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,556. Such dosage forms can be useful for providing controlled- or sustained-release of one or more active ingredients using, for example, hydropropylmethyl cellulose, other polymer matrices, gels, permeable membranes, osmotic systems, multilayer coatings, microparticles, nanoparticles, liposomes, microspheres, or a combination thereof to provide the desired release profile in varying proportions. Suitable controlled- or sustained-release formulations known to those skilled in the art, including those described herein, can be readily selected for use with the active ingredients of the agents described herein. The invention thus provides single unit dosage forms suitable for oral administration such as, but not limited to, tablets, capsules, gelcaps, and caplets that are adapted for controlled- or sustained-release.

Controlled- or sustained-release of an active ingredient can be stimulated by various conditions, including but not limited to, changes in pH, changes in temperature, stimulation by an appropriate wavelength of light, concentration or availability of enzymes, concentration or availability of water, or other physiological conditions or compounds.

Compositions can be prepared according to conventional mixing, granulating, coating or polymerization methods, respectively, and the present compositions can comprise, in one embodiment, from about 0.1% to about 99%; and in another embodiment from about 1% to about 70% of any agent described herein by weight or volume.

In another embodiment, any agent described herein acts synergistically when co-administered with another agent and is administered at doses that are lower than the doses commonly employed when such agents are used as monotherapy.

For example, the dosage any agent described herein as well as the dosing schedule can depend on various parameters, including, but not limited to, the ocular disorder being treated, the subject's general health, and the administering physician's discretion. Any agent described herein, can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concurrently with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of an additional therapeutic agent, to a subject in need thereof. In various embodiments any agent described herein is administered 1 minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, an agent of the invention, including compounds of **Formula I**, methotrexate or their pharmaceutically acceptable salts, and one or more additional therapeutic agents are administered within 3 hours. In another embodiment, an agent of the invention, including compounds of **Formula I**, methotrexate or their pharmaceutically acceptable salts, and one or more additional therapeutic agents are administered at 1 minute to 24 hours apart.

The amount of any agent described herein that is admixed with the carrier materials to produce a single dosage can vary depending upon the subject being treated and the particular mode of administration. In *vitro* or *in vivo* assays can be employed to help identify optimal dosage ranges.

In general, the doses that are useful are known to those in the art. For example, doses may be determined with reference Physicians' Desk Reference, 66th Edition, PDR Network; 2012 Edition (December 27, 2011).

The dosage of any agent described herein can depend on several factors including the severity of the condition, whether the condition is to be treated or prevented, and the age, weight, and health of the subject to be treated. Additionally, pharmacogenomic (the effect of genotype on the pharmacokinetic, pharmacodynamic or efficacy profile of a therapeutic) information about a particular subject may affect dosage used. Furthermore, the exact individual dosages can be adjusted somewhat depending on a variety of factors, including the specific combination of the agents being administered, the time of administration, the route of administration, the nature of the formulation, the rate of excretion, the particular ocular disorder being treated, the severity of the disorder, and the anatomical location of the disorder. Some variations in the dosage can be expected.

In some embodiments, the administering is effected orally or intra-vascularly, or intraocularly, or periocularly, or to the ocular surface

When ophthalmically administered to a human, for example, intravitreally, the dosage of an agent of the invention, including, for example, **Formula I**, methotrexate or a pharmaceutically acceptable salt thereof and/or additional therapeutic agent is normally 0.003 mg to 5.0 mg per eye per administration, or 0.03 mg to 3.0 mg per eye per administration, or 0.1 mg to 1.0 mg per eye per administration. In one embodiment, the dosage is 0.03 mg, 0.3 mg, 1.5 mg or 3.0 mg per eye. In another embodiment, the dosage is 0.5 mg per eye. The dosage can range from 0.01 mL to 0.2 mL administered per eye, or 0.03 mL to 0.15 mL administered per eye, or 0.05 mL to 0.10 mL administered per eye. In one embodiment, the administration is 400 µg of compound, monthly for at least three months.

Generally, when orally administered to a mammal, the dosage of any agent described herein may be 0.001 mg/kg/day to 100 mg/kg/day, 0.01 mg/kg/day to 50 mg/kg/day, or 0.1 mg/kg/day to 10 mg/kg/day. When orally administered to a human, the dosage of any agent described herein is normally 0.001 mg to 1000 mg per day, 1 mg to 600 mg per day, or 5 mg to 30 mg per day. In one embodiment, oral dosage is 600 mg per day. In one embodiment, the oral dosage is two 300 mg doses per day. In another embodiment, oral dosage is 7.5 mg per week to 15 mg per week.

For administration of any agent described herein by parenteral injection, the dosage is normally 0.1 mg to 250 mg per day, 1 mg to 20 mg per day, or 3 mg to 5 mg per day. Injections may be given up to four times daily. Generally, when orally or parenterally administered, the dosage of any agent described herein is normally 0.1 mg to 1500 mg per day, or 0.5 mg to 10 mg per day, or 0.5 mg to 5 mg per day. A dosage of up to 3000 mg per day can be administered.

In some embodiments, it may be desirable to administer one or more any agent described herein to the eye. Administration may be, by way of non-limiting example, intra-ocular, intra-vitreal, topical (including, but not limited to, drops and ointment), sub-conjunctival, sub-Tenon's, trans-scleral, suprachoroidal, subretinal, and via iontophoresis.

Other routes of administration may also be used, such as, for example: intradermal, intramuscular, intraperitoneal, intravenous, subcutaneous, intranasal, epidural, oral, sublingual, intranasal, intracerebral, intravaginal, transdermal, rectally, by inhalation, or topically, particularly to the ears, nose, eyes, or skin.

The mode of administration can be left to the discretion of the practitioner, and depends in-part upon the site of the medical condition. In most instances, administration results in the release of any agent described herein into the bloodstream.

Any agent described herein can be administered orally. Such agents can also be administered by any other convenient route, for example, by intravenous infusion or bolus injection, by absorption through epithelial or mucocutaneous linings (*e.g*., oral mucosa, rectal and intestinal mucosa, *etc*.) and can be administered together with another biologically active agent. Administration can be systemic or local. Various delivery systems are known, *e.g*., encapsulation in liposomes, microparticles, microcapsules, capsules, etc., and can be used to administer.

Further methods of administration include but are not limited to intra-ocular, intra-vitreal, topical ocular (including but not limited to drops, ointments and inserts), sub-conjunctival, sub-Tenon's, suprachoroidal, trans-scleral, intradermal, intramuscular, intraperitoneal, intravenous, subcutaneous, intranasal, epidural, oral, sublingual, intranasal, intracerebral, intravaginal, transdermal, rectally, by inhalation, or topically, particularly to the ears, nose, eyes, or skin. In some embodiments, more than one of any agent described herein is administered to the eye. Administration may be, by way of non-limiting example, intra-ocular, intra-vitreal, topical (including, but not limited to, drops and ointment), sub-conjunctival, sub-Tenon's, trans-scleral, and iontophoresis. The mode of administration can be left to the discretion of the practitioner, and depends in-part upon the site of the medical condition. In most instances, administration results in the release into the bloodstream.

In specific embodiments, it may be desirable to administer locally to the area in need of treatment.

In another embodiment, delivery can be in a vesicle, in particular a liposome (*see* Langer, 1990, Science 249:1527-1533; Treat et al., in Liposomes in the Therapy of Infectious Disease and Cancer, Lopez-Berestein and Fidler (eds.), Liss, New York, pp. 353-365 (1989). In yet another embodiment, delivery can be in a controlled release system. In one embodiment, a slow release intra-ocular device may be used. In some embodiments, this device consists of a locally delivered erodible or non-erodable liquid, gel, polymer, etc.

In another embodiment, polymeric materials can be used (*see* Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J. Macromol. Sci. Rev. Macromol. Chem. 23:61; *see also* Levy et al., 1985, Science 228:190; During et al., 1989, Ann. Neurol. 25:351; Howard et al., 1989, J. Neurosurg. 71:105). In another embodiment, a controlled-release system can be placed in proximity of the target area to be treated, e.g., the retina, thus requiring only a fraction of the systemic dose (*see*, *e.g*., Goodson, in Medical Applications of Controlled Release, supra, vol. 2, pp. 115-138 (1984)). Other controlled-release systems discussed in the review by Langer, 1990, Science 249:1527-1533) may be used.

Administration of any agent described herein can, independently, be one to four times daily or one to four times per month or one to six times per year or once every two, three, four or five years. Administration can be for the duration of one day or one month, two months, three months, six months, one year, two years, three years, and may even be for the life of the subject. Chronic, long-term administration will be indicated in many cases. The dosage may be administered as a single dose or divided into multiple doses. In general, the desired dosage should be administered at set intervals for a prolonged period, usually at least over several weeks or months, although longer periods of administration of several months or years or more may be needed.

The dosage regimen utilizing any agent described herein can be selected in accordance with a variety of factors including type, species, age, weight, sex and medical condition of the subject; the severity of the condition to be treated; the route of administration; the renal or hepatic function of the subject; the pharmacogenomic makeup of the individual; and the specific compound of the invention employed. Any agent described herein can be administered in a single daily dose, or the total daily dosage can be administered in divided doses of two, three or four times daily. Furthermore, any agent described herein can be administered continuously rather than intermittently throughout the dosage regimen.

### Methods of Treatment

In various aspects, the present invention provides an agent of the invention for use in a method for treating an ocular disorder described herein. In these aspects, the "agent of the invention" comprises compounds useful for both monotherapy and combination therapy (e.g. as an additional therapeutic agent).

In some aspects, the invention relates to an agent of the invention for use in a method for treating an ocular disorder in a subject in need thereof, wherein the subject has an increased or decreased expression or activity of one or more of CD64, IDO, SOCS1, CXCL10 Marco, Socs3, Nos2, Il12b, Ptgs2 (Cox2), Il23α (Il23p19), and Ido1.

In some embodiments, the subject has an increased or decreased expression or activity of one or more of MRC1, TGM2, CD23, CCL22 Relma (Fizz1, Retnla), Socs2, Irf4, Chia (Amcase), Chi3l1 (Gp39, Ykl40), Chi3l2 (Ykl39), Chi3l3(Ym1), Cxcl13, Ccl12, Ccl24, and Klf4.

In some embodiments, the subject has an increased or decreased expression or activity of one or more of Ccl5, CD163, Cx3Cr1, Faslg, Gfap, Csf2, Icam1, Ifng, Il10, Il12b, Il13, Il17a, Il18, Il1b, Il22, Il4, Il6, Klf4, Mrc1, Myd88, Nlrp3, Nos2, Ppary, Tgfb1, Tlr4, Tnf, Vcam1, Ccl2, Ccl5, Ccl7, Ccr2, Socs1, Socs3, Stat1, Stat3, and Stat6. In some embodiments, the subject has an increased or decreased expression or activity of one or more of Myd88, Klf4, Nlrp3, Ccl2, Ccl5, Ccl7, Socs1, Socs3, Stat1, Stat3, and Stat6.

In some embodiments, bindarit leads to differential gene expression of any of the following, directly or indirectly Ccl5, CD163, Cx3Cr1, Faslg, Gfap, Csf2, Icam1, Ifng, Il10, Il12b, 1113, Il17a, 1118, Il1b, 1122, 114, 116, Klf4, Mrc1, Myd88, Nlrp3, Nos2, Ppary, Tgfb1, Tlr4, Tnf, Vcam1, Ccl2, Ccl5, Ccl7, Ccr2, Socs1, Socs3, Stat1, Stat3, and Stat6, optionally in macrophages or RPE directly. In some embodiments, bindarit modulates NFκB.

In some embodiments, the subject is not undergoing treatment with and/or is unresponsive to one or more of an anti-factor D antibody (e.g. lampalizumab (Genentech)), an anti-β-amyloid (anti-Aβ) antibody (e.g. GSK933776 (GSK)), a corticosteroid (e.g. fluocinolone acetonide), MC-1101 (MacuCLEAR), a CD34+ stem cell therapy, an anti-VEGF antibody (e.g. Ranibizumab), brimonidine (Alphagan), an anti-C5 complement antibody (e.g. LFG316 (Novartis), doxycycline (ORACEA), emixustat hydrochloride, sirolimus (RAPAMUNE), and glatiramer acetate (COPAXONE). In some embodiments, the subject has evidence of AMD as confirmed by the presence of at least 1 druse greater than about 125 µm in diameter. In some embodiments, the subject has no evidence of prior or active choroidal neovascularization (CNV). In some embodiments, the subject has one or more well-demarcated GA lesions of a total area of about 2 to about 20 mm² in one or more eye. In some embodiments, the subject has a best-corrected visual acuity score of greater than about 35 letters or a Snellen VA equivalent of about 20/200 or better.

An evaluation of any of the treatments disclosed herein can comprise optical imaging, including, by way of non-limiting example, cSLO, FAF, OCT (including with cross-sectional, three-dimensional and *en face* viewing), SD-OCT (with cross-sectional, three-dimensional and *en face* viewing), or other imaging modalities including other wavelengths of fluorescence (e.g. wavelengths ranging from blue to infrared, e.g., 390 nm to 1 mm, including, for example, blue light, white light, red-free, near infra-red, or infrared).

### Subjects and/or Animals

In some embodiments, the subject and/or animal is a mammal, e.g., a human, mouse, rat, guinea pig, dog, cat, horse, cow, pig, rabbit, sheep, or non-human primate, such as a monkey, chimpanzee, or baboon. In other embodiments, the subject and/or animal is a non-mammal, such, for example, a zebrafish. In some embodiments, the subject and/or animal may comprise fluorescently-tagged cells (with e.g. GFP). In some embodiments, the subject and/or animal is a transgenic animal comprising a fluorescent cell, such as, for example, an RPE cell and/or an immune cell. In some embodiments, the subject and/or animal is a human. In some embodiments, the human is a pediatric human. In other embodiments, the human is an adult human. In other embodiments, the human is a geriatric human. In other embodiments, the human may be referred to as a patient.

In certain embodiments, the human has an age in a range of from about 0 months to about 6 months old, from about 6 to about 12 months old, from about 6 to about 18 months old, from about 18 to about 36 months old, from about 1 to about 5 years old, from about 5 to about 10 years old, from about 10 to about 15 years old, from about 15 to about 20 years old, from about 20 to about 25 years old, from about 25 to about 30 years old, from about 30 to about 35 years old, from about 35 to about 40 years old, from about 40 to about 45 years old, from about 45 to about 50 years old, from about 50 to about 55 years old, from about 55 to about 60 years old, from about 60 to about 65 years old, from about 65 to about 70 years old, from about 70 to about 75 years old, from about 75 to about 80 years old, from about 80 to about 85 years old, from about 85 to about 90 years old, from about 90 to about 95 years old or from about 95 to about 100 years old.

In other embodiments, the subject is a non-human animal, and therefore the invention pertains to veterinary use. In a specific embodiment, the non-human animal is a household pet. In another specific embodiment, the non-human animal is a livestock animal.

In various embodiments, a subject's and/or an animal's eye comprises (i) a fluorescent compound in an amount effective to indicate the presence of an ocular disorder in the subject and/or animal and (ii) a toxin in an amount effective to induce atrophy of ocular tissue. In some embodiments, such a subject and/or animal is administered an agent of the invention or is not administered an agent of the invention.

In various embodiments, RPE and immune cells are evaluated and/or effected. In some embodiments, immune cells include cells of a subject's and/or animal's innate immune system. In some embodiments, such cells include, but are not limited to, macrophage, monocyte, and microglial cells. In various embodiments, the invention provides for detecting a presence, detecting an absence, or measuring an amount of immune cells in a subject's and/or animal's eye

This invention is further illustrated by the following non-limiting examples. Examples not falling within the scope of the claims are for illustrative purposes only.

### EXAMPLES

### Example 1: Systemic Injection of the RPE Toxin, NaIO₃, Induces Complex Patterns of FAF Similar to Those of AMD and/or RPD

The RPE toxin, sodium iodate (NaIO₃) generated patchy loss of the RPE and hypofluorescent DNIRA, in the rat eye similar to geographic atrophy (GA). This model not only developed RPE atrophy, it faithfully reproduced complex patterns of FAF associated with aggressive clinical disease, most closely resembling advanced dry AMD, RPD, and the diffuse trickling forms of dry AMD.

Materials: ICG (Cardiogreen), sodium iodate, Harris haematoxylin and eosin, were from Sigma-Aldrich (Oakville, ON, Canada). Tropicamide, 0.8%, in 5% phenylephrine hydrochloride solution (Diophenyl-T) was from Sandoz Canada Inc (Boucherville, QC, Canada), and GenTeal lubricating eye drops were from Novartis Pharmaceuticals Canada Inc (Dorval, QC, Canada). Paraformaldehyde, 32% in phosphate buffered saline (PBS), was from Electron Microscopy Sciences (Hatfield, PA). Mouse anti-rat CD68 antibody was from AbD Serotec (Oxford, UK), and mouse IgG was from Santa Cruz Biotechnology (Santa Cruz, CA, USA). Rabbit anti-lba-1 was from Wako Pure Chemical Industries Ltd (Osaka, Japan). Alexa-labeled fluorescent goat antimouse secondary antibody, Isolectin IB₄ Conjugates and TO-PRO-3 nucleic acid stain were from Invitrogen (Camarillo, CA, USA). Dako fluorescent mounting medium was from Dako North America (Burlington, ON, Canada).

Animal procedures: all procedures were performed in accordance with the Canadian Council on Animal Care and with adherence to the *ARVO Statement for the Use of Animals in Ophthalmic and Vision Research.* For all experiments, Sprague Dawley (SD) rats aged 6-10 weeks were kept at a 12 hour dark/light cycle, with food and water *ad libitum.* For evaluation, rats were anesthetized with ketamine (100 mg/kg) and xylazine (10 mg/kg), and pupils dilated with Diophenyl-T. Eyes were conditioned using GenTeal lubricating eye drops. Animals were humanely sacrificed by intracardiac injection of T61 (Intervet Canada, Whitby, Canada), either after a single imaging session or after serial imaging.

Sodium iodate (45% solution), was prepared fresh for each set of experiments in 0.9% sodium chloride and injected to a final concentration of 45 mg/kg body weight. A total of 192 eyes were evaluated.

Fundus imaging was performed using a commercially available confocal scanning laser ophthalmoscope, (cSLO, Spectralis, Heidelberg Retinal Angiography, HRA-II; Heidelberg Engineering GmbH, Germany), and the Eye Explorer Image Capture system, version 1.1.10. Images were captured at a wavelength of 488 nm excitation with 500 nm barrier filter for fluorescein angiography without the barrier filter for red free (RF, green dominant) imaging, at 795/810 nm excitation/emission for ICG angiography, and with a 830 nm laser for infrared reflectance imaging.

To evaluate 488 nm FAF, cSLO imaging was performed in the absence of fluorescein dye. Fluorescein was not given at baseline or at any time during each study. In cases when it was necessary to simultaneously image the vasculature and provide landmarks for FAF image analysis, ICG was injected via tail vein, at 2 mg/kg or 5 mg/kg, into a previously inserted 23 gauge tail vein catheter. In a small number of clearly indicated situations, such as initial characterization of the model, fluorescein dextran (200 kD) was provided intravenously. Results from these latter studies using fluorescein angiography were performed in a subset of animals and not included in FAF analysis.

Fundus images were routinely obtained at baseline (day 0, i.e. prior to systemic NaIO₃ injection), and at days 3 or 4 (3/4), days 7 or 8 (7/8) and days 13 or 14 (13/14) after NaIO₃. In addition, for initial characterization of the model, images were acquired at 24 hours post- NaIO₃, at 21 to 28 days and out to 88 days (3 months). Where possible, composite images consisting of at least nine images were taken in the following order: optic nerve head, upper right, right, lower right, inferior, lower left, left, upper left, and the superior fields. In some cases, a second or third complete or partial ring of images was obtained more peripherally, typically by realigning the animal relative to the cSLO as needed. By contrast, in other cases, in which the full nine images could not be captured, and a partial composite image was generated.

High resolution spectral domain OCT images were obtained (Envisu R2200 VHR Animal SDOIS System, Bioptogen, USA). A scans were acquired at a rate of 36,000 per second, with a lateral resolution of approximately 2.8 µm at the level of the retina.

Tissue Analysis: hemotoxylin and eosin (H & E) histology of paraffin-embedded tissue was performed as known in the art after fixation with Davidson's fixative. Paraffin-embedded sections were viewed with a Nikon Upright E800 Microscope. Deparaffinization and rehydration procedure was followed as in H&E tissue processing.

For immunohistochemistry, sections were blocked in 5% BSA in TBS at room temperature and incubated overnight with anti-CD68 antibody at 4°C with gentle shaking. After rinsing, samples incubated with secondary antibody for 2 hours at room temperature, and washed again. For negative control, same-species IgG was used at equivalent maximum molar concentrations as the primary antibody. Nuclei were counter-stained with TO-PRO-3.

Confocal and Fluorescent Microscopy: images were acquired using a TCS SL Confocal fluorescent microscope (Leica Microsystems, GmbH, Wetzlar, Germany), and images captured and analyzed using Leica Confocal Software. Upright epifluorescent microscope (Olympus BX50) was also used to view the autofluoresent signals of the cells under 488 nm wave length light. 3D reconstructions of confocal serial z-sections were performed using Imaris software version 7.4.2 (Bitplane).

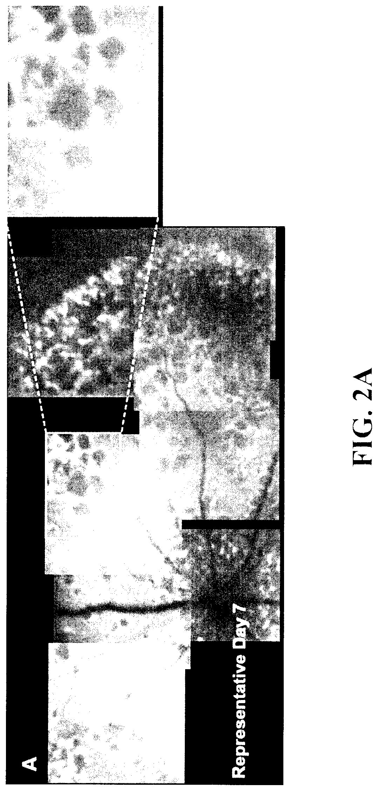

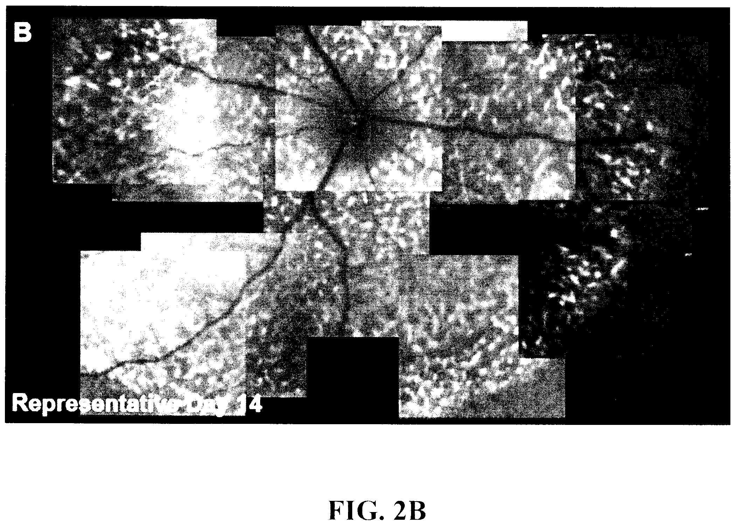

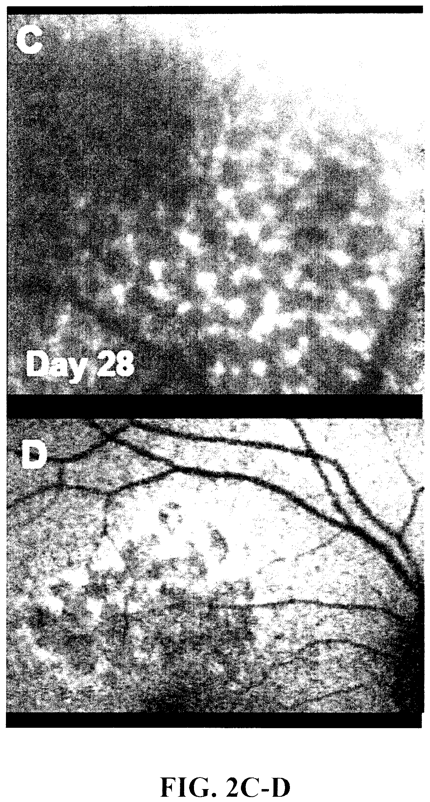

FAF and angiographic imaging *in vivo* of the normal Sprague Dawley rat eye produced a faint, homogenous ground glass glow interrupted by radially-arranged retinal blood vessels that block RPE fluorescence and so appear dark, and by a central hypofluorescent circle at the optic nerve head (ONH), where RPE is absent (**FIGs. 1A-D**). Three days after systemic NaIO₃ injection, a single area of iso- or slightly hyperfluorescent FAF with hyperfluorescent borders appeared, and took the shape of a discrete island, sector, or 360° ring (**FIGs. 1A-D**). Islands or sectors are consistently located inferior to the ONH or in the inferior hemiretina (**FIGs. 1A-D**). When a 360° ring is present, it typically encompasses much of the fundus, with one border adjacent to, surrounding, the ONH and the other in the mid- to far- retinal periphery. Large 360° rings were the most commonly observed shape, occurring in 102/110 (93%) of eyes treated with sodium iodate **(****FIG. 2A-D**). In all cases, the islands, sectors or rings approached the ONH but were not contiguous with it (**FIGS. 1A****-D-3A-C**). These and the following observations were confirmed in over 160 eyes.

Beginning approximately 4-5 days after NaIO₃ injection, a marked reticular or curvilinear pattern of alternating hyper/hypo-fluorescent FAF appeared within the islands, sectors or rings (**FIGS. 1A-D**). By day 7, this pattern was pronounced. In small lesions, it completely filled the island or sector and emerged rapidly. In large 360° rings, it was at first pronounced at the proximal and distal borders and continued to emerge over the next weeks, whereby its curvilinear components contributed to partial loops, ovals, circles, rosettes, and scalloped or "paw-print" patterns of FAF (**FIGS. 2A-D**). In some regions, curvilinear, round or oval shapes coalesce and appeared darker than the surrounding areas, appearing as homogenously dark grey patches of hypofluorescent FAF. With time, the hyperfluorescent reticular pattern matured *in situ,* becoming more granular and less smoothly curvilinear (**FIGS. 2A-D**). However, even as late as 20 weeks, the last time-point analyzed, the borders retained their hyperfluorescent reticular pattern and continued to creep slightly toward the retinal periphery.

Fluorescein angiography performed in a subset of animals confirmed that the reticular pattern does not correspond with the retinal or choroidal vasculature (note that fluorescein-dextran, a 488 nm dye, was not given to animals that underwent FAF imaging, unless indicated) (**FIGS. 1A-D**). However, ICG angiography revealed a permeability change that occurred at the chorio-retinal interface, across the RPE and BM, approximately two to three days after NaIO₃ that coincides with, both spatially and temporally, the emergence of the islands, sectors of rings of FAF (**FIGS. 1A-D**). This increase in permeability was transient and resolved by Day 7, the next timepoint routinely evaluated. The retinal vessels did not leak. In the NaIO₃ model, H&E staining confirms that fluid accumulates in the subretinal space. This was also observed by ultra-high resolution OCT *in vivo.*

To determine the pathological correlate of the FAF pattern observed *in vivo,* excised retinal wholemounts were viewed at low magnification under white light and by epifluorescent microscopy at 488 nm - the same wavelength used for *in vivo* imaging, with no fluorescent IHC labeling. Depending on subsequent tissue analysis, two microscopes were used for this purpose: an epifluorescent device with a narrow excitation/emission window (488/520nm), and the Inverted Leica DM IRE2 viewing system of a confocal microscope with a mercury arc lamp and long-pass (rather than band-pass) filter providing fluorescence from greater than 515 nm following excitation with a 450-490 nm band. Under white light, the normal rat retina appears fairly homogenous throughout, with some artifactuous deformation (**FIG. 3A**). By contrast, a subtle off-white, fine reticular pattern becomes apparent in samples obtained 7 days after NaIO₃ injection. By day 14, this pattern is more apparent and seen to correspond with small, outer retinal folds best illustrated at the cut edge of the retina (**FIG. 3B**). Viewed under 488 nm fluorescent light, this same curvilinear pattern is apparent. Though apparent by day 7 after injection, increasing autofluorescence makes this pattern more visible by Day 14 (**FIG. 3C**). Using higher magnification epifluorescent microscopy, it was demonstrated that this lacy, curvilinear pattern coincides with the spatial distribution of autofluorescent cells that appear to align with grooves or folds of the outer retina (**FIGS. 3A-**C).

**FIG. 4A-C** shows a comparison of the clinical features of RPD with tissue findings in the NaIO₃ model. In addition to clinical OCT imaging of the patient with RPD (that shows a base-down pyramidal structure in the subretinal space), color and red-free fundus images were obtained (**FIG. 4A-C**). Red free images showed small dark spots that constitute individual pseudodrusen of RPD (**FIG. 4A**, top image). These are identified using dark grey overlay. We further highlight, circumscribed by circles, the presence of so-called "target lesions" that characterize RPD. These appear as dark spots with lighter centers. In parallel, a schematized illustration of folded outer rat retina, both *en face* and in cross-section that, when falsely colored and presented as black and white images - both "positive" and "negative" - appears to correlate directly with target-like lesions, and halos of RPD. Taken together, these data suggest that the 2D patterns of dark pseudodrusen within a lacy or reticular pattern of circles, halos and target lesion with dark centers viewed *en face,* in 2D, correspond with elevated rings and craters, considered volumetrically, in 3D. The inter-pseudodrusen matrix consists of ribbons of persistent subretinal inflammation that when cross-sectioned appear as pyramids or spikes by OCT. The lost outer retina between these structures appears dark. This places the OCT-defined pseudodrusen adjacent to the dark patches of RPD. Without wishing to be bound by theory, this correlation with structural deformity of the retina further explains why RPD can be visualized in several imaging modalities and are not limited to a particular wavelength, such as 488 nm, alone as would occur if the signal were fluorophore dependent.

### Example 2: DNIRA of a Rat Eye After Systemic ICG Administration Identifies the Retinal Pigment Epithelial (RPE) Layer In Vivo

It is demonstrated that a near infra-red dye, ICG, can label the RPE/outer retina following systemic injection and so enhance the detection of RPE and RPE atrophy. The observed change in DNIRA using the ICG excitation/emission filters is useful in lieu of FAF in models of disease to identify areas of RPE/outer retinal loss.

ICG dye, or PBS, was injected systemically in 46 Sprague Dawley rats at doses of 0.35 and 5.0 mg/kg, and the fundus was evaluated *in vivo* using confocal scanning laser ophthalmoscopy (cSLO), with 795/810 nm excitation/emission filters in place, prior to injection, and at days 2, 7, and 21 thereafter. Electroretinography (ERG) was performed to evaluate potential toxicity. In a subset of animals, an RPE toxin was injected to induce RPE damage or loss.

Specifically, for animal studies, animals were handled in accordance with the Association for Research in Vision & Ophthalmology (ARVO) guidelines for the humane use of animals in ophthalmic research, and according to the St. Michael's Hospital Animal Care Committee guidelines. 46 Sprague Dawley (SD) rats aged 6-10 weeks, weighing 200-300 g were kept on a 12 hour dark/light cycle, with food and water *ad libitum.* Animals were anesthetized with a combination of ketamine (100 mg/kg) and xylazine (10 mg/kg), and pupils dilated with a single drop of 0.8% tropicamide in 5% phenylephrine hydrochloride solution (Diophenyl-T, Sandoz Canada Inc). GenTeal lubricating eye drops (Novartis, Canada), were repeatedly applied to the corneal surface during all procedures.

CSLO: *in vivo* images were acquired using a commercially available cSLO (Heidelberg Retinal Angiography, HRA-2, Heidelberg Engineering, Germany). Images were obtained in the red-free, FAF (488/500 nm excitation/emission), NIR channel (830 nm) and ICG channel (795/810 nm excitation/emission).

ICG and Fluorescein Angiography: ICG dye (Cardiogreen, Sigma, Cat # I2633) was freshly prepared prior to experimentation to a final stock concentration of 5.0 mg/ml. A 23-gauge catheter was inserted into the tail vein, and ICG dye infused at doses of 0.35, or 5.0 mg/kg. Images were taken prior to injection (baseline), during dye circulation, and at various intervals thereafter out to 20 minutes. In a subset of animals, fluorescein-dextran (200 kD) (Sigma, Cat # FD2000S) solution, at 5.0 mg/ml, was injected IV via tail vein catheter to yield a final dose of 5.0 mg/kg. ICG and fluorescein angiography was performed simultaneously in a subset of animals only, otherwise fluorescein was not injected. Angiographic images were obtained in the fluorescein and ICG channels with excitation and emission filters of 488/500 nm and 795/810 nm respectively. Control animals received PBS.

DNIRA: DNIRA images were obtained in the days and weeks after ICG injection using the ICG angiography settings, i.e. with excitation/emission filters, but without re-injection of dye at 24 hours, 48 hours, 3 days, 7 days, 21 days and 28 days after angiography. Angiography was not performed again during the time-course of the study.

Toxin administration: in a subset of animals (n=7), the RPE toxin NaIO₃ (Sigma, cat #424064) was injected systemically via a 23G catheter inserted into the tail vein at a dosage of 45 mg/kg body weight. In these animals, ICG angiography at 0.35mg/kg was performed immediately prior to NaIO₃ injection. DNIRA was performed 7 days after ICG and NaIO₃ injection

Electroretinography (ERG): animals receiving ICG or PBS were evaluated using the Espion (DiagnosysLLC, USA) mini-Ganzfeld ERG system. Following anesthesia, animals were placed on an electrically silent heating pad and gold coil electrodes placed on the corneal surface after application of GenTeal lubricating drops. Following a short train of dim flashes (0.01 candela s/m², 1.0 Hz), a scotopic b-wave amplitude was evaluated using a single bright flash (3 candela s/m²) that we previously determined consistently produces a response close to the maximum b-wave amplitude. Student t-test was used to compare post-injection amplitudes against baseline (prior to injection).

Statistical analysis used the ANCOVA analysis of regression.

Representative baseline (pre-ICG) cSLO findings in the normal Sprague Dawley rat eye are shown (FIGs. 5A-H), and served along with PBS-treated animals (**FIG.** 6A), as normal controls for all experimentation. Red-free (i.e., green dominant) imaging identified the optic nerve head (ONH) and radial blood vessel of the retina (**FIG. 5A**). Low levels of endogenous signal were also seen at longer wavelengths, in the NIR channel (830 nm), and provided a similar image of the ONH and vasculature with some potential imaging of the deeper choroidal vessels that arises due to deeper light penetration (**FIG. 5B**). Consistent with human studies, FAF appeared as a faint ground-glass glow that is lacking in detail, and is obscured by the radial retinal blood vessels and is absent at the ONH (**FIG. 5C**). This diffuse glow however is very faint.

By contrast, with the NIR excitation/emission filters in place as for ICG angiography, no or negligible signal was observed in the eye prior to ICG injection (**FIG. 5D**). Scan lines were evident and the vasculature was barely detectable or not detectable. This was observed in over 60 eyes. Immediately following ICG injection, both the retinal and choroidal vessels (**FIG. 5F**, and **FIG. 5G** second panel) were identified during the transit phase and out to the experimental endpoint, 20 minutes later. Fluorescein angiography also confirmed the normal retinal vasculature (**FIG. 5E**). In wild-type SD animals, no leakage was seen from either vascular bed using either fluorophore.

Though baseline images prior to ICG angiography exhibited no signal under NIR stimulation in the presence of the ICG excitation/emission filters, images obtained at 3, 8, 21 and 28 days after a single injection of ICG at day 0 (t=0), demonstrated a delayed and persistent fluorescence (**FIG. 5G**). This was not observed in similar experiments performed following angiography with fluorescein-dextran dye and analyzed with the fluorescein angiography filters in place (**FIG. 5H**), where the same level of low pre-injection fluorescence is visible throughout the time course of investigation. It also did not occur in the absence of previous ICG injection (**FIG. 6A**). Qualitative analysis of this delayed NIR fluorescence after ICG showed a "mosaic" pattern of small speckles in the posterior pole that, viewed *en face,* are deep (external) to the retinal vessels and obscure the view to the choroidal vasculature, suggesting its location between the retina and the choroid. The ONH and circumpapillary area did not fluoresce. These features are similar to those of FAF performed in the blue spectrum in patients. It was determined that the same plane of focus is optimal for both fluorescent techniques.

The observations made using DNIRA after ICG injection were dose-dependent (**FIG. 6A-D**). The higher the initial ICG dosage used for angiography, the brighter the NIR signal. The high dose, 5 mg/kg, required that the gain (sensitivity) of the cSLO be reduced to obtain suitable quality images, while the low dose (0.35mg/kg) was less bright, and required that the gain be increased to obtain suitable quality images. Comparative images (**FIG. 6A, FIG. 6B** and **FIG. 6C**), taken with the gain set at the same level, demonstrated a dose-responsive increase in brightness. In the same experiment, it was also determined that by day 28 the speckled pattern faded in animals receiving low-dose ICG (**FIG. 6B**). By contrast, in animals receiving high dose ICG, there was little appreciable difference between days 3 and 28 post-injection (**FIG. 6C**).

NIR reflectance imaging at 830 nm, without 795/810 nm excitation/emission filters necessary for ICG activation, did not yield the speckled, dose-dependent and time-dependent fluorescence findings observed with DNIRA.

Having demonstrated that DNIRA identifies the RPE and/or outer retinal complex, it was determined that DNIRA could be used to identify structural abnormalities of the RPE. DNIRA was used in combination with systemic injection of the RPE toxin, NaIO₃. NaIO₃ is directly toxic to RPE cells and leads to their loss; apoptosis of the overlying photoreceptors occurs thereafter. DNIRA in the days and weeks after NaIO₃ injection identified geographic (spatial) patches of profound hypofluorescence, evident as large black patches within an otherwise continuous background of speckled fluorescence. These hypofluorescent patches were bounded by a defined border. Further, with the gain of the cSLO increased, brighter viewing through these patches permitted clear visualization of the choroidal detail (**FIGs. 7A-B**). Choroidal vessels were identified by their complexity, variable size, and non-radial pattern with respect to the ONH. By contrast, the speckled layer obscured this view but permitted ongoing visualization of the overlying retinal vessels. These data were consistent with the notion that DNIRA detects ICG-labeled RPE/outer retinal layer.

Further, the suitability of DNIRA for pre-clinical experimentation was demonstrated with toxicity studies. Electroretinography, comparing high and low dose systemic ICG concentrations against PBS, was undertaken. These data showed no statistically significant change in the b-wave amplitude in the three weeks following injection compared against changes noted in control animals, and demonstrate that DNIRA, over the dose range used, is safe in pre-clinical studies of chorioretinal disease. While the high dose is well above that used clinically, the low dose is consistent, in grams/body weight, with the dose used for clinical imaging with traditional fundus cameras.

### Example 3: Discovery of Compounds for the Treatment of Ocular Disorders

Having established an animal that models one or more ocular disorders, a test compound is administered to such an animal.

The presentation of the ocular disorder is determined using *in vivo* imaging including fluorescence detection. A candidate compound is identified by observing fluorescence in the eye at or after a time sufficient to present characteristics of an ocular disorder. The candidate compound is evaluated for its ability to reduce or eliminate the characteristics of the ocular disorder as described herein.

### Example 4: Assessing the Activity of Compounds for Ocular Disorder

Having developed an animal that models an ocular disorder, a candidate compound is administered to such an animal.

The presentation of the ocular disorder disease is determined using *in vivo* imaging including fluorescence detection. The activity of a candidate compound is assessed by observing fluorescence in the eye at or after a time sufficient to present characteristics of an ocular disorder disease and the activity of a candidate compound. The candidate compound may be assessed for its ability to reduce or eliminate the characteristics of the ocular disorder as described herein.

### Example 5: Treatment of Dry AMD with Bindarit

Human subjects, 56 to 100 years of age or more, present with dry AMD, as diagnosed by one or more of the following clinical tests: clinical examination, FAF (at any wavelength), near infrared and /or red-free photography, fluorescein angiography, which allows for the identification and localization of abnormal vascular processes; OCT, which provides high-resolution, cross-sectional or *en face* images from within optical scattering media, such as the human retina and choroid; and structured illumination light microscopy, using a specially designed high resolution microscope setup to resolve the fluorescent distribution of small autofluorescent structures (lipofuscin granulae) in retinal pigment epithelium tissue sections.

The subjects are administered bindarit in two 300 mg oral doses (or up to 1200 mg) once a day for 12 weeks. After an initial twelve-week treatment period, the subjects are evaluated for clinical outcomes. Alternatively, patients receive intravitreal injection of a vehicle containing bindarit, with or without a drug delivery vehicle.

A first clinical outcome is determined using a standard visual acuity test, as is well known in the art. The subjects are assessed for the ability to clearly see symbols and objects on a Snellen eye chart from a distance.

A second clinical outcome assesses the rate of progression of geographic atrophy. To do so, the subjects' pupils are dilated with 1.0% tropicamide and 2.5% phenylephrine before retinal imaging. Imaging is carried out with an instrument (e.g., Spectralis HRA+OCT; Heidelberg Engineering, Heidelberg, Germany) that allows for simultaneous recording of cSLO and spectral-domain optical coherence tomography (SD-OCT) with two independent scanning mirrors, as described in Helb, et al. Acta Ophthalmol. 2010 Dec; 88(8):842-9. Five modes of operation are employed: white light, red-free light, near infrared, FAF and OCT.

A third clinical outcome assesses the rate of change of hypofluorescent DNIRA (quantified by the area of hyporfluorescence). To do so, the subject's pupils are dilated with 1.0% and 2.5% phylephrine before retina imaging. Imaging is carried out with an instrument (that may utilize confocal scanning methodology, or a modified fundus camera with the appropriate excitation and emission filters in place, with or without the capacity to perform angiography) and occur prior to ICG injection (baseline), and again in the days after dye injection without further injection of dye but with the excitation/emission filters in place.

A fourth clinical outcome assesses the change in the number and location of hyperfluorescent DNIRA dots. To do so, the subject's pupils are dilated with 1.0% and 2.5% phylephrine before retina imaging. Imaging is carried out with an instrument (that may utilize confocal scanning methodology, or a modified fundus camera with the appropriate excitation and emission filters in place, with or without the capacity to perform angiography) and occur prior to ICG injection (baseline), and again in the days after dye injection without further injection of dye but with the excitation/emission filters in place.

cSLO images are obtained according to a standardized operation protocol that includes the acquisition of near-infrared reflectance (λ = 815 nm) and FAF (excitation at λ = 488 nm, emission 500-700 nm) images. Regions of hypofluorescenct (dark) FAF are measured and compared to similar measurements over time. The increase is size of the hypofluorescent FAF is a measure of the rate of progression of GA. Alternatively, areas of GA, and their change over time, can be calculated from *en face* reconstructions of SD-OCT imaging, carried out for example with an illumination wavelength of 870 nm, an acquisition speed of 40,000 A-scans, and a scan depth of 1.8 mm. Two SD-OCT scans, one vertical and one horizontal, per eye are performed through the approximate foveal center, or in the case of RPD, in proximity to the vascular arcades of the macula. Fluorescein angiography (λ = 488 nm, emission 500-700 nm, 10% fluorescein dye) is performed as needed. Color fundus photographs are obtained with a fundus camera (e.g. FF 450 Visupac ZK5; Carl Zeiss Meditec AG, Jena, Germany).

Interpretation of clinical outcome data informs a decision for further treatment, if any.

### Example 6: Treatment of Dry AMD with a Combination Therapy

Human subjects, 56 to 100 years of age or more, present with dry AMD, as diagnosed by one or more of the following clinical tests: clinical examination, white-light fundus imaging, FAF at any wavelength, near infrared and /or red-free photography, blue-light illumination, and/or fluorescein or ICG angiography, which allows for the identification and localization of abnormal vascular processes; OCT, which provides high-resolution, cross-sectional, three-dimensional and *en face* images from within optical scattering media, such as the human retina and choroid; and structured illumination light microscopy, using a specially designed high resolution microscope or ophthalmoscope set up to resolve the distribution of small autofluorescent structures (lipofuscin, lipofuscin-like, or other granulae) in retinal pigment epithelium or other cells and cell layers.

The subjects are administered bindarit, by way of non-limiting example, in two 300 mg oral doses (or up to 1200 mg) once a day for 12 weeks. The subjects are also administered ranibizumab injection once per month (roughly 28 days) in a dose of 0.5 mg per affected eye. After an initial twelve-week treatment period, the subjects are evaluated for clinical outcomes. Bindarit may also be administered via any of the ocular routes described herein (e.g. intravitreally).

Alternatively, subjects are administered bindarit, by way of non-limiting example, in two 300 mg oral doses (or up to 1200 mg) once a day for 12 weeks. The subjects are also administered lampalizumab injection once per 4-6 weeks in a dose of about 10 mg per affected eye. After an initial twelve-week treatment period, the subjects are evaluated for clinical outcomes. Bindarit may also be administered via any of the ocular routes described herein (e.g. intravitreally).

A first clinical outcome is determined using a standard visual acuity test, as is well known in the art. The subjects are assessed for the ability to clearly see symbols and objects on a Snellen eye chart from a distance.

A second clinical outcome assesses the rate of progression of geographic atrophy. To do so, the subjects' pupils are dilated with 1.0% tropicamide and 2.5% phenylephrine or a comparable agent before retinal imaging. Imaging is carried out with an instrument (e.g., Spectralis HRA+OCT; Heidelberg Engineering, Heidelberg, Germany) that allows for simultaneous recording of cSLO and SD-OCT, as described in Helb, et al. Acta Ophthalmol. 2010 Dec; 88(8):842-9. Multiple modes of operation can be employed: white light, red-free light, blue light, near infrared, and OCT. Similar analysis can be performed with a modified fundus camera.

A third clinical outcome assesses the rate of change of hypofluorescent DNIRA (quantified by the area of hyporfluorescence). To do so, the subject's pupils are dilated with 1.0% and 2.5% phylephrine before retina imaging. Imaging is carried out with an instrument (that may utilize confocal scanning methodology, or a modified fundus camera with the appropriate excitation and emission filters in place, with or without the capacity to perform angiography) and occur prior to ICG injection (baseline), and again in the days after dye injection without further injection of dye but with the excitation/emission filters in place.

A fourth clinical outcome assesses the change in the number and location of hyperfluorescent DNIRA dots. To do so, the subject's pupils are dilated with 1.0% and 2.5% phylephrine before retina imaging. Imaging is carried out with an instrument (that may utilize confocal scanning methodology, or a modified fundus camera with the appropriate excitation and emission filters in place, with or without the capacity to perform angiography) and occur prior to ICG injection (baseline), and again in the days after dye injection without further injection of dye but with the excitation/emission filters in place.

cSLO images are obtained according to protocols known in the art that may include the acquisition of near-infrared reflectance (λ = 800-1000 nm) and FAF (excitation at λ = 280-550 nm, emission 350-700 nm) images. Regions of hypofluorescenct (dark) FAF are measured and compared to similar measurements over time. The increase is size of the hypofluorescent FAF is a measure of the rate of progression of GA. Alternatively, areas of GA, and their change over time, can be calculated from *en face* reconstructions of SD-OCT imaging, carried out for example with an illumination wavelength of 870 nm, an acquisition speed of 40,000 A-scans, and a scan depth of 1.8 mm. Two SD-OCT scans, one vertical and one horizontal, per eye are performed through the approximate foveal center, or in the case of RPD, in proximity to the vascular arcades of the macula. Fluorescein angiography (λ = 488 nm, emission 500-700 nm, 10% fluorescein dye) is performed as needed. Color fundus photographs are obtained with a fundus camera (*e.g.* FF 450 Visupac ZK5; Carl Zeiss Meditec AG, Jena, Germany). DNIRA images are obtained according to protocols described here that may include the acquisition of baseline images the ICG excitation/emission filters in place but before injection of ICG dye. ICG dye, up to 5mg, is injected intravascularly, and angiography may or may not be performed. DNIRA images are again obtained with the ICG excitation/emission filterse in place but with no further injection of dye. Regions of hypofluorescent (dark) DNIRA are measured and compared to similar measurements over time.

Interpretation of clinical outcome data informs a decision for further treatment, if any. Illustrative data analysis includes macular cube analysis and 5 line raster.

### Example 7: Detection and/or Prediction of an Ocular Disorder Subject Response to an Agent

Using multi-modal imaging in the rat eye, it was determined that systemic injection of the RPE toxin, NaIO₃, induces complex patterns of FAF similar to those in patients with aggressive forms of dry AMD and/or RPD. Tissue histology and fluorescent microscopy illustrated that the *in vivo* patterns of FAF correspond with the spatial distribution of autofluorescent cells of the innate immune system recruited to areas of RPE damage or loss.

*Multi-modal and Angiographic Imaging In Vivo:* Fundus imaging was performed using a commercially available confocal scanning laser ophthalmoscope, (cSLO, Spectralis, Heidelberg Retinal Angiography, HRA-II; Heidelberg Engineering GmbH, Germany), and the Eye Explorer Image Capture system, version 1.1.10. Images were captured at a wavelength of 488 nm excitation with 500 nm barrier filter for fluorescein angiography without the barrier filter for red free (RF, green dominant) imaging, at 795/810 nm excitation/emission for ICG angiography, and with a 830 nm laser for infrared reflectance imaging. To evaluate 488 nm FAF, cSLO imaging was performed in the absence of fluorescein dye. In cases in which it was necessary to simultaneously image the vasculature and provide landmarks for FAF image analysis, ICG was injected via tail vein, at 2 mg/kg or 5 mg/kg, into a previously inserted 23 gauge tail vein catheter.

FAF imaging of normal Sprague Dawley rat eye produced a faint, homogenous ground glass glow interrupted by radially-arranged retinal blood vessels that block RPE fluorescence and therefore appear dark. Such imaging was also characterized by a central hypofluorescent circle at the optic nerve head (ONH), where RPE is absent. Systemic injections of NaIO₃ (45% solution, prepared fresh for each set of experiments in 0.9% sodium chloride and injected to a final concentration of 45 mg/kg body weight) were given to experimental rats. Three days after injection, a single area of iso- or slightly hyperfluorescent FAF with hyperfluorescent borders appeared and took the shape of a discrete island, sector, or 360° ring. In all cases, the islands, sectors or rings approached the ONH but were not contiguous with it. These and the following observations were confirmed in over 160 eyes.

Beginning approximately 4 to 5 days after NaIO₃ injection, a marked reticular or curvilinear pattern of alternating hyper/hypo-fluorescent FAF appeared within the islands, sectors, or rings. By day 7, this pattern was pronounced and it continued to emerge over weeks, whereby its curvilinear components contributed to partial loops, ovals, circles, rosettes, and scalloped or "paw-print" patterns of FAF. Even as late as 20 weeks, the last time-point analyzed, the borders retained their hyperfluorescent reticular pattern and continued to creep slightly toward the retinal periphery.

Fluorescein angiography performed in a subset of animals confirmed that the reticular pattern does not correspond with the retinal or choroidal vasculature while ICG angiography revealed a permeability change that occurred at the chorio-retinal interface, across the RPE and BM, approximately two to three days after NaIO₃ that coincides with, both spatially and temporally, the emergence of the islands, sectors of rings of FAF. This increase in permeability is transient and resolved by day 7, the next time point routinely evaluated. The retinal vessels do not leak. Hemotoxylin and eosin (H & E) histology of paraffin-embedded tissue and immunohistochemistry (IHC) (known in the art; performed after fixation with, *e.g*., Davidson's fixative and paraffin-embedded sections, viewed with a Nikon Upright E800 Microscope) confirmed that fluid accumulated in the subretinal space. This was also observed by ultra-high resolution OCT *in vivo* (high resolution 160 nm spectral domain OCT images were obtained using the Envisu R2200 VHR Animal SDOIS System, Bioptogen, USA; scans were acquired at a rate of 36,000 per second, with a lateral resolution of approximately 2.8 µm at the level of the retina).

*Autofluorescent and Immunofluorescent Analysis of Excised Retina and RPE:* To determine the pathological correlate of the FAF pattern observed *in vivo,* excised retinal whole mounts were viewed at low magnification under white light and by epifluorescent microscopy at 488 nm, the same wavelength used for *in vivo* imaging, with no fluorescent IHC labeling. Depending on subsequent tissue analysis, two microscopes were used for this purpose: an epifluorescent device with a narrow excitation/emission window (488/520 nm), and the Inverted Leica DM IRE2 viewing system of a confocal microscope with a mercury arc lamp and long-pass (rather than band-pass) filter providing fluorescence from greater than 515 nm following excitation with a 450-490 nm band. Under white light, the normal rat retina appeared fairly homogenous throughout, with some artifactuous deformation. By contrast, a subtle off-white, fine reticular pattern became apparent in samples obtained 7 days after NaIO₃ injection. By day 14, this pattern was more apparent and was observed to correspond with small, outer retinal folds best illustrated at the cut edge of the retina. Viewed under 488 nm fluorescent light, this same curvilinear pattern was apparent. Using higher magnification epifluorescent microscopy, it was demonstrated that this lacy, curvilinear pattern coincides with the spatial distribution of autofluorescent cells that appear to align with grooves or folds of the outer retina.

To determine the identity of the autofluorescent cells that coincide with the reticular pattern of FAF, excised retina were stained for with markers of the innate immune system, avoiding the 488 nm channel. Iba1 is a pan-microglial/macrophage marker and in the rat retina; antibodies against it identify the major phagocytic cell populations. Using this marker, it was found that Iba1⁺ cells are arranged in a lacy, curvilinear pattern when viewed *en face* in whole mount retina, i.e. in the z-plane. Further, when compared against photoreceptor nuclear staining, this curvilinear pattern is seen to lie interposed between folds of the deformed ONL. This pattern of Iba1⁺ cells corresponds with the pattern of hyperfluorescence observed *in vivo* by FAF imaging, and with the pattern of autofluorescent cells in excised retina. Corresponding patterns of *in vivo* FAF and Iba1⁺ staining were observed at both 7 days and 12 weeks after NaIO₃ injection. Importantly, at 7 days after NaIO₃, all RPE cells are absent from the posterior eye cup, confirming that these cells cannot be responsible for the observed FAF. Also, identification of the autofluorescent cells as phagocytic was confirmed by comparison of imaging to preparations in which monocyte/macrophage depletion has been undertaken. Such depletion was achieved by treatment with gadolinium chloride (GAD). Upon treatment with GAD, far less well defined reticular patterns of FAF were observed, including a reduction in demarcation of the borders.

Such identification of phagocytic cells is indicative of dry AMD and/or RPD and predictive of a subject's response to an agent. Specifically, the observation of such phagocytic cells makes it more likely than not that a subject will respond to treatment with an agent.

Identification of a transient increase in permeability across a subject's epithelial barrier between a choroid and a retina relative to an undiseased state is indicative of dry AMD and/or RPD and predictive of a subject's response to an agent. Specifically, the observation of a transient increase in permeability across a subject's epithelial barrier between a choroid and a retina relative to an undiseased state makes it more likely than not that a subject will respond to treatment with an agent.

*Delayed Near InfraRed Analysis (DNIRA) of Excised Retina and RPE:* Also, identification of abnormal features in comparison with the normal eye is undertaken with Delayed Near Infra Red Analysis (DNIRA). DNIRA images are obtained in the days and weeks after dye injection, such as ICG injection, using the ICG angiography settings, i.e. with excitation/emission filters, but without re-injection of dye at 24 hours, 48 hours, 3 days, 7 days, 21 days or 28 days after angiography. Angiography is not performed again during the time-course of the study.

For toxin administration, a toxin such as NaIO₃ (Sigma, cat #424064) is injected systemically via a 23G catheter at a dosage of 45 mg/kg body weight. ICG angiography at 0.35mg/kg is performed immediately prior to NaIO₃ injection. DNIRA is performed 7 days after ICG and NaIO₃ injection.

### Example 8: DNIRA of a Rat Eye After Systemic ICG Administration Labels Immune Cells In Vivo

To determine the pathological correlate of the FAF pattern observed *in vivo,* excised retinal wholemounts were viewed at low magnification under white light and by epifluorescent microscopy at 488 nm - the same wavelength used for *in vivo* imaging, with no fluorescent IHC labeling. Depending on subsequent tissue analysis, two microscopes were used for this purpose: an epifluorescent device with a narrow excitation/emission window (488/520 nm), and the Inverted Leica DM IRE2 viewing system of a confocal microscope with a mercury arc lamp and long-pass (rather than band-pass) filter providing fluorescence from greater than 515 nm following excitation with a 450-490 nm band. Under white light, the normal rat retina appeared fairly homogenous throughout, with some artifactuous deformation (**FIG. 3A**). By contrast, a subtle off-white, fine reticular pattern became clear in samples obtained 7 days after NaIO₃ injection. By day 14, this pattern was more clear and seen to correspond with small, outer retinal folds best illustrated at the cut edge of the retina (**FIG. 3B**). Viewed under 488 nm fluorescent light, this same curvilinear pattern was apparent. Though clear by day 7 after injection, increasing autofluorescence made this pattern more visible by Day 14 (**FIG. 3C**). Using higher magnification epifluorescent microscopy, we demonstrated for the first time that this lacy, curvilinear pattern coincides with the spatial distribution of autofluorescent cells that appear to align with grooves or folds of the outer retina (**FIG. 3D**).

Without wishing to be bound by theory, this suggests that the *in vivo* pattern of FAF observed corresponds with distribution of autofluorescent cells in the outer retina. This contrasts with the prevailing theories that ascribe clinically-relevant patterns of FAF to abnormal RPE, noting that RPE are absent in the isolated retinal samples we used. Without wishing to be bound by theory, this also suggests that the 2-dimensional pattern of FAF observed in this model corresponds with distribution of autofluorescent cells confined within 3-dimensional curvilinear folds of the outer retina.

Accordingly, the spatial and temporal changes of the outer retinal structure and the identity of the autofluorescent cell types were investigated.

Owing to the laminar nature of the neuroretina, we reasoned that outer retinal deformation could be readily evaluated by observing conformational changes of the normally flat outer nuclear layer (ONL), a layer of densely arrayed photoreceptor nuclei. Therefore, immediately after autofluorescent analysis of freshly excised wholemount retina, nuclear staining was performed and the tissue evaluated by epifluorescent or confocal microscopy in a non-488 channel. Cross-sections were also evaluated by H&E of whole eyes.

At baseline prior to NaIO₃ administration, confocal microscopy using the nuclear stain Topro3 confirms that the ONL was flat and devoid of vascular staining (**FIG. 4C**). Three days after NaIO₃ administration, slight curvilinear grooves of the ONL were noted, accompanied by occasional shallow, linear creases. By Day 7, ONL deformation was more pronounced, producing a distinctly interconnected series of grooves. By Day 14 these changes were more complex and of higher amplitude.

Cross-sectional H&E histology of retina at 90° to images obtained using confocal microscopy, showed that NaIO₃-induced RPE toxicity first leads to small undulations of the ONL that accompany increasing subretinal fluid, with little photoreceptor loss (**FIG. 4B**). Later, frank photoreceptor loss created higher amplitude folds in which the troughs of the ONL are juxtaposed against Bruch's membrane (BM), the basement membrane of the RPE. Eventually, lateral expansion of these troughs led to large contiguous regions of outer retinal degeneration such that much of the inner retina lies juxtaposed against BM.

To determine the identity of the autofluorescent cells that coincide with the reticular pattern of FAF, the excised retina with markers of the innate immune system was stained, avoiding the 488 nm channel. Iba1 is a pan-microglial/macrophage marker and in the rat retina; antibodies against it identify the major phagocytic cell populations. Using this marker, it was shown that Iba1⁺ cells are arranged in a lacy, curvilinear pattern when viewed *en face* in wholemount retina, namely, in the z-plane (**FIGs. 5A-H**). Further, when compared against photoreceptor nuclear staining, this curvilinear pattern was seen to lie interposed between folds of the deformed ONL. As shown in **FIGS. 5A-H****,** this pattern of Iba1⁺ cells corresponded with the pattern of hyperfluorescence observed *in vivo* by FAF imaging (**FIGs 1A-D** and **2A-D**), and with the pattern of autofluorescent cells in excised retina (**FIGs. 3A-C**). Corresponding patterns of *in vivo* FAF and Iba1⁺ staining are shown at both 7 days and 12 weeks after NaIO₃ injection (**FIGs. 5A** and **5B**). By 7 days after NaIO₃ administration, all RPE cells were absent from the posterior eye cup, confirming that these cells were not responsible for the observed FAF (**FIGs 5C** and **5D**).

The 3-dimensional (3D) relationship between the Iba1⁺ cells and the outer retina was next evaluated by *en face* serial confocal microscopy of wholemount retina (i.e., in the z-axis) moving step-wise from the inner plexiform layer (INL) through the outer plexiform layer (OPL), to the inner-, mid- and outer-ONL. Short stacks of confocal images (segments) were projected (flattened) at these three steps (**FIGS. 6A-D**). The co-distribution of activated CD68⁺ macrophages of the monocyte lineage was also evaluated.

At baseline prior to NaIO₃ administration, Iba1⁺ cells were seen in the IPL only. No CD68⁺ cells were identified (**FIG. 6A**). Three to four days after NaIO₃ administration, Iba1⁺ cells increased in number and were increased in the IPL and also throughout the outer retina, including the outer-ONL (**FIG. 6B**). Two weeks after NaIO₃ administration, a complex distribution of Iba1⁺ and CD68⁺ cells was seen interlaced between curvilinear folds of the ONL. Further, well-defined optically-sectioned ectopic circles or ovals of photoreceptor nuclei were observed as far internally as the OPL, the mid-retinal layer of blood vessels, thereby bringing the deformed photoreceptor nuclear layer and vascular layer, two normally separate tissue compartments, in contact. In the mid-ONL, ovoid cross-sections of the deformed nuclear layer appeared more curvilinear or pisciform, and large Iba1⁺ and CD68⁺ cells were found within their central void. In the outer-most third of the ONL, optical cross-sections remain curvilinear and were largely contiguous with many bridging segments. The subretinal space, normally a potential space only, was expanded by outer retinal deformation and shows Iba1⁺ and/or CD68⁺ inflammatory cells and photoreceptor nuclei together immediately internal to Bruch's membrane (in the space where the lost photoreceptor outer segments would normally reside).

Confocal scanning microscopy and three-dimensional reconstruction of the excised wholemount retina was undertaken (**FIG. 7A**). These data showed that Iba1⁺ cells formed a reticular or lacy pattern that is interposed between photoreceptor nuclei. Further, projection of the z-stack, divided into the same three planes (**FIG. 7B** right) showed that the inner-ONL is characterized by circular or oval cross-sections of the ONL. By contrast, the mid-ONL is characterized by looping curved cross-sections of the nuclear layer, and the outer ONL is formed of larger curvilinear segments with multiple bridging elements. Oblique volumetric projections of these layers readily illustrate the resulting pseudo-egg-crate configuration of the ONL, with several conical peaks extending from single contiguous folds at its base (**FIG. 7B** middle and right). Without wishing to be bound by theory, these data demonstrate that the majority of inflammatory cells are not located within or between ONL troughs, but rather are found under (i.e. external to) the ONL peaks in the expanded subretinal space.

Without wishing to be bound by theory, these observations show that the 3-dimensional distribution of autofluorescent inflammatory cells under the deformed outer retina accounts for the 2D pattern of *in vivo* FAF imaging in these animals.

To correlate these observations made in excised tissue to *in vivo* change and FAF findings, 3D volumetric analysis of the living retina using high-resolution OCT *in vivo* was performed. HR-OCT provides non-invasive, *in vivo* cross-sections of the retina previously observed only in post-enucleation histological specimens. OCT imaging is dependent on the signal generated at the interface between layers of differing optical density, and in the normal rat retina confirms its multi-laminar structure. The three major outermost OCT boundaries of the retina are, from internal to external, the external limiting membrane (ELM), the junction of photoreceptor inner and outer segments (IS/OS), and the RPE/BM (**FIG**. **8A**, left). Standard techniques both in the clinic and in these investigations arbitrarily set the nuclear layers, including the ONL, as dark (*i.e.* a signal void).

Following NaIO₃ administration, a reconstructed *en face* image of the living retina captured using OCT demonstrated a subtle change that is limited to the region inferior to the optic nerve (**FIG**. **8A****,** middle). Compared against baseline and the superior retina (which appears grossly normal three days after NaIO₃ administration) (**FIG**. **8A****,** middle), optical cross sections in the inferior retina showed slight deformation of the photoreceptor IS/OS junction and ELM such that bright (hyper-echoic) shallow mounds become visible (**FIG**. **8A****,** right). This signal was found external to the ONL and internal to BM. The fine dark line representing that the RPE was no longer present. By day 14, these mounds matured into pronounced peaks, a portion of which form bright, distinct triangles or pyramids whose bases appear to sit on BM **(****FIGs 8B** and **8C****).** Other mounds formed discrete narrow spikes that extended from the ELM, through the photoreceptor layer to reach the mid-retina. A clinical OCT image from a human patient with RPD shows a pyramidal subretinal deposit that is pathognomic of this disease (**FIG**. **8D****).**

To test if the bright mounds, triangles and spikes that characterize RPD and that seen in the present system, correspond with the distribution of autofluorescent phagocytic cells observed in the sub-retinal space volume-intensity projections (VIPs) were used. These were composed of OCT-generated optical sections to provide a means of evaluating 3-dimensional blocks of living tissue *in vivo* that can be reconstructed in the z- and y-axis.

Reconstruction of adjacent OCT images in the y-axis showed that the mounds or triangles, arrayed side-by-side, contribute to complex structures such as circles, ovals, halos and target-like lesions. In the example shown in **FIG. 9A****,** two subretinal mounds form the "sides" of a circle and lie close together at its superior and inferior aspect and maximally apart in the middle. Single mounds, pyramids and spikes contribute to simpler curvilinear structures.

To further support this finding, VIPs in the z-axis, (that is, in step-wise short segments from the inner-ONL to outer-ONL), were then analyzed (**FIG**. **9B****).** VIPs through the inner-ONL showed a series of punctate spots, regularly spaced throughout the retina. VIPs from the mid-ONL showed less regularly spaced pisciform or curved short lines, some of which are bridging. VIPs from the outer-ONL showed contiguous curvilinear segments with many bridging elements. These findings directly correspond with the 3D data generated from excised wholemount retina **(****FIGs. 6A-D****).**

Further, to confirm that hyperfluorescent FAF or abnormal patterns of FAF and hyperfluorescent DNIRA correlated or co-localized with the distribution of phagocytic cells, the circulating macrophage/monocyte population was depleted using gadolinium chloride (GAD or GdCl₃), a rare earth metal salt which depresses macrophage activity. GAD depletion of immune cells led to profound alteration of the NaIO₃-induced pattern of FAF and DNIRA. GAD depletion of the monocyte/macrophage populations led to profound alteration of the NaIO₃-induced pattern of FAF. This was evident qualitatively both within the geographic areas of damage, and at its border **(****FIGs. 8A-C****).**

Further, DNIRA, performed in a repeated manner, a method we term Pulsed DNIRA, identifies small hyperfluorescent dots in DNIRA that appear to migrate over time **(****FIG 32****).** RPE cells that also can be labeled with DNIRA, do not migrate. This along with the above co-localization with hyperfluorescent FAF suggests that macrophages colocalize and contribute to disease progression.

### Example 9: Clinical In Vivo Imaging of RPD and Diffuse Trickling AMD:

In **FIGs. 10A-C****,** images and interpretive diagrams that directly compare the clinical features of RPD against tissue findings in the NaIO₃ model are provided. In addition to clinical OCT imaging of a human patient with RPD (that shows a base-down pyramidal structure in the subretinal space, **FIG. 8D****),** color and red-free fundus images were also obtained **(****FIGs. 10A-C****).** Red free images showed small dark spots that constitute individual pseudodrusen of RPD (**FIG**. **10A****,** top image). These were identified using dark grey overlay. This further highlights, circumscribed by circles, the presence of so-called "target lesions" that characterize RPD. These appear as dark spots with lighter centers. In parallel, a schematized illustration of folded outer rat retina is presented, both *en face* and in cross-section that, when falsely colored and presented as black and white images-both "positive" and "negative"-correlates directly with targets and halos of RPD.

These data indicate that the 2D patterns of dark pseudodrusen within a lacy or reticular pattern of circles, halos and target lesion with dark centers viewed *en face,* in 2D, correspond with elevated rings and craters, considered volumetrically, in 3D.

### Example 10: Therapeutic Effects of Bindarit

This example shows, *inter alia,* that bindarit protects the retina and RPE against toxin-induced damage in a model of non-exudative "dry" AMD and RPD and that bindarit protects against the development of patches of retinal pigment epithelium (RPE) and photoreceptor loss known as Geographic Atrophy (GA).

Animals were handled in accordance with the Association for Research in Vision and Ophthalmology (ARVO) guidelines for the humane use of animals in ophthalmic research, and according to the Canadian Council on Animal Care guidelines. Animals were anesthetized with a combination of ketamine (100 mg/kg) and xylazine (10 mg/kg), and pupils dilated with a single drop of 0.8% tropicamide in 5% phenylephrine hydrochloride solution (Diophenyl-T, Sandoz Canada Inc). GenTeal lubricating eye drops (Novartis, Canada), were repeatedly applied to the corneal surface during all procedures.

*In vivo* images were acquired using a commercially available confocal scanning laser ophthalmoscope (cSLO) (Heidelberg Retinal Angiography, HRA-2, Heidelberg Engineering, Germany). Images were obtained in the red-free, FAF (488/500 nm excitation/emission), IR reflectance channel (830 nm) and ICG fluorescence channel (795/810 nm excitation/emission). Animals were pre-screened using cSLO, and those with spontaneous defects eliminated from the study. The surface of the cornea was kept moist with repeated application of Genteal lubricating drops (Alcon, Mississauga, Canada) during all *in vivo* imaging. Multiple images were taken of each eye starting at the optic nerve head (ONH), and moving in concentric rings to the mid- and far-periphery where possible. In some cases, due to difficulties in aligning the cSLO with the small pupil, contiguous images were obtained in a hemi-retina only, with re-alignment of the animal for subsequent imaging of the second half. Composite images of the fundus were assembled post-hoc in PowerPoint and exported for analysis.

Angiography: ICG dye (Cardiogreen, Sigma) was freshly prepared prior to experimentation to a final stock concentration of 5.0 mg/ml in sterile water. A 24-gauge catheter was inserted into the tail vein, and ICG dye infused at doses of 0.35, or 5.0 mg/kg. Images were taken prior to injection (baseline), during dye circulation, and at various intervals thereafter out to 20 minutes.

Delayed Near Infra Red Analysis (DNIRA): DNIRA images were obtained in the days and weeks after ICG injection using the ICG angiography settings, with excitation/emission filters in place, but without re-injecting the dye after day 0. Images were taken 2 - 120 days after angiography.

Quantification of FAF images: Composite images were grouped and imported to Image J (National Institutes of Health) for analysis by a masked observer. Comparison between the size of the patch of damage, determined from different images at different dosages of toxin, was achieved by normalizing the image size using the central (optic nerve head) cSLO image, as reference. An assumption was made that the magnification between fundus images did not vary significantly between rats. Areas with abnormal FAF signal were manually outlined by a masked reader using the "polygon" tracing tool, and the regions of interest measured in pixels. For dose-response analysis, the size of the patches was compared against the average of patches from 45 mg/kg NaIO₃ (assigned a value of 100 units).

Electroretinography (ERG): The bright-flash ERG response was evaluated in animals using the Espion (DiagnosysLLC, USA) mini-Ganzfeld system following high (5.0 mg/kg) and low (0.35 mg/kg) dose administration of ICG, or saline control. Following anesthesia, animals were placed on an electrically silent heating pad and gold coil electrodes placed at the edge of the cornea after application of GenTeal lubricating drops. Following a short train of dim flashes (0.01 candela s/m2, 1.0 Hz), the photopic b-wave response was evaluated using a single bright flash (3 candela s/m2) that we previously determined consistently approximates the maximum b-wave amplitude.

Sodium iodate: The RPE toxin sodium iodate (NaIO₃, Sigma) was prepared fresh weekly at a stock solution of 45 mg/ml in saline (Baxter, Mississauga, Canada). This solution was diluted to final concentrations of 5 mg/ml, 15 mg/ml, 30 mg/ml and 45 mg/ml, such that all animals received the same volume to achieve final dosages of 5 mg/kg to 45 mg/kg. NaIO₃ was injected systemically via a 24-gauge catheter inserted into the tail vein. ICG injection (0.35 mg/kg) and angiography were performed at day 0 immediately prior to NaIO₃ injection. To test the effects of bindarit, a standardized dose was used.

Intra-ocular injection of bindarit: intra-vitreal injection of bindarit was performed under a dissecting microscope (SMZ800; Nikon Instruments, Melville, USA) with a Hamilton syringe (Cat# 7634-01, Hamilton Company, Reno, USA). A small incision was made behind the limbus using a beveled 33-gauge needle (BD, Mississauga, Canada). A 30-gauge blunt needle was then inserted and at an angle of approximately 45° to the pupillary axis. Two (2) µL of bindarit solution or control solution (saline) was manually injected.

Autofluorescent fluorescence microscopy: Freshly excised wholemount ocular sample, either retinal or posterior cup devoid of retina, were evaluated using an epifluorescent microscope with wavelength of 488nm/512 nm.

Immunofluorescence microscopy: Immunohistochemistry was performed using standard protocols. Briefly, cells were fixed with 4% PFA/PBS for 15 minutes, and blocked with 1.25% BSA in TBS for 30 minutes at room temperature. Primary rabbit anti-ZO-1 was used at 2.5 µg/ml in 1.25% BSA/TBS and incubated for 1 hour at room temperature. Cells were washed in TBS and incubated with goat anti-rabbit 488 (Invitrogen) at 5 µg/ml for 1 hour at room temperature, washed, and counterstained with To-Pro-3 (Invitrogen). Some RPE monolayers were stained with 647 nm conjugated phalloidin (Invitrogen) for 20 minutes at room temperature and counterstained with Sytox green nuclear stain (Invitrogen). Slides were mounted with fluorescent mounting medium (Dako) and images acquired using a Leica TCS SL confocal fluorescent microscope (Leica Microsystems, GmbH, Wetzlar, Germany), with Leica Confocal Software V 2.61.

Treatment with bindarit provides secondary prevention against the late blinding complication of dry AMD and RPD known as Geographic Atrophy (GA). The purpose of secondary prevention is to maintain central visual acuity and central visual field prior to loss.

Patients with AMD start "dry" and have small deposits in the macula known as drusen. Late in disease, patients convert to advanced disease which is characterized by the patchy loss of the outer retina and RPE. Here it is shown in the present animal model that bindarit can prevent or slow the progression to late disease, i.e. secondary prevention of GA. **FIGs. 16A-F** show that bindarit (TMi-018) reduces the size of the patch of RPE loss in the rat eye. **FIGs. 16A-F** further show that, when evaluated over time using Fundus Autofluorescence (FAF), intra-vitreal bindarit reduces the development of complex patterns of hyperfluorescent FAF. **FIGs. 17A-B** shows that bindarit dose-dependently protects retinal function (electrophysiology), and demonstrates a minimally-effective dose. **FIG. 18A-C** shows that bindarit protects against tissue damage, RPE loss and disruption of the outer retina.

**FIGs. 19A-J** shows that bindarit reduces Monocyte Chemoattractant protein (MCP)-1 expression and reduces the M1 macrophage response, tipping the balance in favor of M2. Specifically, this data shows, among others, a dose-dependent reduction in MCP-1 protein expression in the subretinal space following intra-vitreal bindarit (*see, e.g.,* **FIG. 19D****).** Also, this data shows that during toxin-induced damage to the RPE, a model for ocular disease (e.g. dry AMD and/or RPD) gene expression shifts towards M1 polarization. Also, gene expression shifts back towards neutral, with relative decreases in M1 mRNA expression and increased levels of M2 expression. Without wishing to be bound by theory, these data suggest that bindarit differentially acts to reduce "inflammation-associated" transcription.

Not only can bindarit provide protection against patch development but also it can reduce the growth (expansion) of pre-existent patches of GA. Treatment with bindarit treats pre-pre-existent Geographic Atrophy (GA), a late blinding complication of dry AMD, by reducing the growth of these patches of RPE and outer retinal loss. A goal of such treatment is to reduce the size of the "blind spot" that the patient observes in their central field of vision. **FIG. 20A** shows a negative control (saline) treatment of an existing patch, which expands, while, in contrast, **FIG. 20B** shows bindarit treatment of an existing patch, which does not expand.

### Example 11: Bindarit's RPE Protection and Complement Modulation

MCP-1 is reported to be expressed by RPE, and is a potent Danger Associate Molecular Protein (DAMP) that activates M1 macrophages. This example, among others, investigated if bindarit could directly influence MCP-1 expression in RPE cells independent of its activity on macrophages. Using the immortalized, telomerase negative human RPE cell line (hTERT-RPE1) *in vitro,* it is shown that bindarit directly influences these cells, down-regulating MCP-1 mRNA and the M1 transcriptome (**FIG**. **21****).** Immunocytochemistry demonstrates downregulation of MCP-1 and KFκB protein (**FIG**. **21****).** Further, when challenged with NaIO₃, bindarit protects hTERT-RPE cell viability, suggesting, without wishing to be bound by theory, a second and unexpected mechanism of action highly relevant to its potential efficacy in the treatment of dry AMD (**FIG**. **21****).** Bindarit appears to have the potential to modulate the innate immune response both directly through its influence on macrophage polarization and indirectly by reducing the level of RPE signaling that initiates the local response.

**FIG. 21** shows, in panels A and B, that hTERT cells are confirmed to express markers of RPE differentiation. In panel C, it is shown that TMi-018 reduces NFkb expression and reduces MCP-1 in human RPE (hTERT). Panel D shows that TMi-018 increases RPE survival against toxin challenge.

Further, bindarit is shown herein to be an excellent combination agent. **FIG. 22** shows mRNA species of the complement cascade components change following toxin-induced RPE damage and that bindarit alters complement gene expression. The genes studied include: C1qb, C1qtnf5, C2, C3, C4b, C5, C6, C7, C8a, C9, Cfb, Cfd, Cfh, and Cfi.

Age related macular degeneration is associated with deficiencies in innate immunity, and promising therapies target these pathways. The polymorphism that confers greatest risk for the development of AMD is complement factor H (CFH), a component of the alternate complement cascade. Cfh is a negative-regulator of complement and its absence leads to an accelerated response. Downstream members include C5 and C3, both of which have been targeted unsuccessfully in clinical trials. However, a recent promising phase II study using the complement factor D (CFD) inhibitor, Lampilizumab, suggests that CFD inhibition can slow the growth, i.e. expansion, of geographic atrophy over an 18 month period. This effect was most robust in patients with concurrent complement factors H and I polymorphisms. Here it is shown that changes occur in the complement response over time following NaIO₃ administration. Normalized against baseline (pre-toxin), mRNA levels of many complement genes are seen to increase or decrease by more than 2 or 3 fold **(****FIG. 22****).** Major changes are identified in C1, C3, C5 and C9, and of note, CFH and CFI both decrease. This latter finding may correlate with the finding that the CFD inhibitor, Lampilizumab, was most efficacious in patients with polymorphisms in these genes. CFD mRNA did not change much, less than 3-fold, over much of the time-course.

Next, the effect of bindarit on these genes was explored. Bindarit dose-dependently up- or downregulated the mRNA of multiple complement genes.

Accordingly, bindarit profoundly alters the mRNA response, and works synergistically with modulators of the complement system.

### Example 12: Clinical Evaluation of Drug Efficacy and/or Disease Progression with DNIRA

This example shows that, among others, DNIRA is useful as a diagnostic or prognostic method for ocular disorders. For example, the present data show that DNIRA is useful as a biomarker for the therapeutic efficacy of an agent against one or more ocular disorders and/or a diagnostic for the onset of one or more ocular disorders and/or a diagnostic for progression of one or more ocular disorders. Importantly, the data present findings in human subjects afflicted with ocular disorders.

DNIRA is a novel and previously undescribed biomarker for diagnosis, disease progression, and treatment response. Its application, qualitative description and quantification provides important uses to these ends.

At present, the instruments used to perform ICG angiography require real-time imaging of the dye as it passes through, for example, the ocular (retinal and choroidal) blood vessels. As such, imaging typically starts in the seconds and minutes prior to injection of the dye in the veins of the arm or hand, and continues for typically 10 to 30 minutes thereafter. Late phase angiography typically occurs up to 45 minutes later. Images are thus acquired in the transit phase (as dye passes through the blood vessels) and in the re-circulation phase (as it leaks from the vessels into the tissue). The devices required to image ICG dye thus must capture sequential images originally in the form of film, video, or more recently digital angiography. These systems are expensive and require sustained imaging. Instead, the present example provides use of ICG excitation/emission filters without the use of angiographic imaging. Baseline DNIRA images are obtained with the ICG ex/em filters in place prior to systemic injection of ICG dye. In the days and weeks after injection, individual images are obtained. The use of ICG filters without angiography has not been previously described.

The application of DNIRA to clinical disease is illustrated herein. Briefly, all procedures were approved by the St Michael's Hospital Research Ethics Board and are compliant with Good Clinical Practice (GCP) and the Declaration of Helsinki for the clinical investigation of human subjects. Following full explanation of the nature and possible consequence of the procedures, and with informed consent, patients with a clinical diagnosis of dry AMD were serially recruited to the study. Baseline investigation included spectacle-corrected visual acuity, ophthalmic examination with slit-lamp microscopy and fundoscopy, along with cSLO imaging that included FAF and NIR imaging with the ICG excitation/emission filters in place but no infusion of dye. Angiography was performed less than one week thereafter, simultaneous with administration of a single dose of ICG dye (Cardiogreen, 25mg/5ml) and fluorescein sodium. FAF and DNIRA imaging was performed twice in the following 7 days, with a minimum of 2 and maximum of 4 days between procedures.

DNIRA is compared against ICG angiography. Other than overlying blockage of the central signal (barely detectable) little abnormality is observed with ICG angiography (**FIG**. **23****).** This is in stark contrast to DNIRA, in which areas of marked hyperfluorescence in the central macula (**FIG**. **24****).** In addition, a leopard-like pattern is observed in the peripheral macula that has not been identified by any imaging technique to date. Further, DNIRA is compared to FAF and infra-red (IR) as is also superior against these techniques (**FIG**. **25****).** DNIRA images of the same patient as shown in the left (IR) and center (FAF) panels of **FIG. 25** contrast markedly. For instance, only DNIRA reveals a region of profound hypofluorescence (dark) in the image's center. Smaller patches are noted at the margin of central lesion and in proximity to the optic nerve head. Further, composite images of the same patient shows a lacy, reticular or leopard-like pattern of alternating hyper- and hypo-fluorescent DNIRA that is not seen in other imaging modalities, and not described previously (**FIG**. **26****).** By comparison, an age-matched non-AMD patient, who is not known to have RPE damage, does not show such lacy, reticular or leopard-like pattern of alternating hyper- and hypo-fluorescent DNIRA (data not shown).

By analogy with FAF, areas of chorioretinal damage are seen as hypofluorescent (dark, black or absent) signal. Such areas are readily quantified owing to the stark contrast between normal or hyper-fluorescent background. Algorithms for the quantification of hypofluorescent FAF are now included with commercially available imaging devices (for example the confocal scanning laser ophthalmoscopy system, or modified fundus camera). Quantification of hypofluorescent FAF is now incorporated into clinical trial design as both inclusion criteria and outcome measure. For example, the Mahalo study (Genetech) requires that patients have a minimum and maximum size region of hypofluorescent FAF to enter the study. The size of this area is measured repeatedly during the study and the rates of growth, *i.e*. expansion, is compared between treatment arms. This example shows that DNIRA makes clear regions of hypofluorescence not seen in current imaging modalities. Accordingly, *inter alia,* DNIRA can be used to improve identification of suitable patients for study recruitment and to evaluate progression of disease.

Further, by analyzing single patients over time, it is shown here that DNIRA images change over time. This enables serial quantification of the areas of hypofluorescent or hyperfluorescent signal and provides a strategy for analysis of disease progression (*e.g*. as a surrogate biomarker). For example, the DNIRA images of FIG. 27 show hypofluroescence expansion in a late dry AMD patient.

In addition to hypofluorescent DNIRA, described herein is the presence of hyperfluorescent DNIRA in the clinical setting. Bright punctate dots or spots can be identified in the rodent eye in the days and weeks after ICG injection without further injection of dye, but only with the ICG excitation/emission filters in place (*see, e.g.* Examples herein, such as Example 1, 2, and 8). In animal eyes, Pulsed DNIRA (in which ICG dye is provided repeatedly intravascularly and evaluated intra-ocularly in the living eye in the days after injection) and ImmunoD, it is shown that macrophages can be labeled *in vitro* and *in vivo* with ICG and so become visible as small, motile, hyperfluorescent dots. **FIG. 28** shows that discrete dots of hyperfluorescent DNIRA are identified scattered throughout the macula of patients with AMD. Some are in proximity to the optic nerve head, and other immediately adjacent to regions of geographic atrophy, both at the border and in the so-called junctional zone.

In addition to the application of DNIRA to patients with AMD, the present Example also shows the utility of DNIRA for human patients with RPD (*e.g.* as a diagnostic and/or prognostic tool). Classic drusen and pseudodrusen are associated with subsequent geographic atrophy and/or choroidal neovascularization. **FIG.** 29 shows the eye of a human patient with RPD. Having characterized the patient as having RPD using IR and FAF imaging, DNIRA imaging demonstrates multiple regions of hypofluorescent FAF not seen in any other modality (including other modalities not shown in **FIG. 29****).** These regions are distributed throughout the macula. The leopard-like spots are again seen in the macular periphery. RPD have been shown to mature over time, starting as shallow mounds anterior to the RPE, and later maturing into base-down pyramids or spike superiorly. In several studies, RPD are seen to mature in a centripetal direction, being newer or younger peripherally (in this case superiorly), and older centrally (inferiorly). Consistent with the suggestion DNIRA, the hyperfluorescent dots seen superiorly would coincide with the presence of labeled macrophages that have infiltrated the tissue. In central regions where macrophages have potentially resided in the tissue longer, areas of tissue loss are suggested by the small patches of hypofluorescent DNIRA. **FIG. 30** shows a gradient of hypofluorescent and hyperfluorescent signal in a different RPD patient than above. The lower arrow shows region of discrete hypofluorescent FAF immediately superior to the central macula. Superior to this, there is an increasing accumulation of hyperfluorescent FAF dots

To better understand the source of the DNIRA signal, and to accommodate for the large differences in magnification between rodent and human imaging using the clinical-grade cSLO system, intermediate size rabbit eyes were used to study the source of the punctate hyperfluorescent DNIRA signal.

An RPE toxin was given systemically to New Zealand white rabbits, and DNIRA performed as previously described. Images were taken serially in the days and weeks after ICG injection, without further injection of dye and with the excitation/emission filters in place. Active disease in humans is characterized by hyperfluorescent FAF, but the etiology remains partially unknown. It is understood that abnormal or damaged RPE cells will hyperfluoresce under blue fluorescent light owing to the accumulation of lipofuscin. However, we previously suggested, without wishing to be bound by theory, that macrophages also hyperfluoresce, potentially owing to the phagocytosis of damaged fluorescent RPE cells or to endogenous production of a lipofuscin-like material (as has been described *in vitro).* It follows that if macrophages hyperfluoresce in FAF, and accumulate ICG dye in DNIRA, they should co-localize in the two channels. This is observed.

**FIG. 31** shows a correspondence between hyperfluorescent FAF signal and hyperfluorescent DNIRA signal in the rabbit eye. To further show that DNIRA labels macrophages, the migration of hyperfluorescent DNIRA dots over time was evaluated. Following systemic toxin administration, a wave of bright dots is observed to migrate, or be labeled centrifugally, from the rabbit optic nerve head. **FIG. 32** shows that hyperfluorescent DNIRA dots, likely macrophages, change position over time. The upper row of images in **FIG. 32** shows DNIRA imaging after systemic toxin injection in the rat and shows punctate hyperfluorescent dots encircling the optic nerve head. Over time, these dots appear to migrate centrifugally. The lower row of images in **FIG. 32** shows DNIRA imaging in the rabbit eye and shows a similar apparent migration of individually labeled dots that by immunohistochemistry stain positively for macrophage markers. In both rows, the left-most image is baseline DNIRA (*i.e*., pre-ICG injection with ICG filters in place). Finally, having identified hyperflourescent dots in the rat eye and the rabbit in the region around the optic nerve head, the human optic nerve head was evaluated for similar findings. Similarities between rat, rabbit and human DNIRA permit clinical-pathological correlation and suggest that macrophages account for hyperfluorescence in all three species. **FIG. 33** shows that brightly labeled small DNIRA-positive dots are observed in proximity to the optic nerve head in rat, rabbit and human images.

### Example 13: Bindarit Treatment of Diabetic Retinopathy

Inflammation is suggested to play a central role in diabetic eye disease, including both diabetic retinopathy and diabetic macular edema. Cytokines, chemokines and other pro-inflammatory or pro-permeability molecules are described in human ocular tissues such as vitreous or aqueous, and also in serum. Animal models confirm an inflammatory response in pre-clinical studies.

Amongst the 50+ chemokines identified to date, it is reported that approximately 50% are altered in diabetes. Amongst these are MCP-1/ccl2 and RANTES. Transcription factors such as NFKb are also implicated in diabetic eye disease, and can be directly activated in the face of obesity and excess free fatty acids. Increased IKKb phosphorylation leads to NFkb translocation to the nucleus and activation of the inflammatory transcriptome. Further, serum levels of MCP-1/ccl2 and CRP are elevated in patients with severe diabetic retinopathy, potentially arising from systemic release of eye-derived mediators or, concurrent advanced systemic disease. Agents that can reduce these systemic factors, either locally or systemically might therefore be efficacious in the treatment of diabetic eye disease.

The present example shows, among others, that bindarit reduces inflammatory mediators demonstrated to be involved in diabetic eye disease. Sodium iodate, at a high dose of 45 mg/kg was used to induce an inflammatory response that was treated with high (20 µg/µl, 2 µl volume) and low (2 µg/µl, 2 µl volume) dose intra-vitreal bindarit. The effect of bindarit was assessed at day 16 after toxin injection. As shown in **Fig. 34****,** bindarit reduces inflammatory mediators known to be involved in diabetic retinopathy.

**FIG. 36** shows that bindarit had no significant effect on other interleukins IL-10 and IL-3 or tumor necrosis factor alpha (TNFa) and ccl5 (MCP-3). Fold mRNA changes were compared against baseline (normal) conditions, under the following conditions: 1. Intra-vitreal saline (vehicle), 2. High-dose bindarit, 2ug/ul, (no toxin), 3. Toxin + low dose 2ug/ul bindarit, 4. Toxin + high dose 20ug/ul bindarit + NaIO₃, and 5. Toxin alone.

Further, bindarit had no significant effect on vascular endotheiial growth factor A (VEGF-A) mRNA (**FIG**. **37****).** However, in this paradigm, VEGF-A was not elevated by toxin treatment (right) at any time after injection. All changes are approximately 1.0 fold up or down. Fold mRNA changes were compared against baseline (normal) conditions, under the following conditions: 1. Intra-vitreal saline (vehicle), 2. High-dose bindarit, 2ug/ul, (no toxin), 3. Toxin + low dose 2ug/ul bindarit, 4. Toxin + high dose 20 ug/ul bindarit + NaIO₃, and 5. Toxin alone.

**FIG. 38** shows that, in an immortalized human RPE cell line, bindarit reduces NFkb translocation to the nucleus.

Lastly, the effects of hyperglycemia on isolated human RPE cells were addressed. In the presence of hyperglycemia (*i.e*., elevated serum glucose) and in the absence of insulin, most cells become hypoglycemic. This is also true for cells that are non-responsive or resistant to insulin. However, cells of the brain and retina uptake high levels of glucose in the absence of insulin and so become hyperglycemic. At low levels this can stimulate cell activity, however at higher levels of glucose become toxic, inciting inflammation and leading to the formation of advanced glycated endproducts (AGEs). The immortalized human cell line, hTERT, was incubated with normal, medium-high and very high concentrations of D-glucose. Cell activity, measured in fluorescence unit was normalized against standard concentrations of glucose; L-glucose served as osmotic control. Cells were incubated for five days, with or without bindarit. **FIG. 39** shows that bindarit normalizes cellular stress in hTERT cells incubated with high glucose. This example, inter alia, points to bindarit as an agent for the treatment of diabetic retinopathy.

## Claims

1. A composition for use in treating a diabetic eye disease, comprising an effective amount of a compound of Formula I: 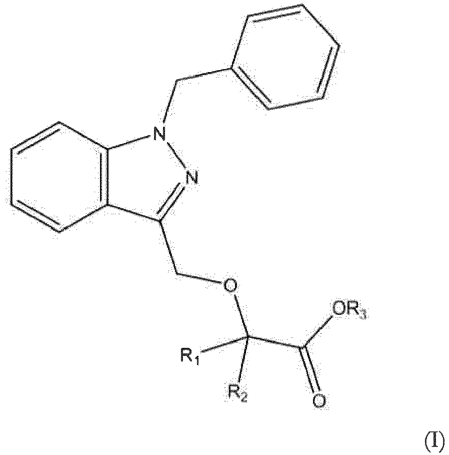 or a pharmaceutically acceptable salt thereof, wherein:
each of R1 and R2 is independently H or a C1-C6 alkyl and R3 is H or a C1-C6 alkyl, and
the compound is to be ophthalmically administered in an effective amount to a subject in need thereof.

2. The composition for use of claim 1, wherein the compound of Formula I is bindarit.

3. The composition for use of claim 1, wherein the diabetic eye disease is diabetic retinopathy or diabetic macular edema (DME).

4. The composition for use of claim 1, wherein the diabetic eye disease is a cataract or glaucoma.

5. The composition for use of claim 1, wherein the subject has type 1 or type 2 diabetes.

6. The composition for use of claim 5, wherein the subject has a blood glucose level that exceeds normal levels.

7. The composition for use of claim 1, wherein the use is to reduce NFxb translocation to the nucleus in eye cells of the subject.

8. The composition for use of claim 1, wherein the use is to normalize cellular stress in hyperglycemic cells.

9. The composition for use of claim 1, wherein the use is to reduce the subject's expression of one or more of Ccl2, Icam1, and Vcam1.

10. The composition for use of claim 1, wherein the use is to further comprise administering one or more additional therapeutic agents.

11. The composition for use of claim 10, wherein the one or more additional therapeutic agents are selected from an anti-VEGF agent, an ACE inhibitor, a PPAR-gamma agonist or partial agonist, a renin inhibitor, a steroid, and an agent that modulates autophagy, a semapimod, a MIF inhibitor, a CCR2 inhibitor, CKR-2B, a 2-thioimidazole, CAS 445479-97-0, CCX140, clodronate, a clodonate-liposome preparation and gadolinium chloride.

12. The composition for use of claim 1, wherein the ophthalmic administration is intravitreal or intraocular administration.

13. The composition for use of claim 12, wherein the compound is formulated for controlled-release or sustained release.

14. The composition for use of claim 12, wherein the compound is to be administered intravitreally.

## Patentansprüche

1. Eine Zusammensetzung zur Verwendung bei der Behandlung einer diabetischen Augenkrankheit, umfassend eine effektive Menge einer Verbindung von Formel I: 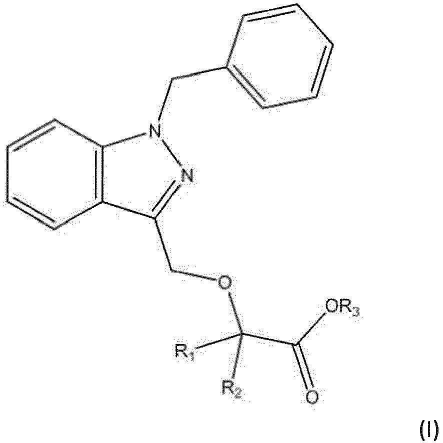 oder ein pharmazeutisch annehmbares Salz davon, wobei:
jedes von R1 und R2 unabhängig H oder ein C1-C6 Alkyl und R3 H oder ein C1-C6 Alkyl ist,
und
die Verbindung ophthalmisch in einer effektiven Menge an ein Subjekt, das daran Bedarf hat, verabreicht werden soll.

2. Die Zusammensetzung zur Verwendung nach Anspruch 1, wobei die Verbindung von Formel I Bindarit ist.

3. Die Zusammensetzung zur Verwendung nach Anspruch 1, wobei die diabetische Augenkrankheit diabetische Retinopathie oder diabetisches Makulaödem (DME) ist.

4. Die Zusammensetzung zur Verwendung nach Anspruch 1, wobei die diabetische Augenerkrankung Katarakt oder Glaukom ist.

5. Die Zusammensetzung zur Verwendung nach Anspruch 1, wobei das Subjekt Typ 1 oder Typ 2 Diabetes hat.

6. Die Zusammensetzung zur Verwendung nach Anspruch 5, wobei das Subjekt ein Blut-Glukose-Level hat, das über das normale Level hinausgeht.

7. Die Zusammensetzung zur Verwendung nach Anspruch 1, wobei die Verwendung zur Reduktion der NFκb Translokation zum Nukleus in den Zellen des Auges des Subjekts ist.

8. Die Zusammensetzung zur Verwendung nach Anspruch 1, wobei die Verwendung zur Normalisierung des zellulären Stresses in hyperglykämischen Zellen ist.

9. Die Zusammensetzung zur Verwendung nach Anspruch 1, wobei die Verwendung zur Reduktion der Expression des Subjekts von einem oder mehreren von Ccl2, Icam1 und Vcam1 ist.

10. Die Zusammensetzung zur Verwendung nach Anspruch 1, wobei die Verwendung ist, um ferner die Verabreichung eines oder mehrerer zusätzlicher therapeutischer Agenzien zu umfassen.

11. Die Zusammensetzung zur Verwendung nach Anspruch 10, wobei das eine oder die mehreren zusätzlichen therapeutischen Agenzien ausgewählt sind aus einem anti-VEGF Agens, einem ACE Inhibitor, einem PPAR-gamma Agonisten oder partiellen Agonisten, einem Renin Inhibitor, einem Steroid, und einem Agens, das Autophagie moduliert, einem Semapimod, einem MIF Inhibitor, einem CCR2 Inhibitor, CKR-2B, einem 2-Thioimidazol, CAS 445479-97-0, CCX140, Clodronat, einer Clodronat-Liposomen-Präparation und Gadoliniumchlorid.

12. Die Zusammensetzung zur Verwendung nach Anspruch 1, wobei die opthalmische Verabreichung intravitreale oder intraokulare Verabreichung ist.

13. Die Zusammensetzung zur Verwendung nach Anspruch 12, wobei die Verbindung für die kontrollierte Freisetzung oder dauerhafte Freisetzung ist.

14. Die Zusammensetzung zur Verwendung nach Anspruch 12, wobei die Verbindung intravitreal verabreicht werden soll.

## Revendications

1. Composition pour son utilisation dans le traitement d'une maladie oculaire diabétique, comprenant une quantité efficace d'un composé de formule I : 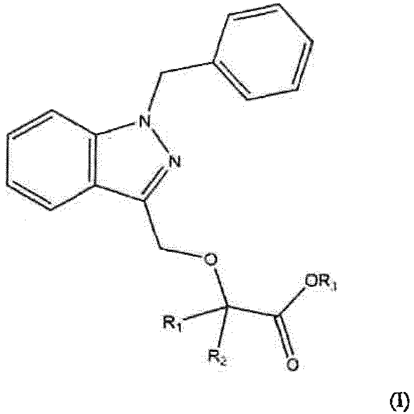 ou d'un sel pharmaceutiquement acceptable de celui-ci, dans lequel :
chacun de R1 et R2 est indépendamment H ou un alkyle en C1-C6 et R3 est H ou un alkyle en C1-C6, et
le composé doit être administré par voie ophtalmique en une quantité efficace à un sujet le nécessitant.

2. Composition pour son utilisation selon la revendication 1, dans laquelle le composé de formule I est le bindarit.

3. Composition pour son utilisation selon la revendication 1, dans laquelle la maladie oculaire diabétique est la rétinopathie diabétique ou l'œdème maculaire diabétique (OMD).

4. Composition pour son utilisation selon la revendication 1, dans laquelle la maladie oculaire diabétique est une cataracte ou un glaucome.

5. Composition pour son utilisation selon la revendication 1, dans laquelle le sujet souffre de diabète de type 1 ou de type 2.

6. Composition pour son utilisation selon la revendication 5, dans laquelle le sujet a un niveau de glycémie supérieur aux niveaux normaux.

7. Composition pour son utilisation selon la revendication 1, dans laquelle l'utilisation vise à réduire la translocation du NFκB au noyau dans les cellules oculaires du sujet.

8. Composition pour son utilisation selon la revendication 1, dans laquelle l'utilisation vise à normaliser le stress cellulaire dans les cellules hyperglycémiques.

9. Composition pour son utilisation selon la revendication 1, dans laquelle l'utilisation vise à réduire l'expression chez le sujet d'un ou plusieurs de Ccl2, Icam1 et Vcma1.

10. Composition pour son utilisation selon la revendication 1, dans laquelle l'utilisation doit comprendre en outre l'administration d'un ou plusieurs agents thérapeutiques supplémentaires.

11. Composition pour son utilisation selon la revendication 10, dans laquelle les un ou plusieurs agents thérapeutiques supplémentaires sont sélectionnés parmi un agent anti-VEGF, un inhibiteur de l'ACE, un agoniste ou agoniste partiel de PPAR-gamma, un inhibiteur de la rénine, un stéroïde, et un agent qui module l'autophagie, un sémapimod, un inhibiteur de MIF, un inhibiteur de CCR2, CKR-2B, un 2-thioimidazole, CAS 445479-97-0, CCX140, le clodronate, une préparation clodronate-liposome et le chlorure de gadolinium.

12. Composition pour son utilisation selon la revendication 1, dans laquelle l'administration ophtalmique est une administration intravitréenne ou intraoculaire.

13. Composition pour son utilisation selon la revendication 12, dans laquelle le composé est formulé pour une libération contrôlée ou une libération prolongée.

14. Composition pour son utilisation selon la revendication 12, dans laquelle le composé doit être administré par voie intravitréenne.
